(19) Europäisches Patentamt
European Patent Office
Office européen des brevets

(11) **EP 1 297 169 B1**

(12) **EUROPEAN PATENT SPECIFICATION**

(45) Date of publication and mention
of the grant of the patent:
**08.08.2012 Bulletin 2012/32**

(21) Application number: **01949436.8**

(22) Date of filing: **26.06.2001**

(51) Int Cl.:
*C12N 13/00* (2006.01)   *C12N 15/861* (2006.01)
*C12N 15/867* (2006.01)   *C12N 15/87* (2006.01)
*C12N 15/88* (2006.01)   *C12N 15/89* (2006.01)
*A61K 41/00* (2006.01)   *A61K 48/00* (2006.01)
*A61K 47/48* (2006.01)

(86) International application number:
**PCT/EP2001/007261**

(87) International publication number:
**WO 2002/000870 (03.01.2002 Gazette 2002/01)**

(54) **METHOD FOR TRANSFECTING CELLS USING A MAGNETIC FIELD**

VERFAHREN ZUR ZELLTRANSFEKTION MITTELS EINES MAGNETISCHEN FELDES

PROCEDE DE TRANSFECTION DE CELLULES A L'AIDE D'UN CHAMP MAGNETIQUE

(84) Designated Contracting States:
**AT BE CH CY DE DK ES FI FR GB GR IE IT LI LU MC NL PT SE TR**

(30) Priority: **26.06.2000 EP 00113083**
        **26.06.2000 US 214286 P**

(43) Date of publication of application:
**02.04.2003 Bulletin 2003/14**

(73) Proprietor: **ethris GmbH**
**82152 Martinsried (DE)**

(72) Inventors:
• **Plank, Christian**
  **82229 Seefeld (DE)**
• **Bergemann, Christian**
  **10823 Berlin (DE)**

(74) Representative: **Vossius & Partner**
**Siebertstrasse 4**
**81675 München (DE)**

(56) References cited:
WO-A-93/26019    WO-A-98/05791
WO-A-99/34908    WO-A1-91/12079
WO-A1-93/05815    WO-A1-99/60149
WO-A2-98/40049    DE-A- 19 624 426
US-A- 5 516 670    US-A- 5 753 477
US-A- 5 916 539

• **MAH ET AL: MOL THERAP, vol. 1, no. 5, 2000, page S239 XP001073433 cited in the application**
• **BERGEMANN C ET AL: "Magnetic ion-exchange nano- and microparticles for medical, biochemical and molecular biological applications" JOURNAL OF MAGNETISM AND MAGNETIC MATERIALS, ELSEVIER SCIENCE PUBLISHERS, AMSTERDAM, NL, vol. 194, no. 1-3, April 1999 (1999-04), pages 45-52, XP004166645 ISSN: 0304-8853 cited in the application**

Remarks:
The file contains technical information submitted after the application was filed and not included in this specification

SEQUENCE LISTING

<110> PLANK, Christian et al.

<120> Method for transfecting cells using a magnetic field

<130> E 1855 PCT

<140>
<141>

<160> 1

<170> Patent In Ver. 2.1

<210> 1
<211> 24
<212> PRT
<213> Artificial Sequence

<220>
<223> Description of Artificial Sequence: artificial

<400> 1

```
Gly Leu Phe Glu Ala Ile Glu Gly Phe Ile Glu Asn Gly Trp Glu Gly
 1               5                  10                  15

Met Ile Asp Gly Trp Tyr Gly Cys
             20
```

**Description**

The present invention relates to an in vitro method for transfecting a cell comprising bringing a complex comprising vector(s) comprising one or more nucleic acid molecules and magnetic particle(s) which are coupled with one or more oligo- or polycations or oligo-or polyanions in contact with a cell by applying a magnetic field wherein said magnetic particle(s) and said vector(s) are assembled in the complex by salt-induced aggregation. The present invention furthermore relates to such a complex as well as to methods for making it. Furthermore, the present invention relates to pharmaceutical compositions, uses and a kit.

The feasibility of gene therapy is ultimately dependent on the availability of efficient gene vectors. Gene vectors are vehicles used to transport a desired genetic information encoded by a nucleic acid (DNA or RNA) into a target cell, and to have it expressed there. Viruses have evolved formidable solutions to this gene transfer problem. Consequently, genetically modified (recombinant) viruses rank among the most efficient vehicles known today for the transfer of foreign genetic information into cells. A multitude of viral species have been engineered as gene vectors, including retroviruses, adenoviruses, adeno-associated viruses, herpes simplex viruses, hepatitis viruses, vaccinia viruses and lentiviruses. In general, the genetic information required for the natural replicative cycle of the virus is removed from the viral genome and replaced by the gene(s) of interest which is/are supposed to exert some therapeutic effect in the case of gene therapy applications. Most recently, also replication-competent viruses have been used as gene transfer vehicles.

As an alternative to viral gene vectors, non-viral, synthetic and half-synthetic vehicles for gene transfer have been developed over the last decade. Most of these non-viral vectors mimic important features of viral cell entry in order to overcome the cellular barriers to infiltration by foreign genetic material. Among these barriers are the plasma membrane, the membranes of internal vesicles such as endosomes and lysosomes and the nuclear membranes. Among the viral functions mimicked in non-viral vectors are the capability of receptor targeting, of DNA binding and compaction and of intracellular release from internal vesicles. These individual functions are represented in synthetic or half-synthetic modules which usually are assembled by electrostatic and/or hydrophobic interactions to form a vector particle. In order to systematically classify non-viral gene vectors according to their modular composition, the following nomenclature has been proposed (Felgner et al. 1997): Lipoplexes are assemblies of nucleic acids with a lipidic component, which is usually cationic. Gene transfer by lipoplexes is called lipofection. Polyplexes are assemblies of nucleic acids with an oligo- or polycationic entity. DNA complexes which comprise both classifications are called lipo-polyplexes or poly-lipoplexes. A huge variety of combinations of this general concept have been described. Examples include the classic cationic lipid-DNA complexes (Felgner and Ringold 1989), polycation-DNA complexes such as poly(lysine)-DNA (Wu and Wu 1987), poly(ethylene imine)-DNA (Boussif et al. 1995), poly(amido amine) dendrimer-DNA (Haensler and Szoka 1993), cationic peptide-DNA complexes (Plank et al. 1999), cationic protein-DNA complexes (histones, HMG proteins) (Zenke et al. 1990). Often such DNA complexes are further modified to contain a cell targeting or an intracellular targeting moiety and/or a membrane-destabilizing component such as an inactivated virus (Curiel et al. 1991), a viral capsid or a viral protein or peptide (Fender et al. 1997; Zhang et al. 1999) or a membrane-disruptive synthetic peptide (Wagner et al. 1992; Plank et al. 1994). Also, the nucleic acid to be transported has been enclosed in the aqueous lumen of liposomes (Nicolau and Cudd 1989), or polycation-condensed DNA is associated with a lipid membrane (Gao and Huang 1996; Li et al. 1998). The lipid membrane has also been composed to be a chimera of natural membranes derived from viruses or cells containing membrane proteins (HVJ liposomes for example (Kaneda 1998)]). Recently, also bacteria (GrillotCourvalin et al. 1998) and phages (Poul and Marks 1999) have been described as shuttles for the transfer of nucleic acids into cells. Apart from these sophisticated vector compositions, also naked DNA is known to be a useful transfecting agent in certain applications (Wolff et al. 1990). The precipitation of DNA with divalent cations has been used successfully for the transfection of cultured cell lines for more than 10 years (calcium phosphate precipitation (Chen and Okayama 1988) ]). Most recently, it has been found that calcium phosphate precipitation protocols are also useful in enhancing both viral and non-viral vector-mediated gene transfer (Fasbender et al. 1998).

Vectors or naked DNA can also be formulated to achieve a sustained release or controlled release effect. For this purpose, DNA or vectors can be immobilized on/in or associated with carrier materials such as collagen (Bonadio et al. 1998), gelatin (Truong-Le et al. 1999) or fibrin glue. Also, DNA or vectors can be incorporated in micro- and nanoparticle formulations such as in copolymers like poly(lactic-co-glycolic acid) (PLGA) (Shea et al. 1999) and similar compositions or in nanoparticles prepared from chitosan (Roy et al. 1999).

No matter how efficient any of the described gene transfer methods are in selected applications, all of them suffer from serious limitations concerning their general applicability in gene therapy. Striking among such limitations are the following, particularly for in vivo gene therapy:

1. Target specificity

a) In the one extreme, limited targetability of vectors to target cells/organs due to the lack of a specific target cell tropism and as a consequence systemic spread of the vector, limited bioavailability at the target site and the possibility of non-specific transfection of non-target cells/organs.

b) In the other extreme, a very specific host tropism limiting the applicability of a vector to a broader target spectrum. This problem prevails for viral vectors, both in ex- and in vivo gene delivery.

2. Rapid inactivation of vectors due to undesired interactions with components of the in vivo milieu before the vector can find its target site. Examples of undesired interactions are opsonization of non-viral vectors (Ogris et al. 1999) or interactions of vectors with host defense systems such as the complement system (Plank et al. 1996) or the immune system (Kass-Eisler et al. 1996).

3. Insufficient vector concentration at the target site (Luo and Saltzman 2000).

Partial solutions to these problems have been provided. The host tropisms of viral vectors have been broadened, narrowed or redirected by genetic engineering of particular surface proteins that act as ligands for cellular receptors (Kasahara et al. 1994; Michael et al. 1995), by coupling of targeting ligands to viral surfaces (Curiel 1999), by co-precipitating viruses with calcium phosphate (Fasbender et al. 1998) or by electrostatic interaction with a non-specific cell-binding molecule such as a polycation (Fasbender et al. 1997). The undesired interactions with components of the in vivo milieu are reduced by vector modification with molecules such as poly(ethylene glycol) (O'Riordan et al. 1999; Ogris et al. 1999; Romanczuk et al. 1999; Finsinger et al. 2000). Improved localization of gene transfer and a limitation of systemic spread is achieved by topical vector application rather than systemic application, eventually in combination with a controlled-release formulation (Rajasubramanian et al. 1994; Bonadio et al. 1998).

The document WO911207 describes a method to isolate macromolecules using magnetically attractable beads which do not specifically bind the macromolecules.

The document by Hughes C et al (2001), Mol. Therap., Vol.3 Nr.4 pp.623-630, describes that Streptavidin paramagnetic particles provide a choice of three affinity-based capture and magnetic concentration strategies for retroviral vectors.

However, there remains a great potential to be exploited for enhancing target specificity and/or for reducing in vivo inactivation of vectors, in particular when a broad scale of targets is to be addressed with a single general technique.

Thus, the technical problem underlying the present invention is to provide an improved method and suitable means therefore for enhancing gene delivery into a desired cell population. In a particular aspect, this technical problem relates to an increase of the concentration of a gene vector at a target site by physical means. In yet another aspect, the technical problem underlying the present invention relates to the provision of a transfection method with favourable dose-response characteristics and favourable transfection kinetics characteristics for rapid high throughput transfection.

SUMMARY OF THE INVENTION :

This problem has been solved by the provision of the embodiments as characterized in the claims.

Accordingly, the present invention relates to an in vitro method for transfecting a cell comprising the step of bringing a complex comprising (i) one or more vectors comprising one or more nucleic acid molecules and (ii) one or more magnetic particles which are coupled with one or more oligo- or polycations or oligo- or polyanions in contact with a cell by applying a suitable magnetic field wherein said magnetic particle(s) and said vector(s) are assembled in the complex by salt-induced aggregation.

The present invention is based on the observation that the efficiency of transfection of a cell is significantly elevated when vectors to be transfected are linked to a moiety susceptible to magnetic force of attraction and said vector is directed to said cell by applying a magnetic field. The term "efficiency" refers to the rate of transfection within a given time unit. Increase of efficiency may be expressed in terms of decreasing the time necessary for transferring a given amount of vector into a given number of cells and/or in terms of increasing the amount of vector that is transferred into a given number of cells within a given time unit Advantageously, in transfection reactions which are carried out for up to 30 min, preferably for up to 20 min and most preferably for up to 10 min, the transfection efficiency is increased by at least two-fold, more preferred by at least 5-fold, still more preferred by at least 10-fold, particularly preferred by at least 20-fold and most particularly preferred by at least 40-fold, when the method of the invention is applied. In addition, increases of at least two-fold may also be obtained when applying the method in transfections for more than 30 min. Alternatively, transfection efficiency can be expressed in the form of the dose-response profile of a given vector. The term "dose-response profile" refers to the degree of an intended effect which is achievable per unit nucleic acid (or virus or nucleic acid analogon) dose applied in a procedure in order to achieve such an effect. For gene transfer experiments, the term "dose-response profile" relates to the level of expression of the transfected gene achievable per unit DNA (or RNA or virus) dose applied in the transfection experiment.

The term "vector" refers to entities comprising one ore more nucleic acid molecules that is/are aimed to exert a desired function in a target cell, preferably to express a desired genetic information. Such entities may be conventional viral or non-viral vectors as well as bacteria or bacteriophages. The nucleic acid molecule(s) may be DNA or RNA or hybrids thereof or any modification thereof that is known in the state of the art (see, e.g., US 5525711, US 4711955, US 5792608 or EP 302175 for examples of modifications). Such nucleic acid molecule(s) are single- or double-stranded, linear or circular, natural or synthetic, and without any size limitation. For instance, the nucleic acid molecule(s) may be genomic DNA, cDNA, mRNA, antisense RNA, ribozyme or a DNA encoding such RNAs or chimeraplasts (Colestrauss et al. 1996). Preferably, said nucleic acid molecule(s) is/are in the form of a plasmid or of viral DNA or RNA. Nucleic acid molecule(s) comprised in the vector may also be oligonucleotide(s), wherein any of the state of the art modifications such as phosphothioates or peptide nucleic acids (PNA) are included.

Preferably, the nucleic acid molecules comprised in the vectors are plasmids, cosmids, viruses or bacteriophages used conventionally in genetic engineering that contain a nucleotide sequence, for instance encoding a polypeptide, that is to be expressed in a target cell. Preferably, said vector is an expression vector and/or a gene transfer or targeting vector. Methods which are well known to those skilled in the art can be used to construct recombinant nucleic acid molecules; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989).

In addition to a gene to be expressed in the target cell, the nucleic acid molecules contained in the above-mentioned vectors may comprise further genes such as marker genes which allow for the selection of the vector in a suitable host cell and under suitable conditions. Preferably, the nucleotide sequence to be expressed is operatively linked to expression control sequences allowing expression in prokaryotic or eukaryotic cells. Expression of said nucleotide sequence comprises its transcription into a translatable mRNA. Regulatory elements ensuring expression in eukaryotic cells, preferably mammalian cells, are well known to those skilled in the art. They usually comprise regulatory sequences ensuring initiation of transcription and, optionally, a poly-A signal ensuring termination of transcription and stabilization of the transcript, and/or an intron further enhancing expression of said nucleotide sequence. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription, such regulatory elements may also comprise transcription termination signals, such as the SV40-poly-A site or the tk-poly-A site, downstream of the nucleotide sequence. Furthermore, depending on the expression system used, leader sequences capable of directing the polypeptide to a cellular compartment or secreting it into the medium may be added to the nucleotide sequence and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of the expressed recombinant product In this context, suitable expression systems are known in the art such as Okayama-Berg cDNA expression vector pcDV1 (Pharmacia), pCDM8, pRc/CMV, pcDNA1, pcDNA3, the Echo™ Cloning System (Invitrogen), pSPORT1 (GIBCO BRL) or pRevTet-On/pRevTet-Off or pCI (Promega).

Preferably, the expression control sequences will be eukaryotic promoter systems in vectors capable of transforming or transfecting eukaryotic host cells, but control sequences for prokaryotic hosts may also be used.

As mentioned above, the vector used in the method of the present invention may also be a gene transfer or targeting vector. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. For example, the vector useful in the method of the invention may consist of nothing but one or more of the above described nucleic acid molecule(s), i.e., in the complex used for transfecting a cell, the magnetic particle(s) is/are linked only to one or more nucleic acid molecule(s) such as for example naked DNA or oligonucleotides. Naked DNA can be a useful transfecting agent in certain in vivo applications as has been first described by Wolff (1990).

Preferably, the vector comprises in addition to the nucleic acid molecule(s) compounds suitable for facilitating transfection of cells and/or enhancing its efficiency. In particular, these vectors refer to non-viral, synthetic or half-synthetic vehicles for gene transfer. Most of these non-viral vectors mimic important features of viral cell entry in order to overcome the cellular barriers to infiltration by foreign genetic material. Among these barriers are the plasma membrane, the membranes of internal vesicles such as endosomes and lysosomes and the nuclear membranes. Among the viral functions mimicked in non-viral vectors are the capability of receptor targeting, of DNA binding and compaction, of intracellular release from internal vesicles and of nuclear targeting. These individual functions are represented in synthetic or half-synthetic modules which usually are assembled by electrostatic and/or hydrophobic interactions to form a vector particle. Non-viral gene vectors are in the following referred to according to the nomenclature proposed by Feigner et al. (1997). Accordingly, the vectors may contain, linked with the nucleic acid molecule(s), one or more functional groups capable of promoting gene transfer.

In one possibility and as a preferred embodiment of the invention, the vector may comprise as such a functional group one or more oligo- or polycationic components to form a polyplex. Such cationic compounds may for example, but not exclusively, be poly(lysine), poly(ethylene imine) (PEI), poly(amido amine) dendrimers chitosan, protamine, spermine and spermidine and derivatives thereof, cationic DNA binding peptides or cationic proteins such as histones. Another possibility refers to an assembly of the nucleic acid molecule(s) with one or more lipidic, preferably cationic lipidic components to form a lipoplex. Examples of suitable lipids include DOTAP, DMRIE, DOGS, DLRIE, DC-CHOL, GL-67, DOSPA to name a few, or their commercially available formulations such as Lipofectamine, Transfectam, GenePorter or Fugene, to name a few (for reviews see (Lee and Huang 1997; Zabner 1997)). The vector may also comprise both oligo- or polycationic and lipidic components, which then form a lipo-polyplex or poly-lipoplex.

Furthermore, the vector may be assembled with one or more proteins, preferably cationic proteins, to form, e.g., protein-DNA complexes, whereby the protein(s) may be recombinant or of natural origin. Examples include HMG-1, histones, protamines and gal-4 binding domains. Examples of the above-identified non-viral gene vectors include cationic lipid-DNA complexes (Felgner and Ringold 1989), polycation-DNA complexes such as poly(lysine)-DNA (Wu and Wu 1987), poly(ethylene imine)-DNA (Boussif et al. 1995), poly(amido amine) dendrimer-DNA (Haensler and Szoka 1993), cationic peptide-DNA complexes (Plank et al. 1999), cationic protein-DNA complexes (histones, HMG proteins) (Zenke et al. 1990).

A further possibility of a non-viral vector is a liposome, wherein the nucleic acid molecule(s) is/are enclosed in the aqueous lumen as described in Nicolau (1989). Optionally, nucleic acid molecule(s) associated with lipid membranes can be compacted by one or more (poly)cationic components; see, e.g., Gao (1996) or Li (1998), or by divalent cations. The lipid membrane can optionally contain or be linked to non-lipid components such as proteins, carbohydrates or glycosaminoglycans. These components can be synthetic or can be taken from natural sources such as cellular or viral membranes, such as for example in HVJ liposomes (Kaneda, 1998). The vector comprised in a complex together with magnetic particles according to the method of the present invention may also be a viral vector. Examples for such viral vectors include but are not limited to recombinant adenoviruses, adeno-associated viruses, retroviruses, herpes simplex viruses, hepatitis viruses and lentiviruses. The genetic information required for the natural replicative cycle of such a virus may be removed from the viral genome and replaced by the gene(s) of interest which may for example exert some therapeutic effect in the case of gene therapy applications. However, also within the scope of the present invention is the use of replication-competent viruses.

Alternatively, viruses or parts thereof may also be used in association with any of the above-described non-viral vectors, including vectors consisting only of nucleic acid molecules such as naked DNA. For producing such vectors being a combination of one or more viruses or part(s) thereof and an otherwise non-viral vector, natural wild-type or recombinant, live or inactivated viruses can be used.

According to the present invention, the vector may additionally include effector molecules that enhance gene delivery and/or facilitate cell or intracellular targeting of gene vectors. Such molecules include but are not limited to membrane-destabilizing or membrane-permeabilizing molecules such as synthetic peptides, natural or synthetic receptor ligands including antibodies, or signal peptides such as nuclear localization signal peptides. Further examples for such molecules are given by an inactivated virus (Curiel et al. 1991), a viral capsid or a viral protein or peptide (Fender et al. 1997; Zhang et al. 1999) or a membrane-disruptive synthetic peptide (Wagner et al. 1992; Plank et al. 1994).

To any of the above described vectors salts of divalent cations, particularly in solution, can be added, e.g., divalent cations such as calcium chloride or calcium phosphate (calcium phosphate precipitation). Most recently, it has been found that calcium phosphate precipitation protocols are also useful in enhancing both viral and non-viral vector-mediated gene transfer (Fasbender et al. 1998).

In addition, any of the above described vectors may have added or have covalently linked thereto polymers or copolymers for steric stabilization and/or minimization of opsonization or complement activation during the in vivo delivery phase.

The vectors used herein can also be formulated so as to achieve a sustained release or controlled release effect. For this purpose the vectors can be incorporated or encapsulated in micro- or nanoparticle formulations such as in copolymers like poly(lactic-co-glycolic acid) (PLGA) (Shea et al. 1999) (Cohen et al. 2000) or similar compositions or in particles, such as nanoparticles, prepared from chitosan (Roy et al. 1999) or gelatin (Leong et al. 1998; TruongLe et al. 1998; Kalyanasundaram et al. 1999; Truong-Le et al. 1999), or alginates (Quong and Neufeld 1999; Rowley et al. 1999) or state-of-the-art biomaterials such as presented at the 5th New Jersey Symposium on Biomaterials Science (Somerset, NJ, USA; Nov. 9-10, 2000), or diffusion nanospheres (Hirosue et al. 2001) or in sol-gel polymers (Gill and Ballesteros 2000) or in lipidic and non-lipidic lamellar phases (Bailey and Sullivan 2000; Freund et al. 2000; Ponimaskin et al. 2000), or in hydrogels (Petka et al. 1998). Furthermore, the vectors as described above can be immobilized on/in or associated with carrier materials such as collagen (Bonadio et al. 1998), gelatin (Truong-Le et al. 1999) or fibrin glue, natural or synthetic hydroxyapatites or other synthetic biomaterials. Such sustained release or controlled release particles may be prepared by incorporating the vector(s) and/or the magnetic particle(s) already when preparing the polymer matrix.

Also within the scope of the invention are vectors that can be bacteria (GrillotCourvalin et al. 1998) or phages (Poul and Marks 1999), which have recently been described to successfully transfer nucleic acids into cells.

The term "magnetic particle" refers to magnetically responsive solid phases which are particles or aggregates thereof of micro- to nanometer-ranged size (preferably not larger than 100 $\mu$m) which contain one or more metals or oxides or hydroxides thereof, that react to magnetic force upon the influence of a magnetic field, preferably resulting in an attraction towards the source of the magnetic field or in acceleration of the particle in a preferred direction of space. The term "magnetic", as used herein refers to temporarily magnetic materials, such as ferrimagnetic or ferromagnetic materials. The term, however, also encompasses paramagnetic and superparamagnetic materials. Some physical properties of such materials have been reviewed in (Fahlvik et al. 1993).

The magnetic particles are synthetic, i.e. they are not obtainable from a biological source, which means from a living organism. The properties of magnetic particles to be used in the method of the present invention must be such that they can be associated with any of the vectors as herein described. Because association with gene vectors can be achieved by a variety of interaction types or galenic formulation, no very specific requirements are imposed on particle composition, shape and size. With respect to the application in in vivo gene delivery, the particles and their degradation products preferably do not induce systemic toxicity. Iron oxide particles are clinically approved as contrasting agent in magnetic resonance imaging. The pharmacokinetics and toxicity profile of iron oxide particles have been described by (Weissleder et al. 1989). Furthermore, since such applications usually require mobility under highly restricted spacial conditions, the particle size should not be greater than a reasonable upper limit.

Accordingly, in a preferred embodiment of the method said magnetic particles have a size (i.e. maximal extension) of up to 2000 nm, more preferred of up to 1500 nm, even more preferred of up to 1000 nm, particularly more preferred of up to 800 nm and most preferred of up to 600 nm.

Particles that can be associated with gene vectors and that are useful in gene delivery are made of one or more materials including ferro-, ferri- or superparamagnetic compounds, such as iron, cobalt or nickel, magnetic iron oxides or hydroxides such as $Fe_3O_4$, gamma-$Fe_2O_3$ or double oxides/hydroxides of two- or three-valent iron with two- or three-valent other metal ions or mixtures of the mentioned oxides or hydroxides.

According to the state of the art (U. Schwertmann and R.M. Cornell, Iron Oxides in the Laboratory, VCH Weinheim 1991), magnetic colloidal iron oxide/hydroxide particles are prepared by precipitation from an acidic iron(II)/iron(III)-salt solution upon addition of bases.

The magnetic particles used in the Examples described below were purchased from Chemicell, Berlin, Germany. The preparation of such particles is disclosed in DE 196 24 426: Iron oxide/hydroxide particles are derived upon addition of equivalent amounts of alkali carbonates (sodium hydrogencarbonate, sodium carbonate and/or ammonium carbonate) to an acidic iron(II)/iron(III) salt solution followed by thermal oxidation to magnetic iron hydroxide and furtheron to iron oxide. The final particle size can be adjusted by thermal control of reaction velocity and by choosing appropriate concentrations of the reactands. Thus, small diameter particles of 20 - 100 nm are obtained by timely separated formation of iron (II,III)-carbonate at temperatures of 1 - 50 °C, preferably at 5 - 10 °C and subsequent heating. Larger particles of 100 - 1000 nm are obtained at reaction temperatures of 60 - 100 °C implying a faster transformation of iron(II, III)-carbonate to iron(II,III)-hydroxide. Nano-crystalline magnetic particles from double-oxides or hydroxides of two- or three-valent iron with two- or three-valent metal ions other than iron or mixtures of the corresponding oxides or hydroxides can also be prepared according to the above-mentioned procedures by using a salt solution of the two- or three-valent metals. Magnetic double oxides or- hydroxides of the three-valent iron are preferentially prepared with two-valent metal ions selected from the first row of transition metals (such as Co(II), Mn(II), Cu(II) or Ni(II)), whereas magnetic double oxides or-hydroxides of the two-valent iron are preferentially prepared with three-valent metal ions such as Cr(III), Gd (III), Dy(III) or Sm(III).

The so-produced magnetic particles can be coated with positively or negatively charged electrolytes, such as phosphates, citrates or amines, with silanes (see US 4,554,088 and 4,554,089), fatty acids (see US 4,208,294) or polymers, such as polysaccharides (US 4,101,435), polyamino acids, proteins or synthetic polymers (see DE 196 24 426). These coating compounds can have reactive or derivatizable functional groups or these can be introduced by chemical modification after the coating process. In this context, also the term "coupling" is used as a synonym for "coating" in order to describe the present invention.

Functional groups can have cation exchange properties such as found in xanthate-, xanthide-, dicarboxyl-, carboxymethyl-, sulfonate-, sulfate-, triacetate-, phosphonate-, phosphate-, citrate-, tartrate-, carboxylate-, or lactate groups of naturally occurring or synthetic polymers. Alternatively, these functional groups can be introduced into natural and synthetic polymers prior to coating or after coating of magnetic particles. Examples of naturally occurring polymers are polysaccharides such as starch, dextran, glycosaminoglycans, agar, gum-gatti or gum-guar or analogues thereof. Suitable derivatives of synthetic polymers can be based on poly(vinyl alcohol) or poly(vinylpyrrolidone) or poly(ethylene glycole), poly(lactic acid), poly(lactic-co-glycolic acid) or poly(caprolactone). Also proteins like casein, collagen, gelatin, albumin or analogous derivatives thereof are useful coating compounds. Other examples of suitable polymers with ion exchange characteristics are polyacrylic acids, poly(styrene sulfonic acid), poly(vinylphosphoric acid) or polymeric arabinic acid, alginate, pectin or polyaspartic or polyglutamic acid.

Anion-exchange polymers carry endstanding or internal primary-, secondary amino-, imino-, tertiary amino- or quarternary ammonium groups, such as amino-, alkylamine, dietylaminoethyl-, triethylaminoethyl-, trimethylbenzylammonium-groups. Again, these polymers can be of natural or synthetic origin and the cationic functional groups can be inherent or can be grafted by synthetic methods prior or after coating of magnetic particles. Examples include polysaccharides, proteins or synthetic polymers and derivates thereof such as chitosan, poly(lysine), poly(ethylene imine), poly(amine), poly(diallyldimethylammonium) or poly(vinylpyridine).

Functional groups for covalent coupling can be inherent in such polymers or can be introduced by synthetic methods well known to the one skilled in the art of synthetic chemistry. Examples are aldehyde, diazo, carbodiimide, dichlortriazine, alkyl halogenide, imino carbonate, carboxyl, amino, hydroxyl, or thiol groups.

The magnetic particles for use in the method of the invention are coupled with one or more oligo- or polycations or oligo- or polyanions.

In a preferred embodiment, said oligo- or polycation or oligo- or polyanion is a compound selected from the group consisting of poly(ethylene imine) (PEI), PEI-streptavidin, PEI-biotin, starch-phosphate, polyaspartic acid, polyacrylic acid, polyacrylic-co-maleic acid and arabinic acid. PEI as well as other compounds mentioned to be useful for coating the magnetic particles may be modified. Examples include PEI-ethoxylated (a monolayer of PEI coating the magnetic particle being ethoxylated), PEI-epichlorhydrin (PEI modified with epichlorhydrin) or PEI-sodium dodecyl sulfate (PEI modified by a covalent coupling of sodium dodecyl sulfate (SDS) by carbodiimide activation (N-Ethyl-N'-(dimethylaminopropyl)-carbodiimide).

The term "complex" used in context with the method of the present invention relates to a finite entity comprising one or more vector(s) and one or more magnetic particle(s) as defined herein above, which are suited for being brought in contact with cells in order to transfect them. The ratio of vector and magnetic particles in a complex may vary and mainly depends on the amounts of vector and magnetic particles mixed together when preparing the complex.

The term "transfection" refers to a process of introducing one or more nucleic acid molecule(s) into a cell. Thereby, "transfection" encompasses any kind of techniques for introducing nucleic acid molecules into cells known in the prior art, also including for instance transduction, transformation and the like. Preferably, said one or more nucleic acid molecule(s) are foreign to the cell. The term "foreign" may refer to (a) nucleic acid molecule(s) which is/are not part of the genome of the cell nor of any other nucleic acid molecule being present in the cell before said transfection such as extrachromosomal DNA, plasmids, cosmids or artificial chromosomes. Likewise, the term "foreign" may refer to nucleic acid molecules which are, at least partially, homologous with respect to the target cell, however, occur in the vector in a different molecular environment than those naturally occurring in the cell. Such homologous nucleic acid molecules include, e.g., overexpression or antisense constructs. The term "magnetofection" as used in connection with the present invention refers to transfection using complexes of magnetic particle(s) and vector(s) as described herein, preferably, involving the application of a magnetic field.

The term "cell" refers to any prokaryotic or eukaryotic cell, preferably to mammalian cells, most preferably to human cells. If the cells originate from multicellular organisms, said cells may be transfected in their original tissue or transfection takes place in vitro, i.e. the cells are outside the organism, preferably outside the tissue and most preferably in cell culture, such as freshly isolated primary cells or immortalized or tumor cell lines. In principle, there are no limitations regarding size, shape and composition of said complex, apart from the features that they comprise vector(s) and magnetic particle(s) and that they are suitable for being brought in contact with cells. The term "to bring in contact with cells" means that a complex is brought in such close proximity to a cell that transfection can take place, i.e. that the passage through the plasma membrane is possible. This term also means to locally enrich said complexes within reach of the cells to be transfected which would otherwise, in the absence of a suitable magnetic field, diffuse and therefore would have a pronouncedly lower concentration within reach of the target cells. The term "reach" refers to the space around the cells from where the cells are accessible to the vectors for transfection.

According to the method of the invention, the complete complex may enter the cell or only a part thereof, containing at least the nucleic acid molecule(s) or the vector but being devoid of the magnetic particle(s). Thus, cellular incorporation of the magnetic particle(s) may or may not be included in said method. In the latter case, the linkage between magnetic particle(s) and vector(s) can be designed reversible.

The magnetic particle(s) and the vector(s) are assembled in said complex by salt-induced aggregation.

The preparation of the complexes comprising one or more magnetic particles and one or more of the above described vectors may be achieved by suitable methods of salt-induced aggregation common to the person skilled in the art and available from the literature. Preferably, regarding preparation of some of the complexes conceivable, any of the following mixing procedures may be used.

For example, a complex wherein the vector is naked DNA, may be prepared by adding said naked DNA to cationic magnetic particles.

A complex comprising a poly- or lipoplex vector may be prepared by, first, adding naked DNA to cationic or anionic magnetic particles, followed by addition of the appropriate polycation(s) or polyanion(s) or cationic or anionic lipid(s). Alternatively, such complexes may be obtained by, first, preparing a poly- or lipoplex, i.e. by adding naked DNA to polycation or cationic lipid, followed by adding cationic or anionic magnetic particles. Another procedure to obtain complexes comprising a lipoplex is via the intermediate preparation of liposomes. Accordingly, cationic or anionic magnetic particles are incorporated in cationic, anionic on neutral liposomes, which is followed by addition of naked DNA or any kind of vector described herein.

For vector assembly, the process of salt-induced aggregation, a phenomenon well-known in colloid science (Hiemenz 1986), is exploited: Colloidal systems with charged surfaces tend to aggregate (flocculate) due to over-compensation of repulsive (electrostatic) forces by attractive forces upon increasing the ionic strength. For gene vectors described herein, this requires nothing more than mixing of the components in salt-containing solvent or mixing in salt-free solvents, followed by addition of salt.

Preferably, to such a complex, an additional effector or component may be applied, such as by incorporation of a chemically inactivated, biotinylated, E1A-deleted adenovirus or a membrane-destabilizing peptide (disclosed in US 5,981,273). Similarly, magnetic particles can be connected with the vectors using antigen-antibody interaction.

The method of the present invention comprises the application of a suitable magnetic field. The term "suitable magnetic field" refers to magnetic fields that, with regard to the shape of the field and its strength, are suited for attracting the above described complexes against other forces acting on the complexes, such as diffusion or hydrodynamic forces. If, for example, culture cells which are spread all over the bottom of a culture dish shall be transfected, a suitable magnetic field would be a field drawing the complexes towards the bottom. If, in that case, an inhomogenous field is applied, which, for instance, effects only a part of the cells, only those cells in the dish become transfected, where a magnetic field of a high enough intensity runs through. Suitable magnetic fields, preferably when applied in vivo, have an intensity of more than 0.5 Tesla, preferably of more than 1 Tesla. For applying the herein described method in vivo to a certain region of the body, an inhomogenous magnetic field is directed to said region so that the complexes can enrich there. For accelerating superparamagnetic particles, as mentioned herein, in a desired direction of space, magnetic gradient fields are applied (Zborowski et al. 1995). The physical theory of magnetic force acting on superparamagnetic and the like particles in fluids can be found in text books such as L.D. Landau et al. (The Field Theory. Vol 2, Moskow, Nauka (1973), 128-136) or publications such as Zborowski et al. 1995.

A preferred embodiment refers to the method of the present invention, wherein said magnetic field is a permanent field or an electromagnetic field.

The term "permanent field" refers to magnetic fields which are generated by a permanent magnet. Examples of suitable permanent magnets include high energy, permanent magnets, e.g., made of materials containing neodym or magnets as used in the appended Examples. In order to adapt the geometry of the target region, such permanent magnets may be constructed as arrays, as yoke and magnetic return path or in aperture or sandwich configurations. The intensity may be controlled with a suitable measuring instrument such as a Hall probe.

The term "electromagnetic field" refers in the context to the present invention to magnetic fields which are generated by electric current. Applicable examples include nuclear magnetic resonance tomographs. Such devices may be at the same time usable for generating the field and for diagnosing, supervising and documenting the distribution and local enrichment of the complexes applied.

A further preferred embodiment refers to the method of the invention, wherein said electromagnetic field oscillates.

The term "oscillate" refers to magnetic fields which periodically change their direction. Such an oscillation may induce kinetic energy in the complexes which may be useful in cases where the vectors shall be released from the complex and the movement promotes its diffusion. High frequency oscillation in the kHz to GHz range for example may be used to induce a local hyperthermia in order to support, e.g., an anti-tumor gene therapy. Low frequency pulsating weak electromagnetic fields (1 - 250 Hz; 4 - 10 mT) have been used clinically in bone healing (Gossling et al. 1992; Rubin et al. 1993). Physiologic effects on a molecular as well as macroscopic level are well documented (Trock 2000). An influence on intracellular calcium levels has been demonstrated in cell culture (Carson et al. 1990). As pulsating electromagnetic field(PEMF) treatment also improves blood supply for example in hypoxemic tissue, it may well be used advantageously with the application of said complexes.

The method of the present invention as described above in detail constitutes a major improvement over prior art transfection methods regarding in vitro as well as in vivo applications. In the experiments which are documented in the appended Examples, it was surprisingly found that, under the influence of a magnetic field, vectors which are in a complex with magnetic particles are transferred with a significantly enhanced efficiency into cells. The attracting force of a magnetic field promotes at least the enrichment of vector concentration in the region surrounding the target cells which alone may contribute to the enhanced transfection efficiency. Example 20, however, provides evidence that the magnetic field may promote transfection beyond concentrating a vector in the region surrounding the target cells, e.g.

by forcing a vector into the target cell.

Enhancement was shown for each type of vector so far tested. Examples 2 and 24-27 describe the successful application of the method of the invention using lipoplexes or liposomes. Example 4, shows the same for polyplexes whereby the polyplexes were further equipped with a protective copolymer. Examples 5 and 6 describe experiments with polyplex-based complexes which are furthermore associated with inactivated adenoviruses.

For example, transfection with lipoplexes or liposomes as shown in Example 2, resulted at least in a two fold increase of transfection by applying a magnetic field compared to the corresponding experimental setup without magnetic field. In these experiments, the highest increase was shown to be 43-fold and was obtained for the highest dilution of liposomes containing DNA and magnetic particles. The fact that in Example 2 always the highest dilutions yielded the greatest relative increase may be taken for evidence that the positive effect of the magnetic field is essentially based on a local enrichment of the vectors near the target cells.

The herein presented data obtained in in vitro transfection experiments is directly transferable to in vivo conditions. The animal experiments described in Example 17 successfully demonstrate that magnetofection improves targeted gene expression at a desired site of the body, i.e. tissue or organ. Expectedly, a local enrichment of gene delivery vectors at the desired region of the body will lead to an increased transfection efficiency in the respective tissue or organ. In addition, it is conceivable that magnetofection not only enhances gene transfer into cells but also facilitates gene transfer which would not occur in the absence of a magnetic field. It is furthermore contemplated that the use of the complex of the invention in vivo, as for instance in gene therapy, enabling specific localization of vectors principally in any desired body region, overcomes many of the limitations connected with conventional gene therapeutic methods, as discussed above. A major obstacle was the limited availability of target specificity for gene therapy vectors, e.g., due to the lack of molecular markers suitable for addressing a desired body region. With the provisions of the present invention, this obstacle is overcome, since, due to the neutrality of biological tissues regarding magnetic fields, in principle any desired body region may be object to specific localization of vectors and transfection therein. As a consequence, the complex of the invention is well suited to reduce another limitation which results from rapid inactivation of vectors under in vivo conditions. The specifically localized enrichment at the desired site lowers the average time period, that for example a molecularly targeted vector has to stay in the blood circulation system until it reaches its target. Hence, the time during which such a vector is accessible to inactivation mechanisms is reduced. This time is furthermore reduced by way of the enhanced transfection efficiency which is, above all, expressed by a faster transfer into the cell.

The present invention may be seen against the background of developments in classical drug delivery, where a recently re-discovered regimen of drug localization is its immobilization on a magnetic particle and the concentration to a particular place in the "patient" organism by a magnetic field (Lübbe et al. 1996; Lübbe et al. 1996; Babincova et al. 2000). From the prior art some approaches are known where magnetic particles were used in connection with transfection methods. "Dynabeads" (Dynal, Hamburg, Germany) which are commonly used for the separation of biomolecules and cells based on receptor-ligand type interactions under the influence of a magnetic field have been well known among researchers. Most recently, such particles have been exploited in gene delivery by reportedly creating holes in cell membranes by mechanical forces, thus facilitating transfection (Bildirici et al. 2000). In a further approach, naked DNA has been adsorbed to magnetic particles by electrostatic interaction and has consequently been electroporated into CIK cells with the aim of a subsequent separation of transfected and non-transfected cells by a magnetic field (Bergemann et al. 1999). A kit for separation of transfected and non-transfected cells with the help of magnetic particles is commercially available from Miltenyi, Germany (http://www.miltenvibiotec.com). The technical principle is to co-express a cell surface antigen together with the gene of interest and then use magnetic particles coated with a specific antibody recognizing this antigen to bind to such cells and separate them from untransfected ones (Padmanabhan et al. 1993). At a recent expert meeting, the incorporation of magnetic bacterial organelles, socalled magnetosomes, into cationic lipid-DNA complexes and successful gene delivery was reported (Reszka et al. 2000). Also, magnetic microspheres have been associated with adeno-associated virus (AAV), yielding favourable gene transfer characteristics (Mah et al., poster abstract in: Mol. Therapy 1(5) (2000), p. S239).

However, in none of the cited approaches, a magnetic field was applied during transfection. Thus, the surprising advantages regarding enhancement of transfection efficiency and, optionally, specific localization in in vitro and in vivo applications, as reported in connection with the present invention, could not be perceived. In addition, regarding the magnetosome approach, it can be foreseen that commercial and routine applicability of this development will suffer from the difficult availability of the magnetosomes, whereas the magnetic particles used in the method of the invention are available to in principle unlimited extent at low production costs.

The method of the invention is applied in vitro.

The definition of the term "in vitro" has already been given, above. The preferred embodiment includes also "ex vivo" application in the sense that cells which are transfected (transduced) according to the method of the invention with a recombinant nucleic acid molecule are afterwards (re-)implanted in said subject.

Yet another preferred embodiment of the invention relates to the above described method, which is applied in vitro for high-throughput transfection and transduction. Thus, the present invention furthermore relates to the use of the aforementioned magnetic particles and/or complexes for the transfection of cells in a high-throughput assay. In particular, such transfection involves the application of a magnetic field. The preferred embodiment includes automatization of the method for purposes such as, but not limited to, high-throughput gene screening. The term "high-throughput" gene screening refers to procedures where multiple transfections are carried out preferably in parallel and preferably in an automatized fashion in order to identify or assign a function to known or unknown nucleic acid sequences or in order to select among known or unknown nucleic acid sequences those exerting a particular function when introduced into a cell. The particular function may be exerted by the expression of said nucleic acid sequence in the transfected cell but also by other mechanisms as a consequence of the nucleic acid's presence in the cell, such as, but not limited to, ribozyme or antisense action. Such nucleic acid sequences may be, but not exclusively, represented in cDNA libraries, viral and nonviral expression libraries, phage libraries or bacterial libraries.

In connection with this embodiment, but also applicable for other embodiments described herein, the magnetic particles can be used to isolate and/or purify and/or concentrate nucleic acid molecules, vectors or complexes prior to transfection. Such a procedure involves the application of magnetic particles to bind the nucleic acid molecules, vectors or complexes and separating them from the surrounding medium by application of a magnetic field followed by using these purified materials for magnetofection. Similar isolation, purification and concentration procedures have been described for the isolation of, e.g., plasmids, PCR products or mRNA and are known to the person skilled in the art (see for example internet page http://www.dynalbiotech.com).

The complex of the invention can be formulated for administration in vivo.

The term "in vivo" refers to any application which is effected to the body of a living organism wherein said organism is multicellular, preferably a mammal and most preferably a human.

Accordingly, the step of bringing the complex in contact with a cell may be preceded by administering said complex in pharmaceutically acceptable form to a subject.

The term "administering" encompasses any method suitable for introducing the complex into the body of a subject so that the complex is then manoeuvrable therein. Administration of the suitable compositions may be effected in different ways, e.g., by intravenous, intraperitoneal, subcutaneous, intramuscular, topical, intradermal, intranasal, intrabronchial or oral administration.

The term "pharmaceutically acceptable form" means that the complex is formulated as a pharmaceutical composition, wherein said composition may further comprise a pharmaceutically acceptable carrier and/or diluent. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one subject depend upon many factors, including the subject's size, body surface area, age, the particular compound to be administered, sex, time and route of administration, general health, and other drugs being administered concurrently. A typical dose of active substances can be, for example, in the range of 1 ng to several grams. Applied to gene therapy, the dosage of nucleic acid for expression or for inhibition of expression should correspond to this range; however, doses below or above this exemplary range are envisioned, especially considering the aforementioned factors. Generally, the regimen as a regular administration of the pharmaceutical composition should be in the range of 1 $\mu$g to 10 mg units per day. If the regimen is a continuous infusion, it should also be in the range of 1 $\mu$g to 10 mg units per kilogram of body weight, respectively. Progress can be monitored by periodic assessment. Dosages will vary but a preferred dosage for intravenous administration of DNA is from approximately $10^6$ to $10^{19}$ copies of the DNA molecule. The compositions of the invention may be administered locally or systemically. Administration will preferably be parenterally, e.g., intravenously, although formulation for other ways of administration are within the scope of the invention; DNA may also be administered directly to the target site, e.g., by catheter to a site in an artery. Administration can, for example, also occur by direct injection into a tumor. Also within the scope of the invention is formulation for administration by aerosolization or nebulization. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition may comprise further agents such as interleukins or interferons depending on the intended use of the pharmaceutical composition.

When the complex of the invention is used in vivo, the magnetic field may be applied to a region of the body where said vector(s) shall have an effect.

For that purpose any of the above mentioned magnets which are suitable to carry out the method of the present invention may be used.

The present invention furthermore relates to the use of the complex of the invention for the preparation of a pharmaceutical composition for preventing, treating or vaccinating against a disease by way of delivery of a therapeutically useful vector, i.e. a vector having a therapeutically useful effect, comprising one or more nucleic acid molecules into a subject, said preventing, treating or vaccinating involving the transfection of cells with the vector(s) present in said complex by way of applying a suitable magnetic field.

The term "vector(s) having a therapeutically useful effect" refers to any of the above defined vectors wherein the desired function which they exert is therapeutically useful. In particular, said effect is exhibited by the nucleic acid molecule(s) comprised in said vector. Such an effect may be carried out by any of the nucleic acid molecule(s) as herein above defined, such as oligonucleotides, antisense RNA, ribozymes or coding sequences capable of giving rise to expression of a therapeutically useful protein or (poly)peptide. Corresponding nucleotide sequences are known to those skilled in the art as well as means and methods to combine them, if necessary, with sequence elements that allow for transcription and/or expression of said nucleic acid molecule in the target cell. Methods which are well known to those skilled in the art can be used to recombinantly construct such nucleic acid molecules; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989). Suitable vectors and methods for gene therapy are described in the literature and may be adapted by the person skilled in the art to apply the respective method of the present invention; see, e.g., Giordano, Nature Medicine 2, (1996), 534-539; Schper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO 94/29469; WO 97/00957 or Scharper, Current Opinion in Biotechnology 7 (1996), 635-640, and references cited therein.

The complex of the invention may be used in a method of treatment comprising

(a) the step of the method of the present invention, wherein said vector(s) have a therapeutically useful effect and wherein transfection of said cells takes place ex vivo; and
(b) administering said transfected cells to a subject.

The term "ex vivo" refers to any gene therapeutic method wherein cells are transfected in vitro with the intention to administer the transfected cells into a subject. Definition regarding "vector(s) having a therapeutically useful effect" and "in vitro" are already given above. Administration of the transfected cells may be accomplished by any of the methods known in the art. For this purpose, the cells may be admixed with pharmaceutically acceptable carrier(s) and/or diluent(s) as defined above.

Preferably, the above method of treatment comprises steps (a) and (b) and furthermore the step

(c) applying a suitable magnetic field to the region of the body where said ex vivo transfected cells shall have an effect; wherein magnetic particles have been incorporated into the cells during transfection.

The complex of the present invention opens the possibility to apply the effect of local enrichment in a body also to ex vivo gene therapy. In order to apply this feature, the magnetic particles of the complex have to be incorporated into the cells, a condition which is, although not inherently, encompassed by the method of the present invention. For being incorporated, the magnetic particles should be of a size not exceeding the low micrometer range, preferably they should not be larger than 1000 nm. Application of the magnetic field may be carried out as described above for in vivo gene therapy.

In a further embodiment, the invention relates to a method for the preparation of a complex useful for transfecting a cell, said method comprising the step of linking together one or more vectors comprising one or more nucleic acid molecules and one or more magnetic particles which are coupled with one or more oligo- or polycations or oligo- or polyanions, wherein the components of said complex are assembled by salt-induced aggregation.

For carrying out this method, every necessary information is given to the person skilled in the art above, where the complex and, in particular, the vectors, magnetic particles and the modes of linking them are described in connection with the transfection method of the invention.

Furthermore, an embodiment of the invention relates to a complex useful for transfecting a cell, said complex comprising

(a) one or more vectors comprising one or more nucleic acid molecules; and
(b) one or more magnetic particles which are coupled with one or more oligo- or polycations or oligo- or polyanions;

wherein said magnetic particle(s) and said vector(s) are assembled in the complex by salt-induced aggregation or obtainable by the method for its preparation as described above.

Said complex corresponds to any of the characteristics given above with regard to the complex as well as to the vectors and magnetic particles comprised in connection with the transfection method of the present invention.

The invention relates in a further embodiment to a pharmaceutical composition comprising the complex of the invention and optionally a pharmaceutically acceptable carrier and/or diluent.

The features of the pharmaceutical composition are already given when defining the term "pharmaceutically acceptable form". Moreover, the pharmaceutical composition of the invention preferably may be in a lyophilized form, optionally admixed with a sugar, such as sucrose or dextrose, in an amount which yields a ready for use solution having a physiological concentration. The composition may also be in a form of a cryoconcentrate or a cooled solution.

The above-mentioned use of the complex of the present invention for the preparation of a pharmaceutical composition related to the delivery of a therapeutically useful vector into a subject.

The term "delivery" refers to the introduction of a therapeutically useful vector into a subject by way of administering the vector directly or via in vitro transfecting of cells and applying the cells to the subject, according to the in vivo and ex vivo application of the complex of the invention described above.

In addition to the above mentioned prepartion of a pharmaceutical composition for medical use, the provisions of the invention may be prepartion of a pharmaceutical composition for use to prevent diseases that have not yet broken out and to vaccinate, as a special form of preventing, by way of enabling the immune system to protect against certain pathogens.

The person skilled in the art is capable to select suitable nucleic acid molecules that may be used for preventive or vaccination applications or may be taken from the literature.

The disclosure also relates to the use of magnetic particles or the complex of the present invention for transfecting a cell by bringing one or more vectors linked to one or more of said magnetic particles 'or said complex in contact with a cell by applying a suitable magnetic field. Preferably, said use is made for screening methods involving high-throughput transfection as mentioned above.

Furthermore, the invention relates to the use of the complex of the present invention for transfecting a cell in vitro, wherein the complex is brought in contact with a cell by applying a suitable magnetic field.

In addition to the surprising positive effects with respect to transfection efficiency that have been achieved by applying a magnetic field, it could be observed that the complexes of the present inventions result in an enhanced transfection efficiency compared to the same construct without a magnetic particle when no magnetic field is applied. Such an effect is for instance demonstrated in Examples 7, 8, 9 and 10.

Another embodiment of the present invention relates to a kit comprising the complex of the present invention, and optionally magnetic particles and/or vectors comprising one or more nucleic acid molecules and/or vector components suitable for taking up a nucleic acid molecule to be expressed and instructions for applying a method of the present invention, and optionally cells and/or gene therapeutical adjuvant(s) and magnets useful for applying the above-described methods.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http:llwww.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

The present invention is further described by reference to the following non-limiting figures and examples.

The Figures show:

**Figure 1** DNA-binding capacity of polyethylene imine-coated-magnetic particles (fIMAG-PEI). Saturation of binding is approached above particle to DNA weight ratios of 2. However, optimal transfection is only observed at higher ratios (see Fig. 3).

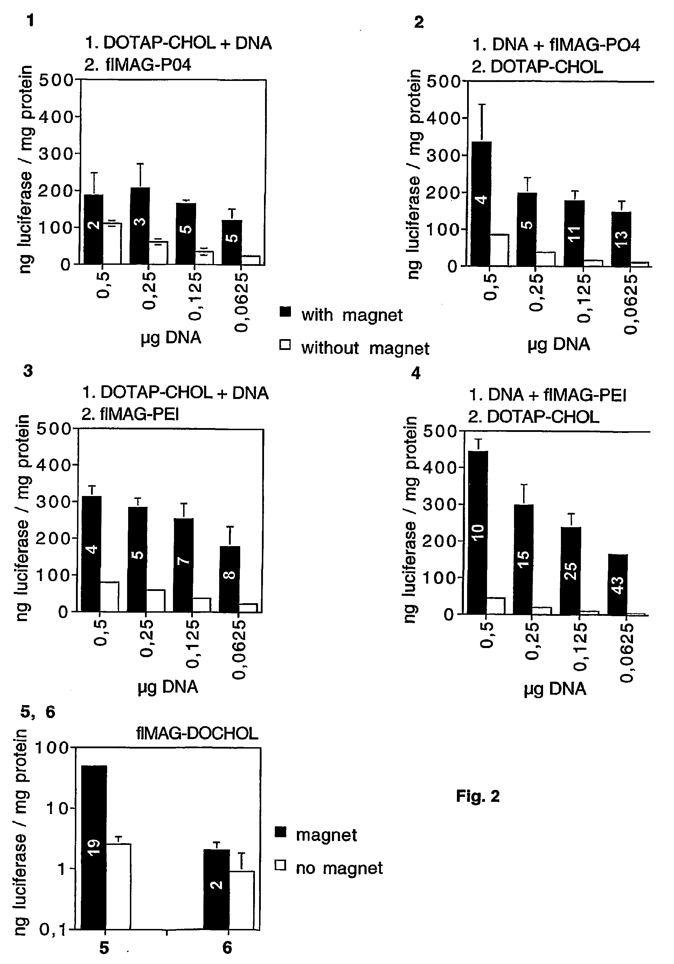

**Figure 2** Comparisons of transfection efficiencies of different complexes of the invention, carried out with and without applying a magnetic field. The complexes used differ with regard to the coating of the magnetic particle (end-standing phosphate groups (= $PO_4$) on a starch coating: 2.1, 2.3, 2.5 and 2.6, or PEI: 2.2 and 2.4); with regard to mixing order and concentration of the DNA complexes used (in Figure 2.1 to 2.4) or the amount of fIMAG-DOCHOL liposomes to which DNA was added (Figure 2.5 and 2.6). Toxicity was high at the highest concentrations of DNA. Consequently, the protein levels in these wells were low, resulting in an only apparently higher specific transfection efficiency with higher amounts of DNA. Absolute luciferase levels were virtually unaffected by using lower amounts of DNA. The white-colored numbers on the black bars indicate the fold-enhancement of transfection by the action of the magnetic field, exemplified by luciferase activity.

**Figure 3**   Comparisons of transfection efficiencies obtained with complexes of naked DNA and fIMAG-PEI with and without applying a magnetic field. Black-colored numbers on the gray bars indicate fold-increase of the transfections with magnetic field compared to those without magnetic field.

**Figure 4**   Comparisons of transfection efficiencies obtained with complexes comprising PEI and the protective copolymer (PROCOP) P6YE5C (Finsinger et al. 2000). White-colored numbers on the black bars indicate the fold-increase of transfection rate induced by a magnetic field.
<u>Formulations applied:</u>

1. PEI-DNA added to fIMAG-PEI, finally coated with protective copolymer P6YE5C. Complexes in 5 % glucose.
2. PEI-DNA added to fIMAG-PEI$^{Stav}$, finally coated with protective copolymer P6YE5C. Complexes in 5 % glucose.
3. PEI$^{biotin}$-DNA added fIMAG-PEI stock, finally coated with protective copolymer P6YE5C. Complexes in 5 % glucose.
4. PEI$^{bioin}$-DNA added to fIMAG-PEI$^{Stav}$ stock, finally coated with protective copolymer P6YE5C. Complexes in 5 % glucose.
5. Pre-mixing of PEI and fIMAG-PEI at high concentration, then addition of DNA. Finally coated with protective copolymer P6YE5C. Complexes in 5 % glucose.
6. Pre-mixing PEI and fIMAG-PEI$^{Stav}$ at high concentration, then addition of DNA. Finally coated with protective copolymer P6YE5C. Complexes in 5 % glucose.
7. Pre-mixing of PEI$^{biotin}$ and fIMAG-PEI at high concentration, then addition of DNA. Finally coated with protective copolymer P6YE5C. Complexes in 5 % glucose.
8. Pre-mixing of PEI$^{biotin}$ and fIMAG-PEI$^{Stav}$ at high concentration, then addition of DNA. Finally coating with protective copolymer P6YE5C. Complexes in 5 % glucose.
9. 12: Same as 1-4, but complexes mixed in HBS. With the types of complexes applied in Example 4, no beneficial influence of a biotin-streptavidin bridge between individual complex components is observed.

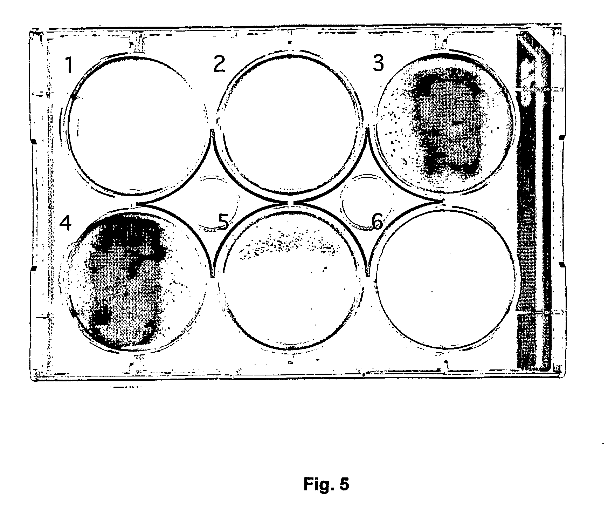

**Figure 5**   Transfection results of NIH3T3 cells obtained with PEI$^{biotin}$-DNA associated with inactivated adenovirus-particles (Adv$^{biotin}$) and complexes with fIMAG-PEI (wells 2 and 3) or fIMAG-PEI$^{stav}$ (wells 4 and 5). Wells 2 and 5 without magnet, wells 3 and 4 with magnet. Well 6 is empty, well 1 is untransfected cells. β-galactosidase activity (dark color in wells 3 and 4) indicates effective transfection. The dark rectangular shape in wells 3 and 4 shows the area under which the magnet was positioned. The example demonstrates that with help of a magnetic field, transfection can be directed to a particular area within a larger cell population.

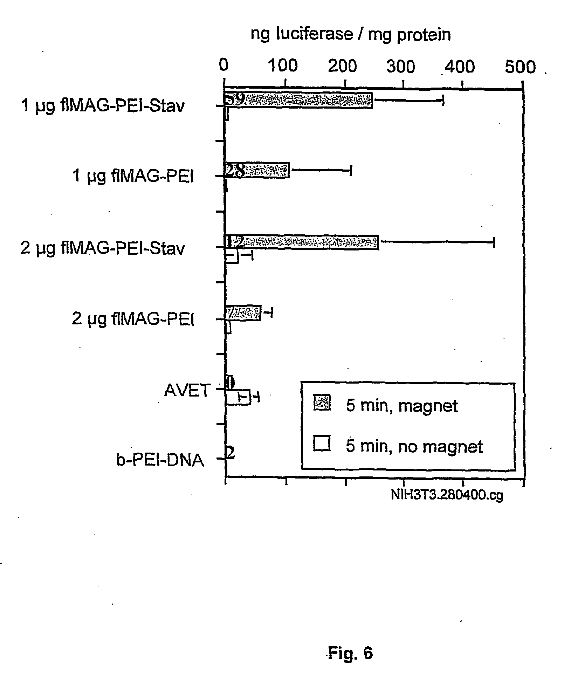

**Figure 6**   Adenovirus-enhanced transfection of NIH3T3 cells with PEI$^{biotin}$-DNA complexes containing magnetic particles with luciferase as reporter gene. The DNA complexes were prepared in HBS. The adenovirus used is chemically inactivated and biotinylated. An enhancing effect of a biotin-streptavidin bridge between individual components of the complex is clearly evident. The gray bars indicate transfection levels under the influence of a magnetic field, the white bars without magnetic field. The numbers at the bottom of the gray bars indicate -fold enhancement by the magnetic field. For comparison, transfection with PEI$^{biotin}$-DNA (b-PEI-DNA) or the same associated with an adenovirus but without magnetic particles yields significantly lower rates (rows 5 and 6).

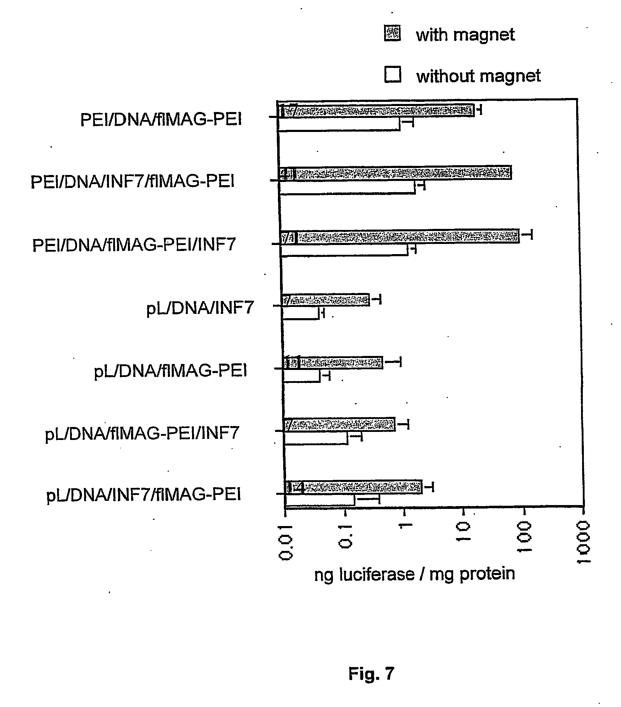

**Figure 7**   Transfection of NIH3T3 cells with PEI-DNA and pL-DNA complexes containing magnetic particles, enhanced by the endosomolytic peptide INF7. The gray bars indicate transfection levels under the influence of a magnetic field, the white bars without magnetic fields. The numbers at the bottom of the gray bars indicate -fold enhancement by the magnetic field. Apart from an additional enhancement by the influence of the peptide, it is also evident, that even in the absence of a magnetic field, the incorporation of magnetic particles into DNA complexes enhances transfection (compare row 4 to rows 5 - 7).

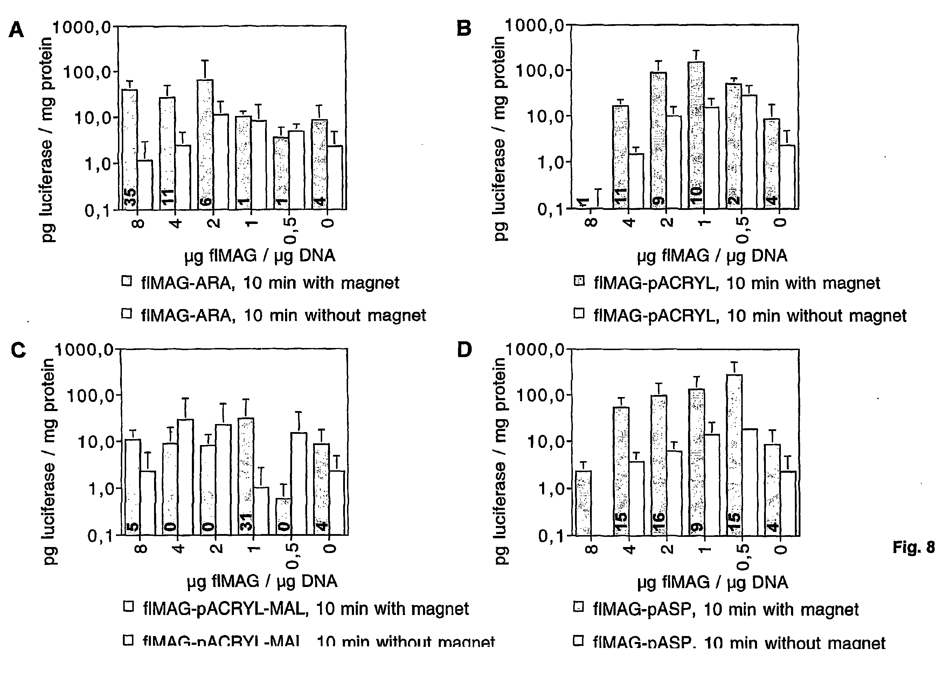

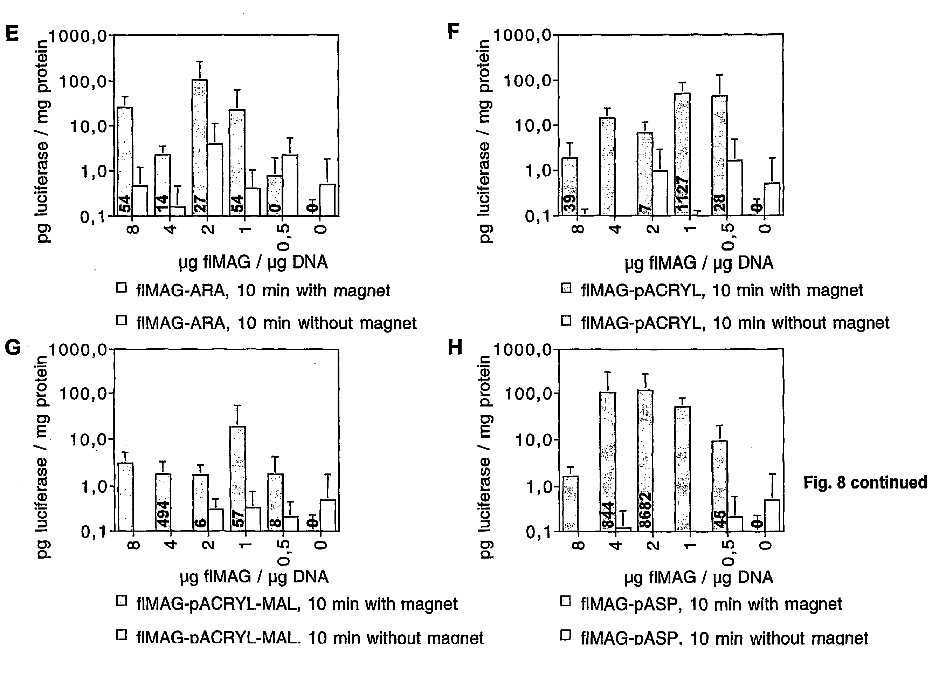

**Figure 8**   Transfection of NIH3T3 cells and HepG2 cells with PEI-DNA complexes containing increasing amounts of negatively charged magnetic particles. The complexes were mixed in 5 % glucose. The gray bars indicate transfection levels under the influence of a magnetic field, the white bars without magnetic field. The numbers at the bottom of the gray bars indicate -fold enhancement by the magnetic field. The figure shows that different particle types can have different influences on transfection, and this again differing from cell line to cell line. With most compositions, an enhancement by a magnetic field is observed. Apart from that, it is also evident, that even in the absence of a magnetic field, the incorporation of magnetic particles into DNA complexes can enhance transfection (the bars to the furthest right in each graph show transfection without magnetic particles).

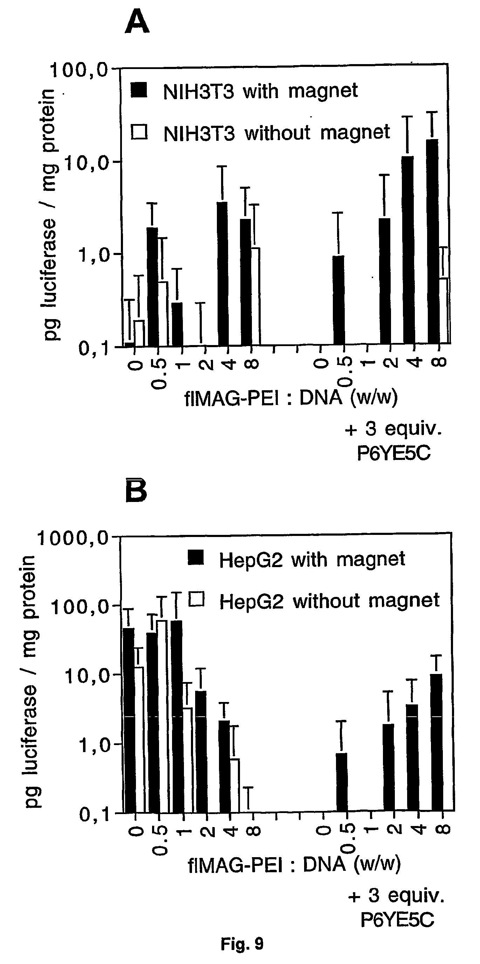

**Figure 9**      Transfection of NIH3T3 cells and HepG2 cells by calcium phosphate co-precipitation in the presence and in the absence of magnetic particles. Additionally, the protective copolymer P6YE5C was incorporated into the particles in order to stabilize particle size (right parts of the graphs). Again, the enhancement by a magnetic field is clearly evident for most compositions, particularly if the vectors are stabilized with P6YE5C.

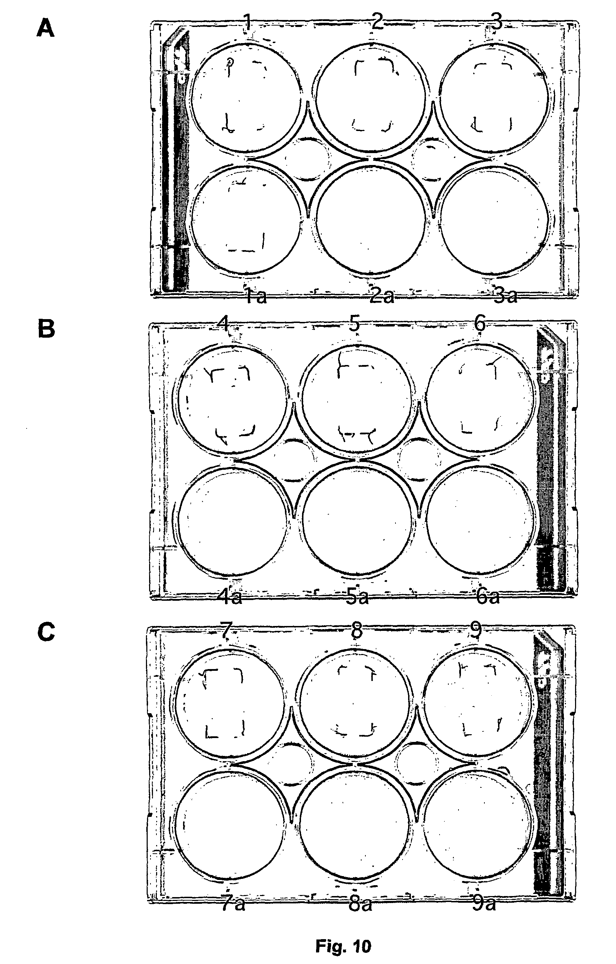

**Figure 10**      Transduction of NIH3T3 cells with a recombinant adenovirus carrying the lacZ gene. (AdLacZ)
1. Control: PBS buffer
1a Control: AdLacZ, without magnetic particles
2. AdLacZ + fIMAG-PEI, added to plasmid DNA (coding for GFP), added to PEI; with magnet, incubation for 20 min.
2a = 2 without magnet
3. chemically inactivated, biotinylated adenovirus (without lacZ) + fIMAG-PEI, added to plasmid DNA (coding for GFP), added to PEI; with magnet, incubation for 20 min.
3a = 3 without magnet
4. AdLacZ + 0.75 $\mu$g fIMAG-PEI per well; with magnet, incubation for 20 min.
4a = 4 without magnet.
5. AdLacZ + 1.5 $\mu$g fIMAG-PEI per well; with magnet, incubation for 20 min.
5a = 5 without magnet.
6. AdLacZ + 3 $\mu$g fIMAG-PEI per well; with magnet, incubation for 20 min.
6a = 6 without magnet.
7. AdLacZ + 6 $\mu$g fIMAG-PEI per well; with magnet, incubation for 20 min.
7a = 7 without magnet.
8. AdLacZ + 12 $\mu$g fIMAG-PEI per well; with magnet, incubation for 20 min.
8a = 8 without magnet.
9. AdLacZ + 24 $\mu$g fIMAG-PEI per well; with magnet, incubation for 20 min.
9a = 9 without magnet. The figure demonstrates that also viral gene delivery can be enhanced with magnetic particles under application of a magnetic field. Furthermore, it demonstrates, that gene delivery can be directed to a particular area within a larger cell population. In addition, enhancement of gene delivery is also evident without the application of a magnetic field (wells 8a and 9a, compared to well 1a). Most notably, no functional gene delivery is observed without magnetic particles (well 1a), as the applied cell line (NIH3T3) is not infected by the adenovirus. With the help of magnetic particles, the viral particles can bind to the cells and are consequently internalized leading to functional transfection.

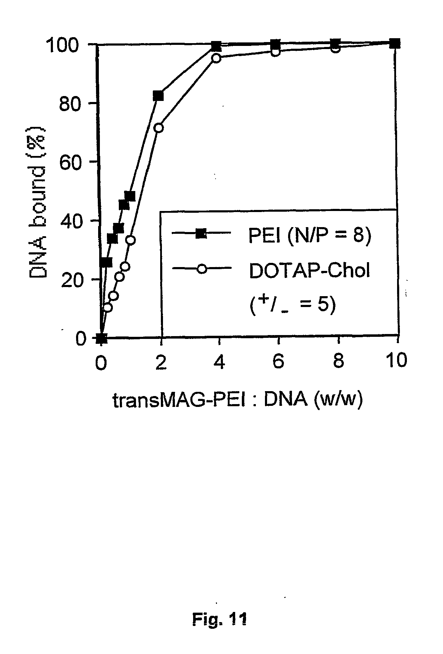

**Figure 11**      DNA binding isotherms of ternary complexes of transMAG-PEI / DNA /PEI and transMAG-PEI / DNA / DOCHOL Ternary complexes were prepared in water. Salt-induced aggregation was initialized by mixing with 1/4 volume of 600 mM sodium chloride and continued for 20 - 30 minutes followed by 30 min magnetic sedimentation. Radio-labeled DNA was quantified in the supernatants of triplicates. The figure demonstrates that at the frequently used transMAG-PEI:DNA ratio of 2 (w/w) approx. 70 - 80 % of the DNA dose are magnetically sedimentable, which means associated with magnetic particles. The data points are averages of triplicates $\pm$ standard deviation (error bars too small to be seen).

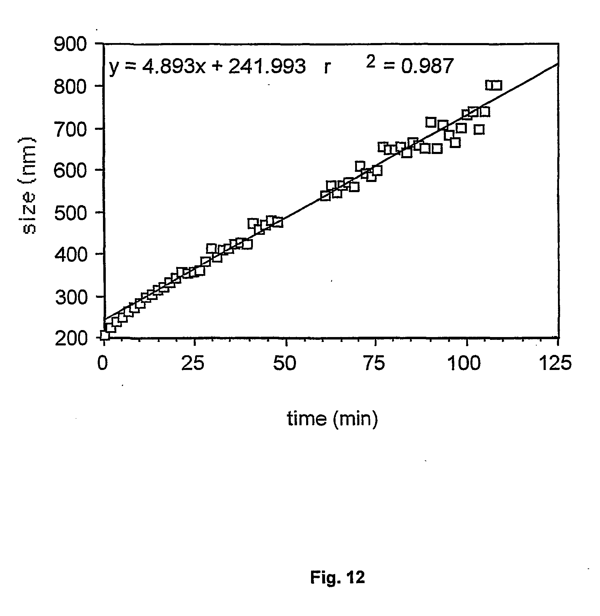

**Figure 12**      Salt-induced aggregation of ternary complexes of transMAG-PEI / DNA / PEI. The ternary complex was prepared in water (10 $\mu$g DNA/ml final concentration) and had a size of 217.0 $\pm$ 2.0 nm before salt addition. Salt induced aggregation was induced by adjusting the ionic strenght to 150 mM sodium chloride. The ternary complex aggregates with approx. linear kinetics remaining in the sub-micrometer range within two hours. The linear regression line is shown together with its equation which allows to pre-calculate particle sizes at given time points after induction of aggregation.

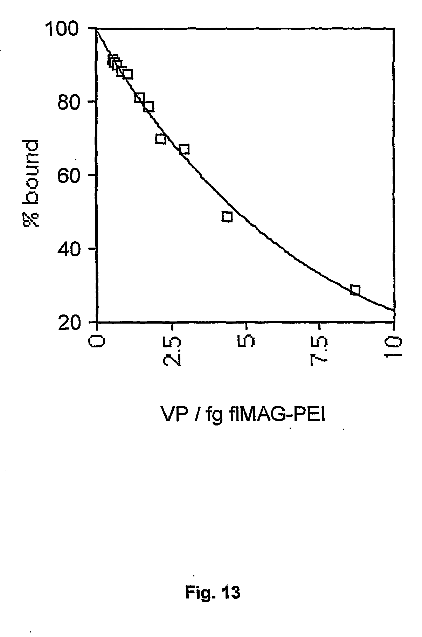

**Figure 13**      Binding isotherm of adenovirus and transMAG-PEI Iodine-125-labeled adenovirus and magnetic particles were mixed at ratios covering the range and under conditions applied in gene transfer experiments. After 20 min incubation, the mixtures were subjected to magnetic sedimentation for 1 hour. Unbound virus was determined in supernatants of triplicates using a gamma counter. The figure shows that under the conditions used in the gene transfer experiments, 70 and more percent of virus are associated with magnetic particles. A logarithmic curve fit has been applied to the data points which are averages of triplicates $\pm$ standard deviation (error bars too small to be seen).

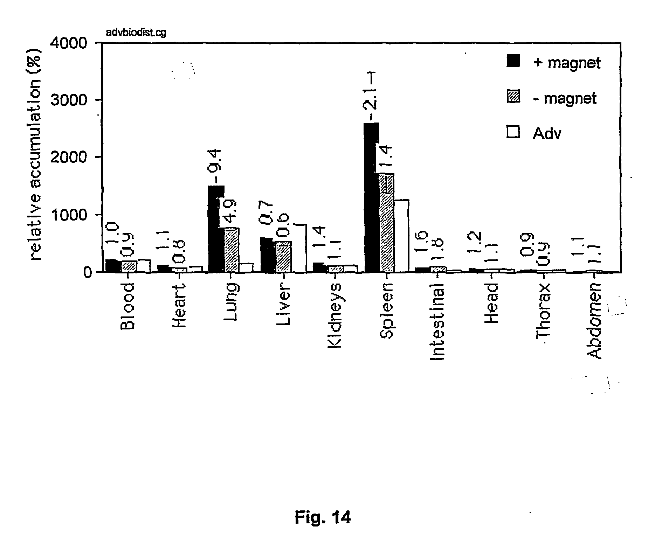

**Figure 14**     Biodistribution of $^{125}$I-labeled adenovirus in mice

Approx. $6.5 \times 10^8$ viral particles, associated with 0.3 $\mu$g transMAG-PEI (corresponding to approx. 73 % virus binding according to the binding isotherm) were injected in mice via the tail veins. A subset of the animals (n = 3) had a magnet block attached to the right sides of their chests for one hour after injection. Virus in the various organs was determined using a gamma counter. The relative accumulation in per cent (y-axis) presented here is the quotient of counts (CPM) per organ weight and total recovered dose (CPM) per total organ weight multiplied by 100 (total organ weight = sum of the weights of all organs). While the highest percentage of the absolute dose accumulated in the liver, the specific accumulation per tissue weight is most pronounced in the spleen. Association of the virus with magnetic particles results in a non-significant relative accumulation in the spleen compared to "naked" virus and a 5-fold relative accumulation in the lung. If, in addition, the magnetic field was applied to the area of the lungs, a further slight accumulation was observed in the spleens for reasons not known and a 10-fold accumulation compared to "naked" virus was observed in the lungs. The experiment demonstrates the applicability of magnetofection in vivo.

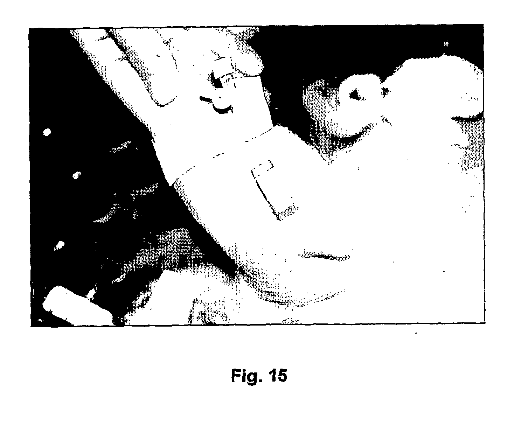

**Figure 15**     Magnetofection in ear veins of pigs

A variety of cardiovascular diseases are promising indications for gene therapy. Therefore, the vasculature is an important target for localized rather than systemic gene delivery. Currently, localized transfection of blood vessels is achieved with catheterization and/or clamping techniques (Isner et al. 1996). Magnetofection can provide a useful complementation to such techniques or even an alternative. This is supported by the results obtained when transMAG[PEI]+DNA+PEI (DNA dose 500 $\mu$g) was infused into the right and left ear veins of pigs and a Nd-Fe-B permanent magnet block was attached above the right veins proximal to the injection sites. No reporter gene expression (luciferase) was observed in the control blood vessels (left ears) and distal from the magnet positions (right ears), while reproducible, though variable (741,897 $\pm$ 693,298 RLU / g), luciferase expression was found in all vein samples which were under direct influence of the magnetic field. No luciferase signal (light emission) was found in samples of any other major organ. The figure shows the anesthetized animal with injection site and attached magnet.

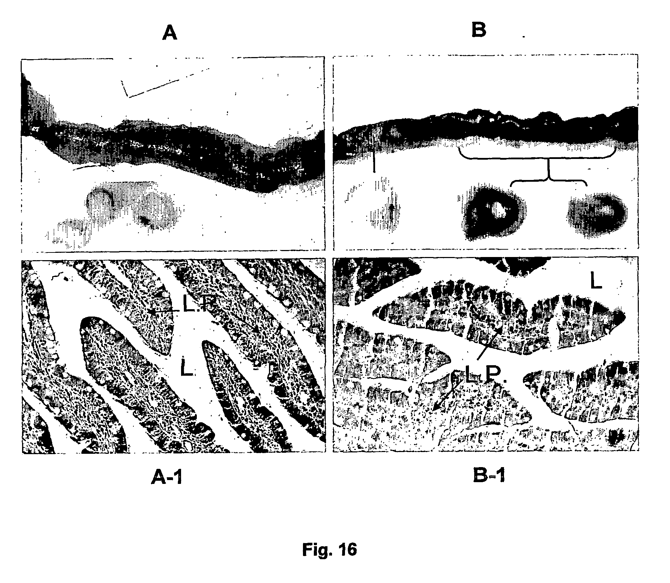

**Figure 16**     Nonviral magnetofection in the ilea of rats (A)-(B) DNA-transMAG-PEI was applied to the ilea of rats in the absence (A) and under the influence of a magnetic field for 20 min (B). X-Gal staining performed 48 hours after gene delivery reveals efficient gene delivery only in the presence of the magnet (B), both on the macroscopic level (upper panel) and on the microscopic level (lower panel). Upper panel: intestinal tubes after X-Gal stain. Inserts: cross sections of tubes embedded in paraffin. Lower panel: Paraffin sections counter-stained with eosin, 400 x magnification. X-Gal staining is found in the lamina propria. L: lumen; L.P. lamina propria.

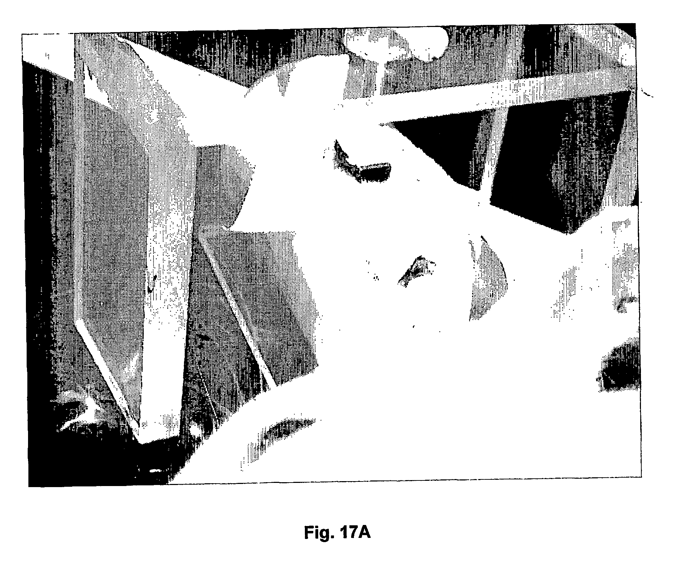

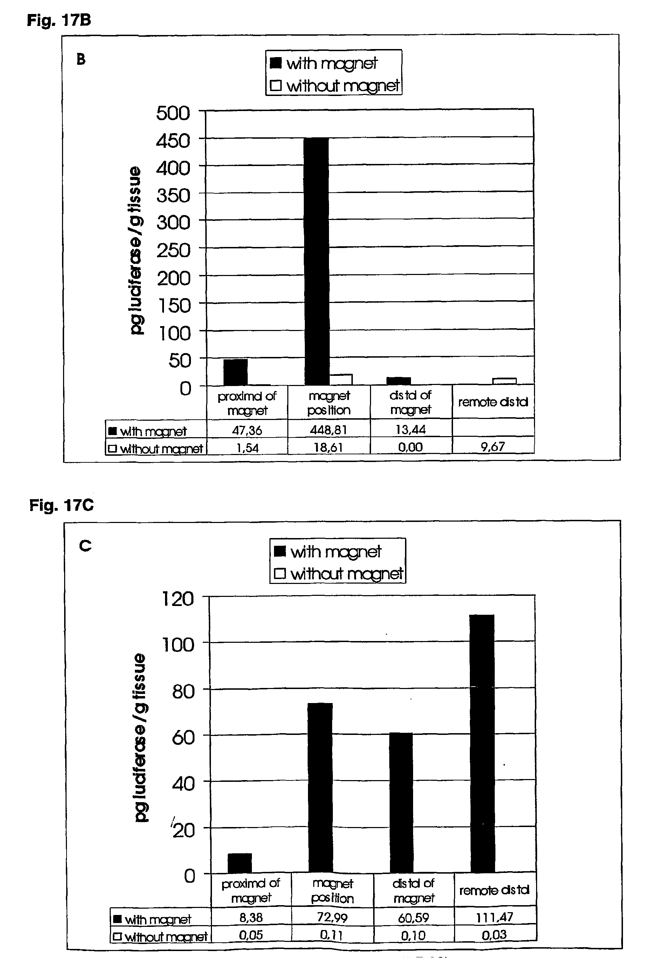

**Figure 17**     Magnetofection in the ear arteries of rabbits

(**A**) Experimental setup for the administration of gene vectors. In the foreground, the injection site can be seen and more distal the placement of the magnet block.
(**B**) Vector preparation with 2 $\mu$g transMAG-PEI 16/1 per $\mu$g of DNA in 5 % glucose injected in the ear artery. A 24-fold higher reporter gene expression was found at the magnet section of the artery where the magnet was placed as compared to the analogous section of the control artery (left ear) which received the same vector but without magnet positioned.
(**C**) Vector preparation with 1 $\mu$g transMAG-PEI per $\mu$g DNA in 150 mM sodium chloride injected in the ear artery. Reporter gene expression was found at the magnet position and in distal sections of the artery, while no expression or little expression (only in distal sections) was found in the control artery. The expression in the distal sections of both magnet ear and control ear may reflect the influence of vector particle size on gene delivery. As opposed to (B), the vector was prepared by salt-induced aggregation implying a particle size of several hundred nm at the time of vector administration. As a consequence, these particles may get stuck in the capillaries downstream of the injection sites.

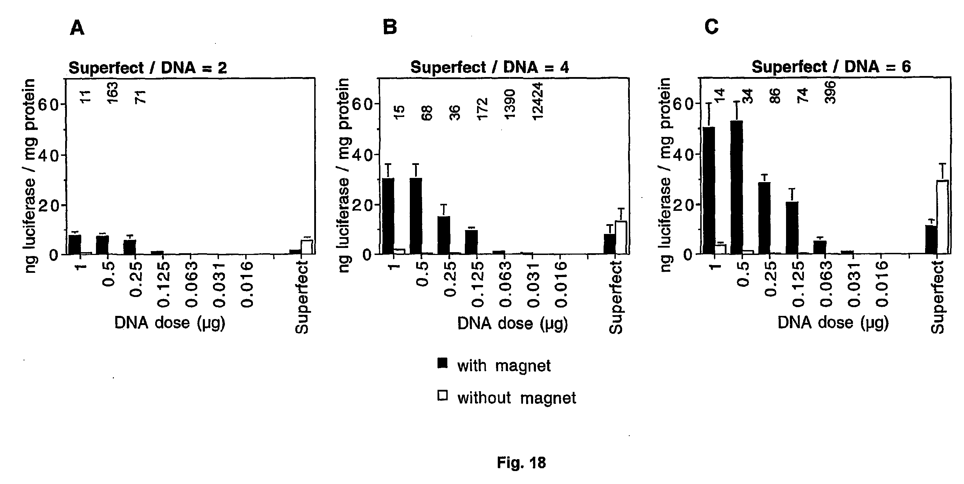

**Figure 18**     Transfection of NIH3T3 cells with Superfect $\pm$ transMAG-PEI (Example 19) The cells were transfected with transMAG-PEI/DNA/Superfect complexes prepared by salt-induced aggregation. The Superfect:DNA ratios (N/P) were 2, 4, and 6. The columns labeled "Superfect" to the far right show the results of standard transfections with Superfect without magnetic particles at the respective N/P ratios and at the DNA dose of 1 $\mu$g/well. Compared to these controls, association with magnetic particles led to a reduction of gene transfer efficiencies (white bars to the furthest left of each graph). This reduction, however, was overcompensated by the application of the magnetic field (black bars). The numbers above the columns show the -fold enhancement over transfection with magnetic particle-containing complexes without application of the magnetic field. In the presence of the magnetic field, there is a strong dose-response dependence.

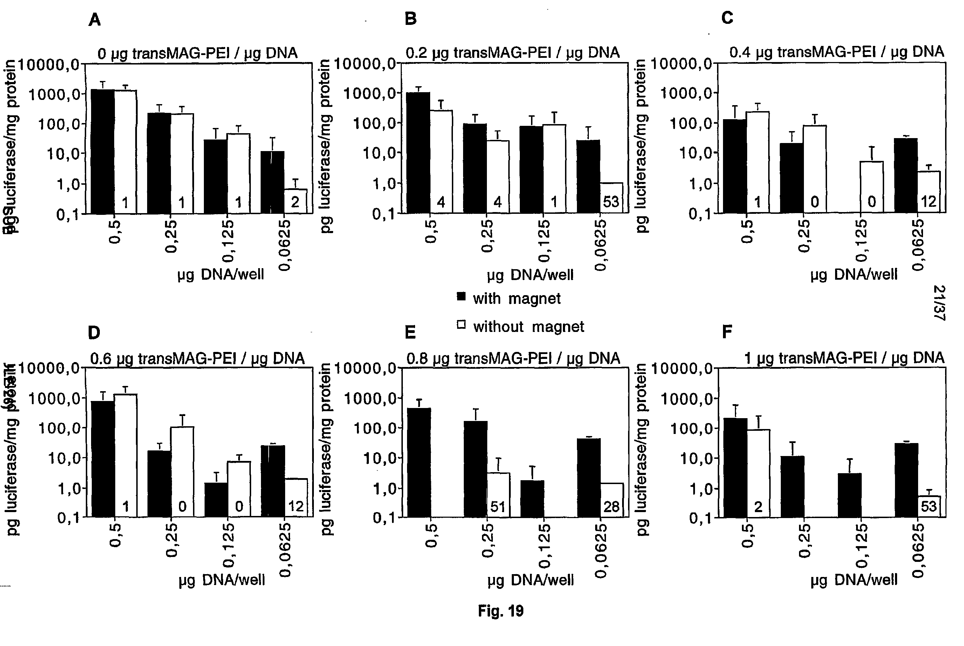

**Figure 19**   Transfection of NIH3T3 cells with Superfect $\pm$ transMAG-PEI to study the influence of a magnetic field apart from its role in particle concentration at the target cell (Example 20)
Cells were transfected with transMAG-PEI-containing Superfect-DNA complexes (N/P = 6) prepared by salt-induced aggregation. The cells were incubated with the complexes on two separate plates for 20 min, followed by washing with medium. Subsequently, one of the two plates was positioned on the magnetic plate for 40 minutes. This procedure warrants that cells on both plates bind, on average, the same amount of gene vectors during the first 20 min of incubation. Uptake of gene vector is entirely dependent on natural transport processes for the plate without influence of a magnetic field, while cell entry and transfection is influenced by the magnetic field for the other plate. The data shows that there is a consistent strong enhancing effect of the magnetic field particularly at the lowest DNA dose. At the higher DNA doses, a threshold amount of transMAG in the DNA complexes appears to be required in order to observe the enhancing effect of the magnetic field (0.8 $\mu$g of transMAG per $\mu$g of DNA under the settings applied here). The bars show averages of quadruples $\pm$ standard deviation. Black bars show results with magnetic field applied, whithe bars without magnetic field.

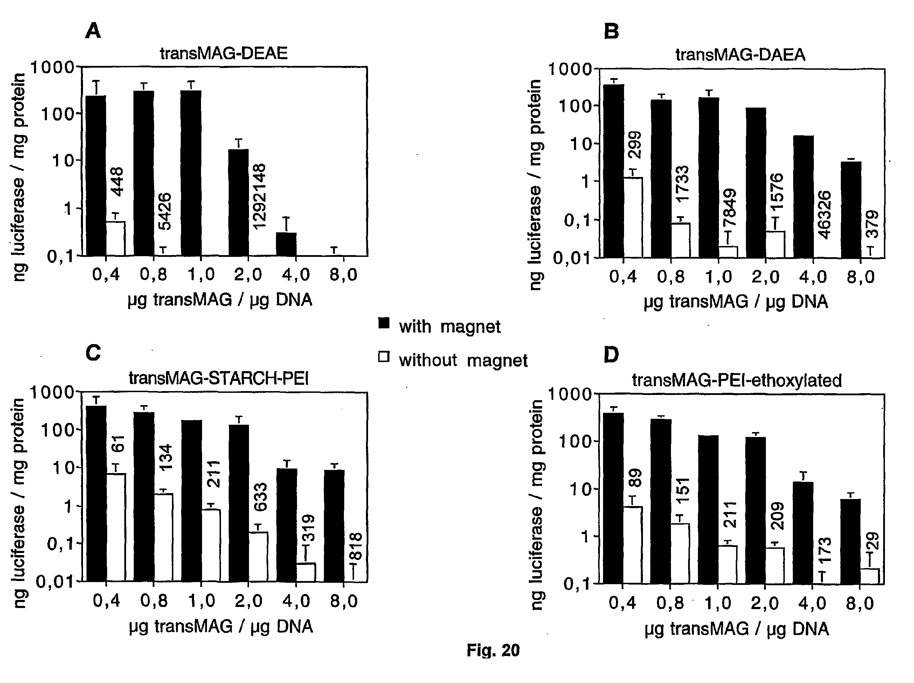

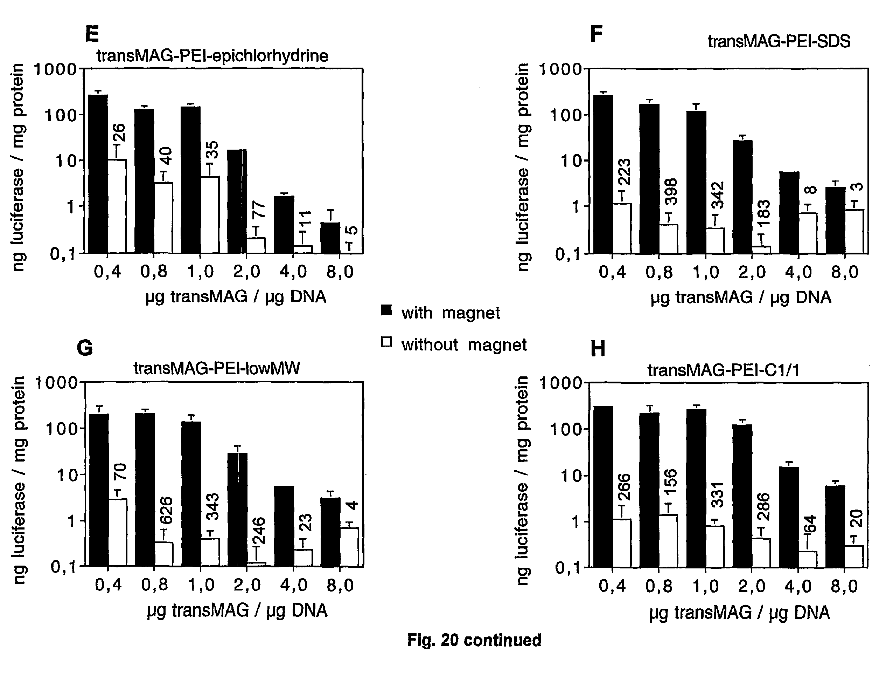

**Figure 20**   Transfection of CHO-K1 cells with PEI-DNA + transMAGs with various polycationic surface coatings (Example 21)
Cells were transfected with DNA complexes prepared by salt-induced aggregation containing free PEI at an N/P ratio of 8 and transMAGs with various surface coatings. The transMAG content of the complexes was titrated. The bars show averages of quadruples $\pm$ standard deviation. Black bars show results with magnetic field applied, whithe bars without magnetic field. Numbers above the bars represent -fold enhancements by application of the magnetic field. The figures show that any of these magnetic particles can mediate magnetofection to similar orders of magnitude. The dependence of transfection efficiency on magnetic particle content, however, varies between formulations. For all formulations, there is a decrease of transfection efficiencies above transMAG:DNA ratios of 1, probably due to increasing toxicity to the cells.

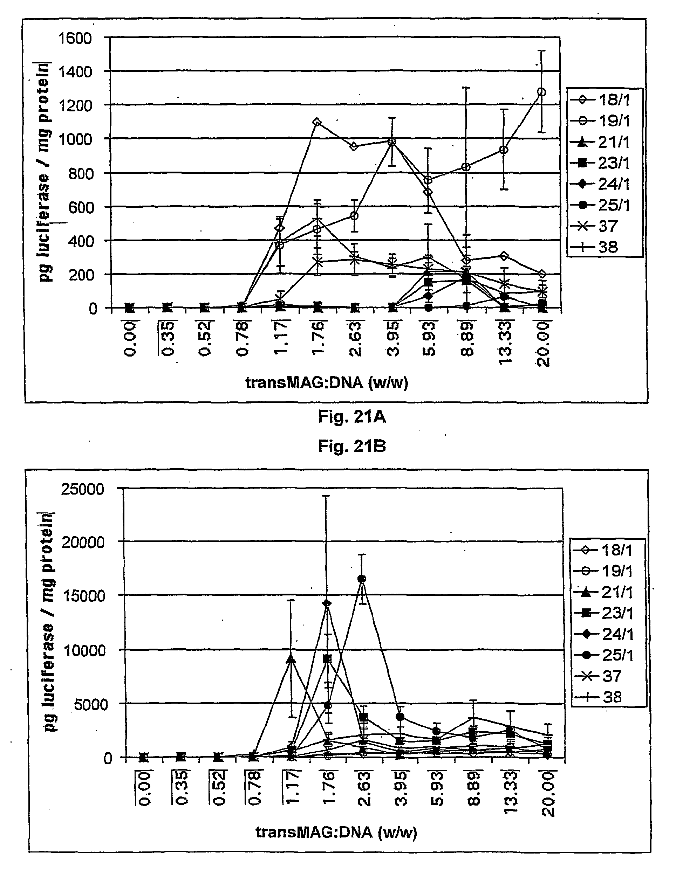

**Figure 21**   Transfection of NIH3T3 cells with DNA + transMAGs with various PEI surface coatings. Vector preparation in glucose and salt-containing solutions. Titration of optimal transMAG:DNA ratios (Example 22)
(A) Complexes prepared in 5 % glucose; (B) complexes prepared in 150 mM sodium chloride (salt-induced aggregation). The data points show averages of triplicates $\pm$ standard deviations. Both graphs show gene delivery under the influence of the magnetic field.
The titration shows that the gene transfer efficiency depends drastically on whether the vectors are prepared in salt-containing solution or not. Two particle classes can be discriminated: transMAGs 18/1, 19/1, 37, and 38 are superior under salt-free conditions but comparatively inactive under salt containing conditions. TransMAGs 21/1, 23/1, 24/1, and 25/1 display the opposite behaviour. These particles carry a surface coating of 800 kD PEI (Fluka) while the other particle class carry low molecular weight PEI (2 kD; Aldrich) surface coatings. TransMAGs 21/1, 23/1, 24/1, and 25/1 are a good choice for transfections where no third component besides DNA and transMAG ought to be applied.

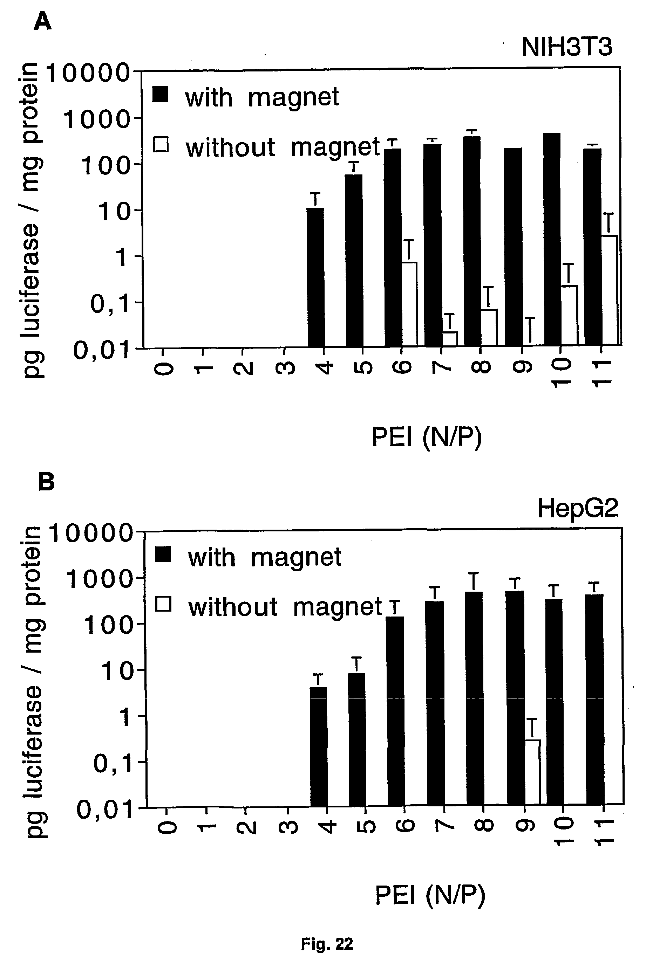

**Figure 22**   Transfection NIH3T3 and HepG2 cells with transMAG-pASP-DNA and various amounts of PEI (Example 23)
DNA complexes containing a constant amount of transMAG-pASP (1 $\mu$g per $\mu$g DNA) and increasing amounts of PEI were prepared by salt-induced aggregation. The bars show averages $\pm$ standard deviations of quadruples. Black bars are transfections under the influence of a magnetic field, white bars without magnetic field. In both cell lines, transfection efficiency is dependent on the PEI content of the DNA complexes. Optima are found around an N/P ratio of 8. In this particular experiment, very little, if any, transfection was found without application of a magnetic field.

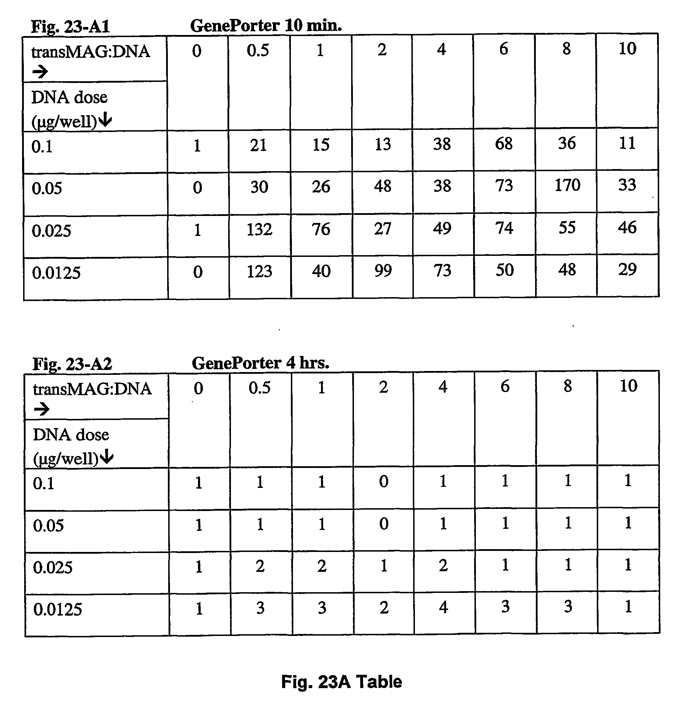

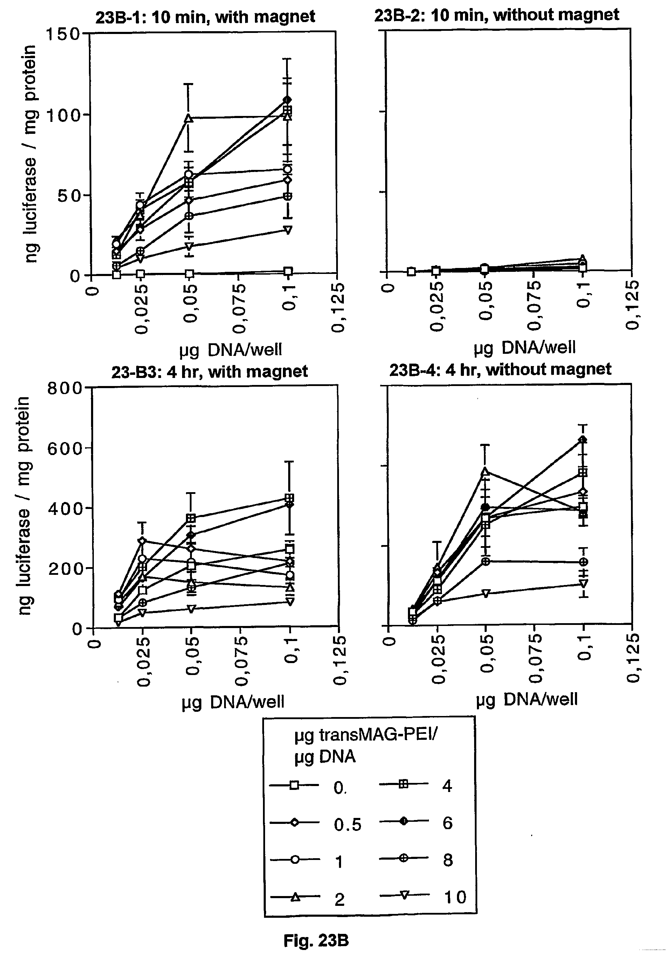

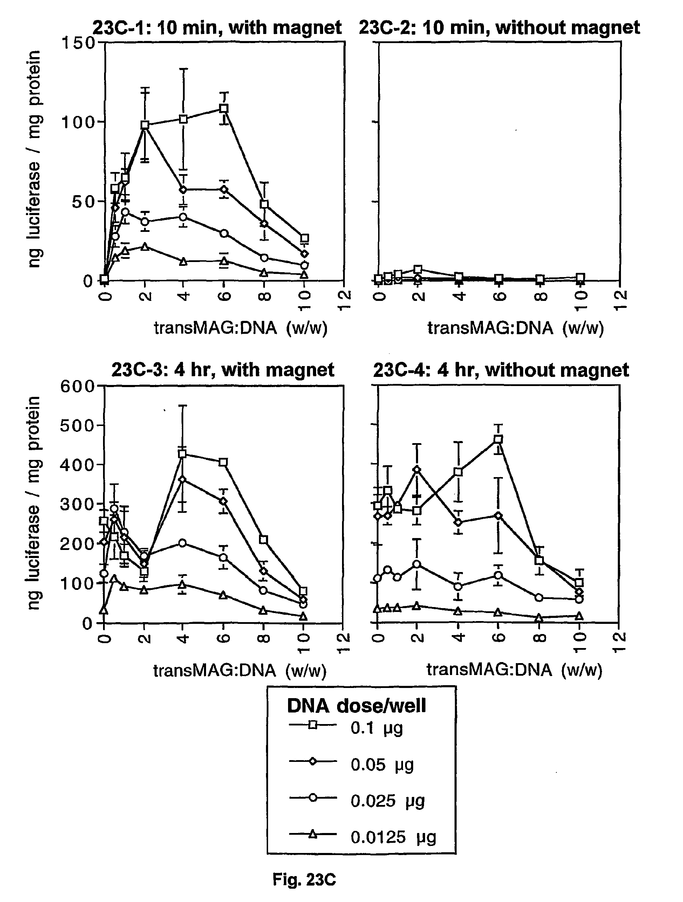

**Figure 23**   Transfection of CHO-K1 cells with GenePorter-DNA $\pm$ transMAG-PEI (Example 24)

DNA complexes containing increasing amounts of transMAG-PEI and a constant amount of GenePorter transfection reagent were prepared essentially according to the instructions of the manufacturer of the transfection reagent with the exception that DNA was pre-incubated with transMAG-PEI. DNA complexes were serially diluted in order to obtain a two-dimensional dose-response profile. Cells were incubated with vector formulations for 10 min or 4 hrs, respectively, in the presence and in the absence of a magnetic field in order to derive data on the kinetics of transfection.

(**A**) Tables showing the -fold enhancements of transfection by application of the magnetic field. The data shows that enhancements are strong after 10 min of incubation, moderate and apparent only at lowest vector doses upon long-term incubation. Also during short-term incubation, enhancements are dependent on the vector formulation. At the higher DNA doses at high transMAG content, the vector formulations were toxic to the cells, particularly upon long-term incubation with magnetic field.
(**B**) Dose-response profiles in terms of DNA dose. The data points represent the averages of triplicates $\pm$ standard deviations. The strong dose-response relationship at 10 min only in the presence of the magnetic field but not in its absence demonstrates the drastic influence of the field on transfection kinetics.
(**C**) Dose-response profiles in terms of transMAG dose. Transfection efficiency decreases at the higher transMAG doses probably due to toxicity. A transMAG to DNA ratio of 4 turns out useful at all DNA doses. The data points represent the averages of triplicates $\pm$ standard deviations.

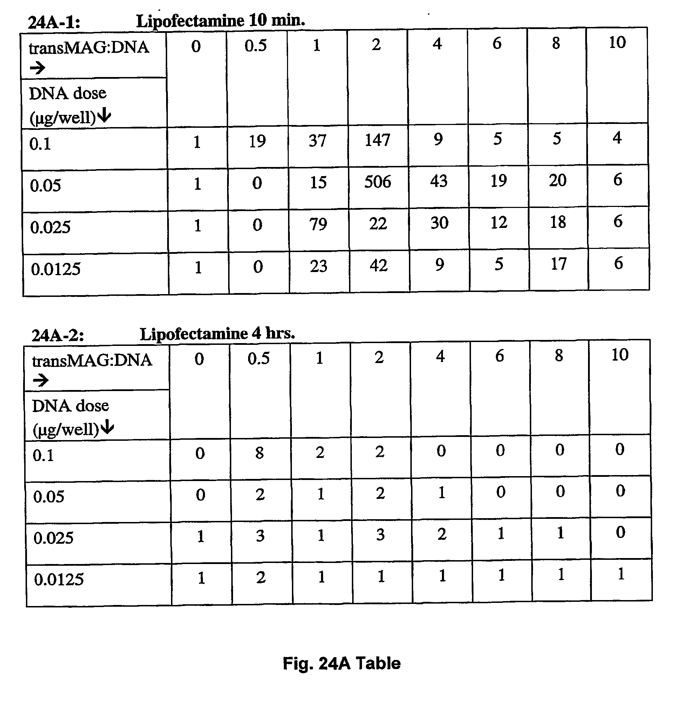

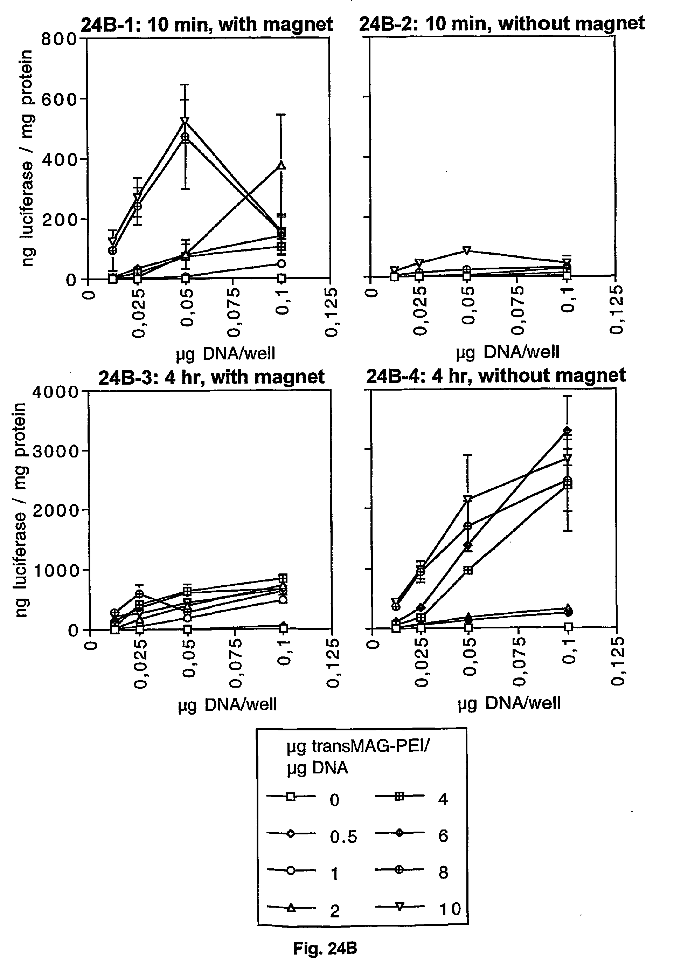

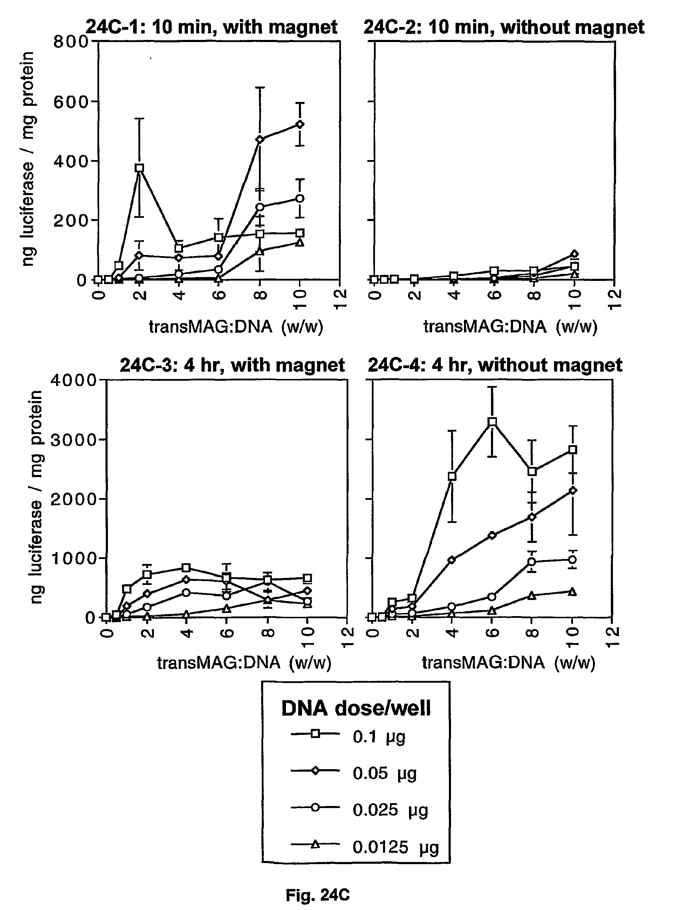

**Figure 24**   Transfection of CHO-K1 cells with Lipofectamine-DNA $\pm$ transMAG-PEI (Example 25)
The experiment was carried out in analogous manner to Example 24 (Figure 23) with Lipofectamine transfection reagent instead of GenePorter. The general trends are similar to this latter experiment. Again, toxicity was pronounced at high transMAG:DNA ratios at high DNA doses (not shown). Maximum enhancements of transfection by the magnetic field are observed at transMAG.DNA ratios of 1 to 4.

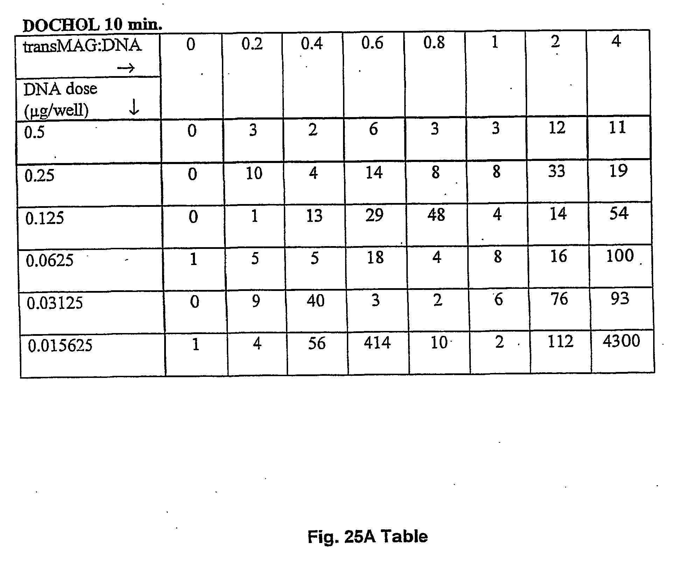

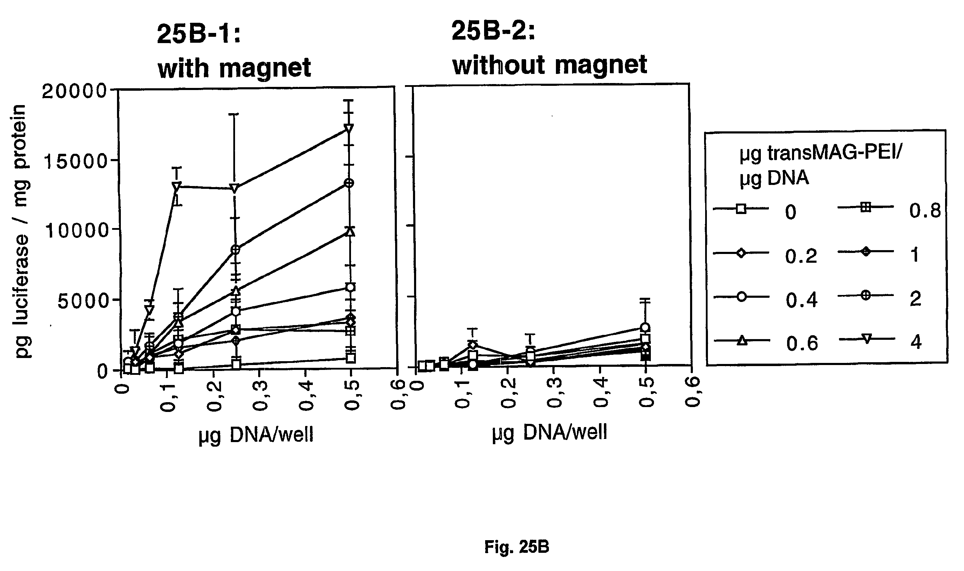

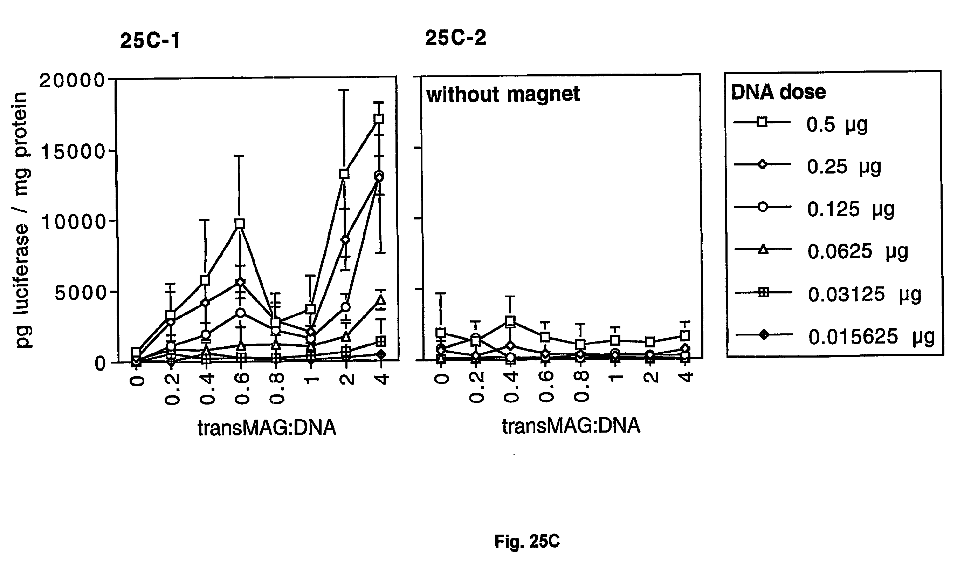

**Figure 25**   Transfection of CHO-K1 cells with DOCHOL-DNA $\pm$ transMAG-PEI (Example 26)
Transfections were carried out in analogy to Examples 24 and 25 (Figures 23 and 24) with DOTAP-Cholesterol liposomes as transfection reagent, however at lower transMAG:DNA ratios and over a broader DNA dose range.

**A.** Table showing the -fold enhancements of transfection by application of the magnetic field. The data shows that enhancements are strong after 10 min of incubation. Also, enhancements are dependent on vector formulation. At higher DNA doses, the vector formulations were toxic to the cells (not shown), although highest reporter gene expressions were found under these conditions. Enhancements are particularly pronounced at lower DNA doses, again demonstrating the strength of the method in rapid transfection at low DNA dose.
**B.** Dose-response profiles in terms of DNA dose. The data points represent the averages of quadruples $\pm$ standard deviations. The strong dose-response relationship at 10 min only in the presence of the magnetic field but not in its absence demonstrates the drastic influence of the magnetic field on transfection kinetics. The composition with the highest ratio of transMAG-DNA (4) turned out to be most favourable and gives rise to high expression levels already at low DNA dosage.
**C.** Dose-response profiles in terms of transMAG dose. The graph reveals that transfection efficiency runs through a minium at around 0.8 $\mu$g transMAG per $\mu$g DNA flanked by a maximum at 0.6 $\mu$g and a potential maximum at 4 $\mu$g in the presence of the magnetic field, identifying suitable formulations for rapid and efficient gene delivery.

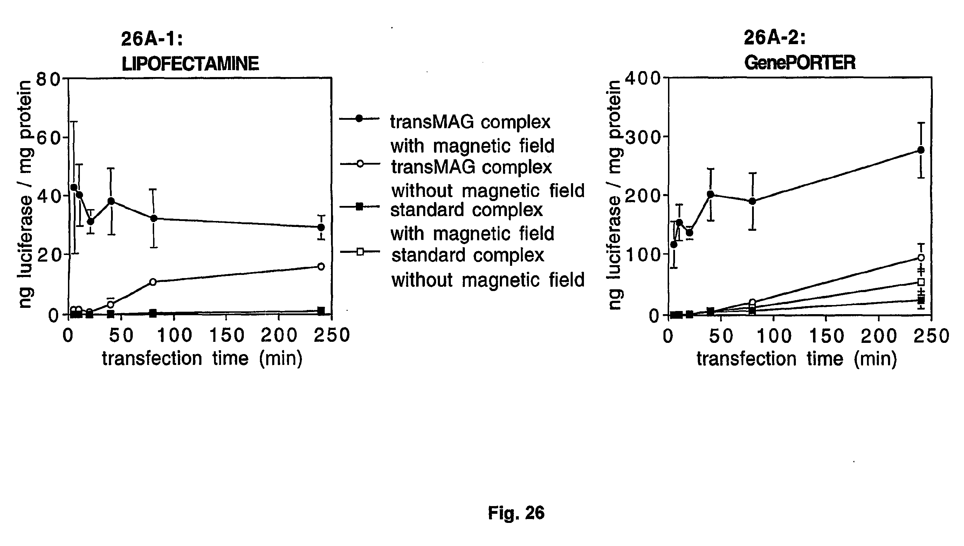

**Figure 26**   Kinetics of magnetofection with cationic lipids in NIH3T3 cells (Example 27)
As an extension to Examples 24 and 25, the transfection kinetics of one particular vector formulation each with GenePorter and Lipofectamine was examined (DNA dose: 0.1 $\mu$g/well and transMAG-PEI:DNA = 2: 1 w/w). Vector formulations were removed from the cells and cells were washed after 5, 10, 20, 40, and 240 minutes of incubation. Under the conditions tested, maximum expression was found already after 5 min with Lipofectamine, while transfection efficiency increased over the time but with a moderate slope with GenePorter (40 % of the final reporter gene expression level was already achieved after 5 min). At any time point, GenePorter formulations were more efficient than Lipofectamine formulations.

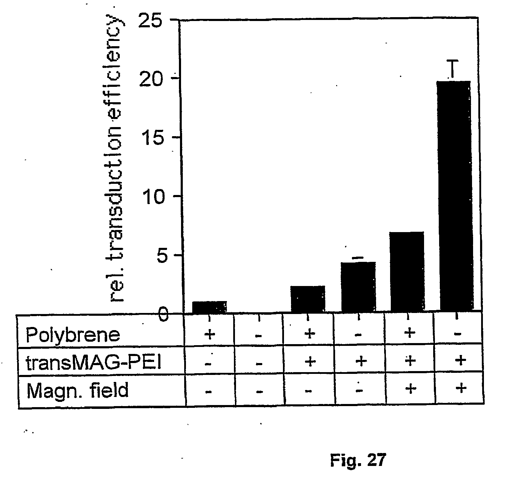

**Figure 27**   Retroviral magnetofection (Example 28)
NIH 3T3 cells were incubated for 3 hrs with 1 ml aliquots of 24 hr supernatants from low titer MuLV producing ecotropic packaging cells. These supernatants were applied untreated or treated with transMAG-PEI (3

μg/ml for 20 min) and/or polybrene (8 μg/ml immediately prior infection). Magnets were applied to specified groups for 1 h. After 48 h, the cells were stained with X-Gal, and blue nuclei were counted. Results are expressed as transduction efficiency relative to the efficiency of a standard transduction (virus + polybrene). In comparison to the standard transduction in the presence of polybrene, the association with transMAG$^{PEI}$ alone enhanced vector efficacy two-fold. Additional application of a magnetic field resulted in a 7-fold increase in transduced cells. If polybrene was omitted from the transducing preparation, virtually no transduction was observed with virus alone. In contrast, omission of polybrene improved superparamagnetic particle guided transduction in the absence of a magnetic field 4-fold over the standard transduction (virus + polybrene) and culminated in a 20-fold enhancement in the presence of a magnetic field. These results demonstrate that magnetofection is applicable to retroviral gene delivery, suggesting that cationic nanoparticles are superior mediators of retroviral transduction compared to polybrene.

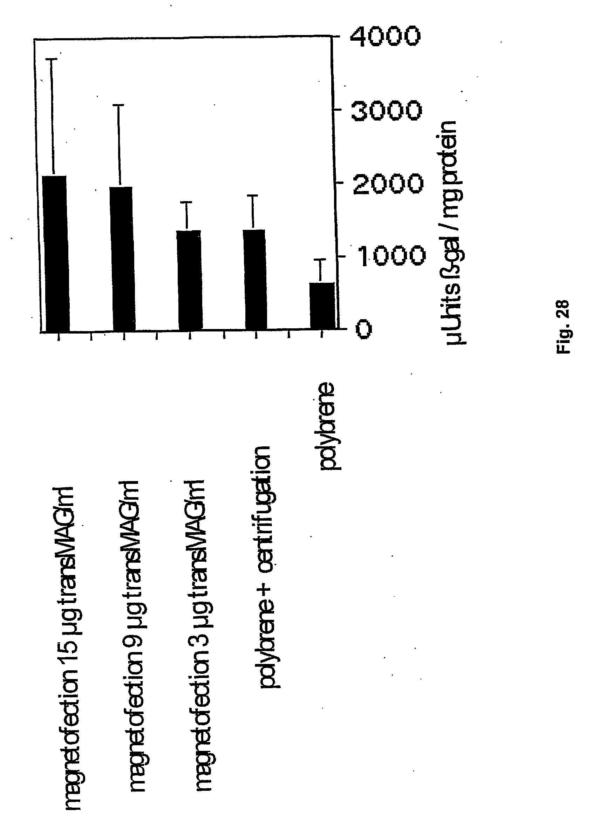

**Figure 28**    Retroviral magnetofection - comparison to vector concentration at the target cell surface by centrifugal force (Example 29) Cells were incubated with 24 hr supernatants of a low MuLV titer-generating producer cell line. The supernatants were either mixed with 3, 9 and 15 μg of transMAG-PEI per ml of supernatant or with 8 μg/ml polybrene. NIH3T3 cells in 96-well plates were incubated for 1 hr with transMAG-containing preparations while positioned on the magnetic plate in 96-well format. Two plates were incubated for 48 hrs with polybrene-mixed supernatants, where one plate was centrifuged for 90 min at 1330 x g. ß-galactosidase expression was quantified after 48 hrs using the CPRG assay (Plank et al. 1999). The data confirms that retroviral magnetofection is superior to the standard polybrene-mediated transduction. Standard transduction assisted by centrifugation improves transduction efficiency by about 2-fold. However, highest transduction levels are achieved by magnetofection, dependent on the transMAG to virus ratio.

**The Examples illustrate the invention:**

**EXPERIMENTAL SETUP**

**1. Magnetic particles**

Magnetic nanoparticles with an average size of 200 nm or 100 nm (by dynamic light scattering) were purchased from Chemicell, Berlin, Germany. According to a previous nomenclature, these particles have been referred to as fluidMAG particles with an extension to their name such as -PEI, referring to the surface coating of the iron oxide core. In the following, these particles are also referred to as fIMAG-*. According to the current nomenclature of Chemicell, Berlin, these particles are referred to as transMAG * particles, with otherwise identical extensions as in the fluidMAG nomenclature. Hence, the only difference between fluidMAG and transMAG is the nomenclature. The transMAG nomenclature is used below starting with Example 11 and the following Examples. Except otherwise stated, all particles are based on magnetite ($Fe_3O_4$).

A. fluidMAG-PEI, HCl; (flMAG-PEI; transMAG-PEI) coated with a monolayer of polyethylenimine (Mw 800 kDa; Fluka)

B. fluidMAG-Polyaspartic acid; (flMAG-pASP; transMAG-pASP) coated with polyaspartic acid, sodium salt, Mw 3000 kDa

C. fluidMAG-Phosphate; (flMAG-PO4) coated with starch-phosphate, Mw 20 kDa

D. fluidMAG-Polyacrylic acid; (flMAG-pACRYL) coated with polyacrylic acid, sodium salt, Mw 20 kDa

E. fluidMAG-Polyacrylic acid-co-maleic acid; (flMAG-pACRYL-MAL) coated Polyacrylic acid-co-maleic acid, sodium salt, Mw 50 kDa

F. fluidMAG-Arabinic acid; (flMAG-ARA) coated with arabinic acid, sodium salt, Mw 250 kDa

G. transMAG-16/1; coated with a multilayer of PEI 800 kD (Fluka)

H. transMAG-18/1 and -19/1; coated with a multilayer of PEI 2000 kD (Aldrich). The difference between 18/1 and 19/1 is the coating procedure

I. transMAG-37 is analogous to transMAG-18/1 but has been autoclaved

J. transMAG-38; same as -37 but magnetite core oxidized to $\gamma$-$Fe_2O_3$

K. transMAG-20/21/23/24/25; multilayer coating with PEI 800 kD (Fluka) using differing coating procedures

L. transMAG-PEI-ethoxylated; monolayer coating with PEI 50 kD (Aldrich) which has been ethoxylated (80 %)

M. transMAG-PEI-epichlorhydrin; monolyer coating with PEI 20 kD (Aldrich) modified with epichlorhydrin

N. transMAG-PEI-lowMW, monolayer coating with PEI, MW 1700 (Aldrich)

O. transMAG-PEI-SDS; monolayer coating with PEI 800 kD (Aldrich) modified by a covalent coupling of sodium dodecyl sulfate (SDS) by carbodiimide activation (N-Ethyl-N'-(dimethylaminopropyl)-carbodiimide)

P. transMAG-STARCH-PEI, multilayer coating with dextrin, MW 60 kD (Fluka) followed by covalent coupling of PEI via amino groups to the periodate-oxidized starch layer.

Q. transMAG-DEAE; monolayer coating with dextrin, introduction of end-standing DEAE groups with 2-diethylamino-ethyl chloride-hydrochloride

R. transMAG-DAEA; coated with a polymer prepared from dimethylamine, epichlorhydrine and ethylene diamine.

S. transMAG-C1/1; PEI-coated particles of approx. 100 nm size (by dynamic light scattering).

### 2. Polyethylene imine (PEI)

PEI (25 kD) as supplied by the manufacturer (Sigma-Aldrich, Deisenhofen, Germany) was dissolved at 10 mg/ml in water and the pH was adjusted to 7.4 by the addition of hydrochloric acid. The material was dialyzed against water followed by sterile filtration (0.20 $\mu$m CA membrane; Peske, Aindling-Pichl, Germany). The concentration of PEI relative to the original solution was determined using ninhydrin assay.

### 3. Biotinylation of PEI (PEI[biotin])

An aliquot of PEI solution (17.2 mg) was lyophilized and redissolved in 0.5 ml 20 mM HEPES pH 7.4. Two equivalents of NHS-LC-Biotin (Pierce, Rockford, IL, USA, #21226T; 68.8 $\mu$l of a 20 mM solution in DMSO) were added. After reaction at room temperature for 3 hrs, the material was purified by gel filtration (Sephadex G-25 filled in a HR 10/10 column, Pharmacia. Flow rate 1 ml/min with water as eluent). The PEI concentration of the product fraction was 4.39 mg/ml according to a ninhydrin assay.

### 4. Biotinylation of flMAG-PEI (flMAG-PEI[biotin])

8.38 $\mu$l of an 8 mM stock solution of NHS-LC-Biotin (Pierce) were added to a dispersion of 2.5 mg flMAG-PEI in 125 $\mu$l water, followed by addition of 250 $\mu$l 20 mM HEPES pH 7.4. After overnight reaction, excess reagent was removed by exhaustive washing with water, where the product flMAG-PEI[biotin] was recovered by magnetic sedimentation and supernatants were discarded.

### 5. Coupling of streptavidin to flMAG-PEI (flMAG-PEI[Stav])

Streptavidin-SPDP: Five mg streptavidin (Molecular Probes, S-888) were dissolved in 500 $\mu$l HBS (20 mM HEPES/150 mM sodium chloride pH 7.4) and purified by gel filtration (Sephadex G-25; PD-10 columns, Pharmacia, Sweden) using the same buffer. The pooled product fractions were concentrated to 520 $\mu$l containing 3.4 mg (56 nmol) streptavidin using a speed-vac. To this solution, a 3.5-fold excess of succinimidyl-pyridyl-dithiopropionate (SPDP; 32 mM in abs. ethanol) was added. After reaction at room temperature over night, the material was purified by gel filtration in HBS (Sephadex G-25 filled in a HR 10/10 column; Pharmacia, Sweden flow rate 0.5 ml/min). The concentration of coupled pyridyl-dithiopropionate was 75 $\mu$M, the concentration of streptavidin was 1.6 mg/ml, corresponding to a substitution of ca. 2.8 PDP per streptavidin molecule.

flMAG-PEI-SH: Thiolation of flMAG-PEI was carried out by adding 4 $\mu$l SPDP (10 mM in ethanol) to 5 mg flMAG-PEI in 250 $\mu$l water, followed by addition of 246 $\mu$l 20 mM HEPES pH 7.4. The reaction was carried out in a microcentrifuge tube which was shaken over night at full speed at 37°C in an Eppendorf shaker (Thermomixer 5436). Subsequently, the material was washed exhaustively with 0.1 % TFA. After reduction by addition of ß-mercaptoethanol, the total amount of coupled pyridyl-dithiopropionate was determined to be ca. 13 nmol. The material was again washed exhaustively with 0.1 % TFA.

A 3-fold excess of streptavidin-SPDP (thiopyridyl groups over thiol groups) was added. After reaction over night, one third of the available thiopyridyl groups had reacted, indicating a quantitative reaction. The product was washed exhaustively with water.

### 6. DOTAP-Cholesterol liposomes

DOTAP (1,2-dioleoyl-3-trimethylammoniumpropane) was purchased from Avanti Polar Lipids (Alabaster, AL, USA). DOTAP/Cholesterol (1:1 mol/mol) liposomes were prepared essentially as described (Meyer et al. 1995). Briefly, 5 ml of a 5 mM solution of DOTAP and cholesterol (1:1 mol/mol) was evaporated to dryness in a silanized scew cap glass tube using a rotary evaporator. The tubes were further held under high vacuum over night. The dried lipid film was rehydrated with 5 ml 5 % glucose in water by vortexing for 30 seconds. A stable liposome emulsion was obtained by sonication for 30 min using an ice-cooled ultrasonic water bath (Sonorex RK510H, Bandelin, Berlin, Germany).

DNA complexes with DOTAP-Cholesterol liposomes are also referred to as DOCHOL-DNA complexes in the following.

**7. DOTAP-Cholesterol liposomes containing flMAG-PO$_4$ (flMAG-DOCHOL)**

The liposome preparation was carried out as described above, except that the rehydration solution was 5 ml 5 % glucose in water containing 825 μg flMAG-PO$_4$. The sonication time was increased to 60 min.

**8. NIH3T3 cells, Hep G2 cells, CHO-K1 cells**

NIH-3T3 mouse fibroblasts (DSMZ #ACC 59) were grown at 37°C in an athmosphere of 5 % CO$_2$ in DMEM supplemented with 10% fetal calf serum, 100 units/ml penicillin, 100 μg/ml streptomycin and 2 mM glutamine (in the following also referred to as "complete DMEM"). Hep G2 (human hepatoblastoma; ATCC #HB-8065) and CHO-K1 cells were grown under the same conditions.

**9. Magnet types**

Magnet type A: Sintered Neodym-Iron-Boron permanent magnet 30 x 10 x 6 mm (NeoDelta Magnet; IBS Magnet, Berlin, Germany. Ordering number NE3010). (B x H)$_{max}$ = 220 - 245 kJ/m$^3$. Br = 1080 - 1150 mT. $_BH_C$ = 795 - 860 kA/m. $_JH_C$ = >1300 kA/m.

Magnet type B: Same material as type A. Cylindrical; d = 6 mm, h = 5 mm (Ordering number NE65). Holes of 6 mm diameter were drilled in 96-well format in an acrylic glass plate with 5 mm thickness. The glass plate was attached to a galvanized steel plate of 1 mm thickness, with otherwise the same dimensions as the acrylic glass plate. The magnet cylinders were inserted into the holes of the acrylic glass plate with strictly alternating polarization. In the following, this format of magnetic plate is also referred to as the "magnetic plate in 96-well format".

Magnet type C: MPC®-96 (Dynal, Hamburg, Germany). (B x H)$_{max}$ = 220 - 210 kJ/m$^3$. Br = 1100 mT. $_BH_C$ = 765 kA/m. $_JH_C$ = >900 kA/m.

Magnet type D: Constructed similarly to magnet type B with cylindrical NeoDelta magnets (d = 15 mm, h = 5 mm; IBS Magnet, Berlin, Germany). Holes in 24-well plate format were drilled in an acrylic glass plate, otherwise the magnetic plate was manufactured in an identical manner as the 96-well format plate. In the following, this format of magnetic plate is also referred to as the "magnetic plate in 24-well format".

**10. Plasmid DNA**

The plasmid DNA p55pCMV-IVS-luc+ used, coding for the firefly luciferase as a reporter gene, was kindly provided by Andrew Baker, Bayer Corp., USA. In the following, the plasmid is also referred to as pCMV-Luc. The plasmid pCMV-ß-gal was kindly provided by Walter Schmidt, Intercell, Vienna, Austria. The plasmids were purified by cesium chloride gradient.

**11. Chemically inactivated adenovirus, Adenovirus-Enhanced-Transfection (AVET) system**

The individual components of the AVET were kindly provided by Ernst Wagner (Boehringer Ingelheim Austria, Vienna, Austria). In this system, a chemically inactivated, biotinylated, E1A-deleted serotype 5 human adenovirus is used that has been treated with psoralen. The AVET is described in detail in US 5,981,273. The adenovirus, stock solution contained 2.87 x 10$^{12}$ viral particles per ml.

**12. Luciferase assay**

Twenty-four hours after transfection cells were washed once with PBS and then incubated with 100 μl of lysis buffer (0.1% Triton X-100 in 250 mM Tris pH 7.8). Ten to fifty microliters each of the cell lysates were transferred to black 96 well plates, mixed with 100 μl of luciferin buffer (60 mM dithiothreitol, 10 mM magnesium sulfate, 1 mM ATP, 30 μM D (-)-luciferin, in 25 mM glycyl-glycine pH 7.8) and assayed for bioluminescence using a TopCount instrument (Canberra Packard). The protein content of the cell lysates was determined using the Bio-Rad protein assay adapted for use in a 96-well plate format. Specific luciferase activity in nanograms luciferase per mg of protein were calculated from a calibration curve which was obtained from the luminescence of a serial dilution of luciferase (Boehringer Mannheim).

**13. HBS buffer**

Unless otherwise stated, HBS buffer means 20 mM HEPES pH 7.4/150 mM sodium chloride.

### 14. HBSa buffer

Has been used in experiments with recombinant adenoviruses. 8 μ/l sodium chloride, 0.37 g/l potassium chloride, 0.27 g/l di-sodium hydrogen phosphate dihydrate, 1 g/l dextrose.

### 15. GenePorter transfection reagent

The reagent was purchased from Gene Therapy Systems, Inc. (San Diego, CA, USA) and rehydrated according to the instructions of the manufacturer.

### 16. Lipofectamine transfection reagent

The reagent was purchased from Life Technologies (Karlsruhe, Germany).

### 17. Superfect transfection reagent

The reagent was purchased from Qiagen (Hilden, Germany).

### 18.Salt-induced aggregation

This term is used in the following to describe a method of vector assembly which exploits colloid aggregation/ flocculation upon increasing ionic strength. This phenomenon is particlularly pronounced with particles formed from polyelectrolytes and has been known for a long time (Hiemenz 1986). In vector assembly, it is sufficient to mix the vector components in salt-containing solution or to mix them in water, followed by mixing with a salt-containing solution.

### Reference Example 1: DNA binding isotherm of flMAG-PEI

<u>Nick labeling of plasmid DNA</u>: One μg of plasmid DNA per reaction was labeled with $^{32}P$ using the Nick Translation Kit from Amersham-Pharmacia with the protocol of the supplier modified such that the incubation time was 15 min at 15°C instead of 2 hrs. $\alpha$-$^{32}P$-dATP (Hartmann Analytic, Braunschweig, Germany) with a specific activity of 3,000 Ci/mmol was used for the labeling reaction. The labeled plasmid was purified using MicroSpin™ columns (Pharmacia, Freiburg, Germany) and the Promega Wizard™ PCR Preps DNA Purification System (Promega, Mannheim, Germany) for removal of unincorporated nucleotides and enzymes from the reaction mixture. The final volume of the product was 100 μl with an activity of 234,248 CPM/μl, determined as described below. The product DNA had the same size as the starting plasmid as confirmed by gel electrophoresis.

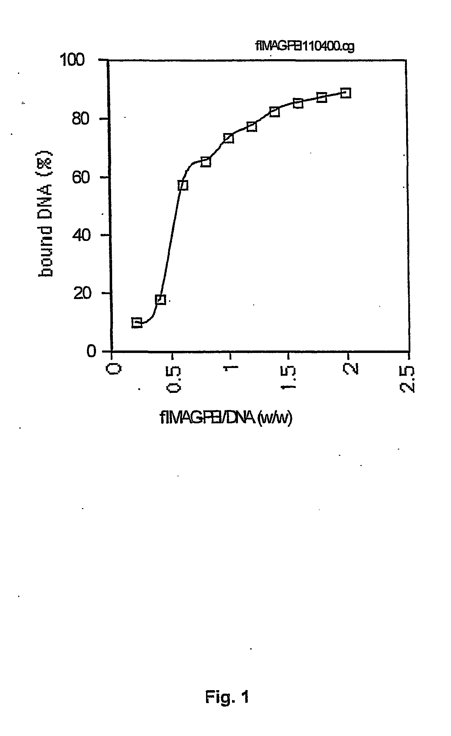

To 0, 1, 2, 3, 4, 5, 6, 7, 8, 9 and 10 micrograms of polyethylene imine-coated magnetic particles (flMAG-PEI), supplied from Chemicell, Berlin, Germany, suspended in 40 μl of water in Eppendorf tubes, 40 μl each of a plasmid DNA stock solution (125 μg/ml in water) containing 200,000 CPM each of labeled plasmid (< 0.1 μg) were added and mixed by pipetting. After 15 min of incubation, the samples were centrifuged at 20,000 x g for 20 minutes. Forty μl each of the supernatants were transferred to a white 96-well plate (Costar) and mixed with 160 μl Microscint 20 cocktail (Canberra Packard). Radioactivity in the wells was determined using a TopCount instrument (Canberra Packard; Count delay 10 min, count time was 3 x 5 min). The results are shown in Figure 1.

### Example 2: Lipo-magnetofection of NIH3T3 cells

Cells were seeded in 96-well plates at a density of 30.000 cells per well 7 hours prior transfection. Immediately preceding transfection, the medium was exchanged with 150 μl of fresh medium. DNA complexes were added in a volume of 50 μl per well.

<u>Source solutions for preparing DNA complexes:</u>

<u>DOTAP-Chol stock solution:</u>

90.91 μl liposome stock (see above) per ml HBS

<u>flMAG-DOCHOL liposome stock solution:</u>

90.91 μl stock (see above) per ml HBS

DNA stock solution:

30 μg plasmid p55pCMV-IVS-luc+ per ml HBS

DOTAP-Chol-DNA stock solution:

Equal volumes of DNA stock were added to equal volumes of DOTAP-Chol stock and mixed by pipetting

flMAG-PO$_4$ dispersion: 30 μg per ml HBS
flMAG-PEI dispersion: 30 μg per ml HBS

Mixing orders for the formulations tested:

1. 560 μl DOTAP-Chol-DNA stock were added to 280 μl flMAG-PO$_4$ dispersion
2. 280 μl DNA stock were added 280 μl flMAG-PO$_4$ dispersion. The resulting mix was added to 280 μl DOTAP-Chol stock.
3. Analogous to 1. with flMAG-PEI.
4. Analogous to 2. with flMAG-PEI.
5. 160 μl of DNA stock were added to 160 μl of DOTAP-Chol-flMAG-PO$_4$ liposome stock.
6. 160 μl of DNA stock were added to 320 μl of DOTAP-Chol-flMAG-PO$_4$ liposome stock.

Dilution series

A dilution series was carried out for formulations 1 - 4. Aliquots of 360 μl each of the DNA complexes were added to row A of a 96-well plate. Rows B - D contained 180 μl HBS each. Aliquots of 180 μl were transferred from row A to row B with a multichannel pipettor, mixed by pipetting, 180 μl were transferred from row B to row C and so on.

Transfection

Fifty μl each of the resulting dilutions were added to the cells in triplicates (in quadruples for formulations 5 and 6) in two different 96-well plates. After DNA complex addition, one plate was set upon magnet type B. After 10 min of incubation, the transfection media were removed, the cells were washed with 150 μl of fresh medium and incubated over night in fresh medium. The results are shown in Figure 2.

**Reference Example 3: Magnetofection of NIH3T3 cells with flMAG-PEI and naked DNA**

Cells were seeded in 96-well plates at a density of 30,000 cells per well 2 days prior transfection. The cells were confluent at the time of transfection. Immediately preceding transfection, the medium was exchanged with 150 μl of fresh medium. DNA complexes were added in a volume of 50 μl per well.

Source solution for preparing DNA complexes:

DNA stock: 25 μg of p55pCMV-IVS-luc+ in 562.5 μl water

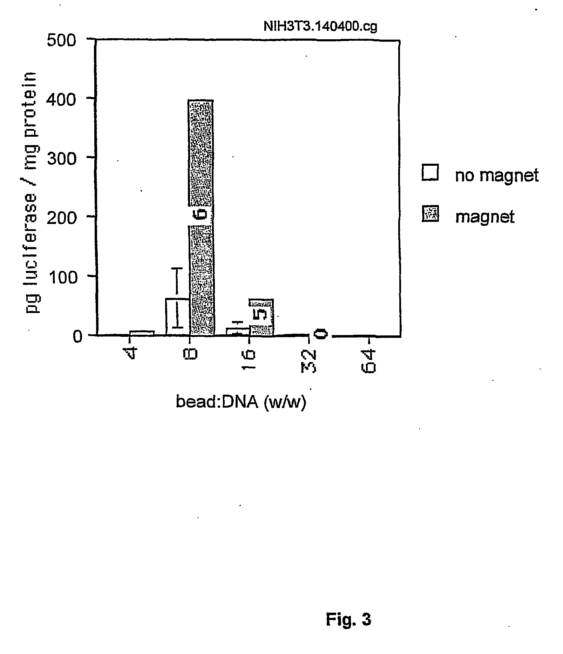

DNA stock (112.5 μl each) was added to 112.5 μl each of a flMAG-PEI dilution series in water containing 20, 40, 60 or 80 μg flMAG-PEI. After 15 min of incubation, 60 μl each 50 % glucose in water were added. Fifty μl each (0.5 μg DNA each) of the resulting suspension was added to the cells. Row H of the plate was positioned upon magnets of type A. After 1.5 hrs of incubation, the medium was exchanged. The luciferase assay was carried out 24 hrs after transfection, the results are shown in Figure 3.

**Example 4: Magneto-PEI-polyfection of NIH3T3 cells in the presence of protective copolymer (PROCOP) P6YE5C (Finsinger et al. 2000)**

Cells were seeded in 96-well plates at a density of 30.000 cells per well 7 hours prior transfection. Immediately preceding transfection, the medium was exchanged with 150 $\mu$l of fresh medium. DNA complexes were added in a volume of 50 $\mu$l per well.

Source solutions for preparing DNA complexes:

PEI stock solution:

46.3 $\mu$g per ml water or HBS, respectively
Variant a): 1 mg/ml in water

PEI<sup>biotin</sup> stock solution:

46.3 $\mu$g per ml water or HBS, respectively
Variant a): 4.39 mg/ml in water

DNA stock solution:

44.4 $\mu$g plasmid p55pCMV-IVS-luc+ per ml water or HBS, respectively

PEI-DNA (PEI<sup>biotin</sup>-DNA) stock solution:

Equal volumes of DNA stock were added to equal volumes of PEI-DNA
(PEI<sup>biotin</sup>-DNA, resp.) stock and mixed by pipetting

P6YE5C stock solution:

269 nmol negative charge per ml water or HBS, respectively

flMAG-PEI dispersion: 44.4 $\mu$g per ml water or HBS, respectively
Variant a): 1 mg/ml in water
flMAG-PEI<sup>stav</sup> dispersion: 44.4 $\mu$g per ml water or HBS, respectively
Variant a): 0.9 mg/ml in water

Mixing orders for the formulations tested:

1. 216 $\mu$l PEI-DNA stock (water) were added to 108 $\mu$l flMAG-PEI stock and mixed by pipetting. After 15 min, the DNA complex was added to 108 $\mu$l P6YE5C stock and mixed by pipetting. Finally, 48 $\mu$l 50 % glucose in water were added.
2. 216 $\mu$l PEI-DNA stock (water) were added to 108 $\mu$l flMAG-PEI<sup>Stav</sup> stock and mixed by pipetting. Rest as in 1.
3. 216 $\mu$l PEI<sup>biotin</sup>-DNA stock (water) were added to 108 $\mu$l flMAG-PEI stock and mixed by pipetting. Rest as in 1.
4. 216 $\mu$l PEI<sup>biotin</sup>-DNA stock (water) were added to 108 $\mu$l flMAG-PEI<sup>Stav</sup> stock and mixed by pipetting. Rest as in 1.
5. Pre-mixing of 5 $\mu$l PEI and 4.8 $\mu$l flMAG-PEI stocks variants a), filled up with 206.2 $\mu$l water, addition of 108 $\mu$l DNA stock. After 15 min, the resulting DNA complex was added to 108 $\mu$l P6YE5C stock and mixed by pipetting. Finally, 48 $\mu$l 50 % glucose in water were added.
6. Pre-mixing of 5 $\mu$l PEI and 5.33 $\mu$l flMAG-PEI<sup>Stav</sup> stocks variants a), filled up with 205.7 $\mu$l water, addition of 108 $\mu$l DNA stock. Rest as in 5.
7. Pre-mixing of 1.14 $\mu$l PEI<sup>biotin</sup> and 4.8 $\mu$l flMAG-PEI stocks variants a), filled up with 210 $\mu$l water, addition of 108 $\mu$l DNA stock. Rest as in 5.
8. Pre-mixing of 1.14 $\mu$l PEI<sup>biotin</sup> and 5.33 $\mu$l flMAG-PEI<sup>Stav</sup> stocks variants a), filled up with 209.5 $\mu$l water, addition of 108 $\mu$l DNA stock. Rest as in 5.
9. Analogous to 1, but carried out in HBS. In the final step, 48 $\mu$l HBS were added instead of glucose solution.
10. Analogous to 2, but carried out in HBS. In the final step, 48 $\mu$l HBS were added instead of glucose solution.
11. Analogous to 3, but carried out in HBS. In the final step, 48 $\mu$l HBS were added instead of glucose solution.
12. Analogous to 4, but carried out in HBS. In the final step, 48 $\mu$l HBS were added instead of glucose solution.

Transfection

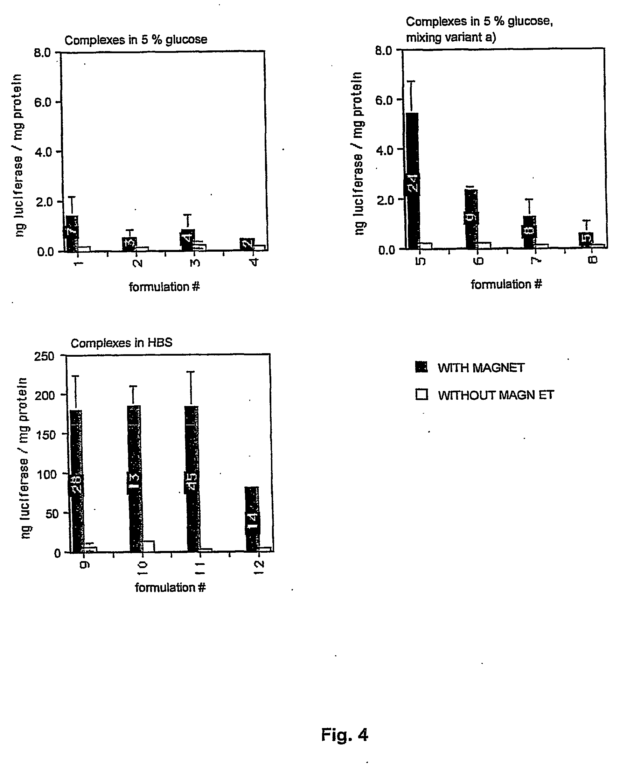

Fifty $\mu$l each (corresponding to 0.5 $\mu$g DNA) of the resulting dilutions were added to the cells in quadruples in two different 96-well plates. After DNA complex addition, one plate was set upon magnet type B. After 10 min of incubation, the transfection media were removed, the cells were washed with 150 $\mu$l of fresh medium and incubated over night in fresh medium; The results are shown in Figure 4.

**Example 5: Adenovirus-enhanced magnetofection (AVEM)-Gene transfer with**

**flMAG-PEI:PEI.biotin-DNA:Advbiotin, and flMAG-PEIStav:PEIbiotin-DNA:Advbiotin to**

**NIH3T3 cells; Reporter gene: ß-galactosidase**

Cells were seeded in a six-well plate at a density of 350.000 cells per well the day prior transfection. The medium was exchanged with 1.5 ml of fresh medium per well immediately before transfection. DNA complexes were added in total volumes of 500 $\mu$l.

DNA complexes:

Thirty $\mu$g DNA (pCMV-ßgal) in 625 $\mu$l HBS (20 mM HEPES pH 7.4/150 mM sodium chloride) were added to 31.25 $\mu$g PEIbiotin in the same volume of the same buffer. Fifteen microliters of psoralen-inactivated, biotinylated adenovirus (Adv biotin) were diluted to 625 $\mu$l with HBS and added to the preformed DNA complexes. Half of the resulting material was added to 18 $\mu$g flMAG-PEI in 313 $\mu$l HBS, the other half was added to 18 $\mu$g flMAG-PEIStav in 313 $\mu$l HBS. Aliquots of 500 $\mu$l per well of these final DNA complex preparations were added to the cells corresponding to a DNA dose of 6 $\mu$g per well (wells 2 and 3 with flMAG-PEI; wells 4 and 5 with flMAG-PEIStav). Magnets type A were attached under wells 3 and 4. After 20 min incubation, the cells were washed once with fresh medium and then incubated for 24 hrs. The magnets were removed 30 min after addition of the gene vectors. After 24 hrs, the cells were washed with PBS and incubated with X-gal substrate solution (5 mM $K_4Fe(CN)_6$, 5 mM $K_3Fe(CN)_6$, 2 mM magnesium chloride, 1 mg/ml X-gal in PBS). After 45 min of staining, the plate was scanned for documentation (Figure 5).

**Example 6: Gene transfer with flMAG-PEI:PEIbiotin-DNA:Advbiotin, and flMAG-PEIStav:PEIbiotin-DNA:Advbiotin to NIH3T3 cells. Reporter gene: luciferase**

Cells:

NIH3T3 cells (28.000 per well) were seeded in 96-well plates the day prior transfection. Immediately before adding the transfection solutions (50 $\mu$l each), the medium was exchanged with 150 $\mu$l fresh medium.

PEIbiotin-DNA stock: 25 pg of p55pCMV-IVS-luc+ DNA in 625 $\mu$l HBS was added to 26.06 $\mu$g PEI in 625 $\mu$l HBS and mixed by pipetting.
flMAG-PEI stock A: 40 $\mu$g/ml in HBS.
flMAG-PEI stock B: 80 $\mu$g/ml in HBS.
flMAG-PEIStav stock A: 40 $\mu$g/ml in HBS.
flMAG-PEIStav stock B: 80 $\mu$g/ml in HBS.
Chemically inactivated Adenovirus: 20 $\mu$l per ml in HBS.

Aliquots of 240 $\mu$l each of PEIbiotin-DNA stock were mixed with 120 $\mu$l each of either flMAG-PEI stock A or B, or flMAG-PEIStav stock A or B, respectively, incubated for 15 min followed by addition of 120 $\mu$l of virus stock.

AVET: Virus stock (2.4 $\mu$l in 120 $\mu$l HBS) was added to Stav-pL stock (240 ng in 120 $\mu$l HBS), incubated for 15 min, added to DNA stock (4.8 $\mu$g in 120 $\mu$l HBS) followed by addition of poly(lysine) (pL170) (4.8 $\mu$g in 120 $\mu$l HBS).

After a final incubation of the thus assembled complexes for 15 min, aliquots of 50 each were added to the cells in quadruples. The plates were incubated at 37°C for 5 min, one plate placed upon a 96-well magnetic plate (magnet type C), the other plate without magnet. Cells were washed once with 150 $\mu$l of fresh medium and then incubated over night in 150 $\mu$l medium. The luciferase assay was carried out 24 hrs after transfection. The results are shown in Figure 6.

**Example 7: Additional incorporation of an effector component: incorporation of the membrane-destabilizing peptide INF7 in polylysine (pL) and PEI polyplexes together with flMAG-PEI**

Cells:

NIH3T3 cells (28.000 per well) were seeded in 96-well plates the day prior transfection. Immediately before adding the transfection solutions (50 µl each), the medium was exchanged with 150 µl fresh medium.

pL-DNA stock:

28 µg p55pCMV-IVS-luc+ in 700 µl HBS added to 141.9 µg pL170 in 700 µl HBS and mixed by pipetting (resulting in N/P = 8).

PEI-DNA stock:

16.8 µg DNA in 420 µl HBS added to 17.5 µg PEI in 420 µl HBS (resulting in N/P = 8).

flMAG-PEI stock:

33.6 µg flMAG-PEI in 840 µl HBS.

INF7 stock:

24.2 µM INF7 peptide having the amino acid sequence GLFEAIEGFIENGWEGMIDGWYGC (SEQ ID NO: 1) in HBS (corresponding to 121.1 µM neg charge).

Mixing orders for the formulations tested:

> 1. PEI/DNA/flMAG-PEI: 240 µl PEI-DNA stock were added to 120 µl flMAG-PEI stock, followed by addition of 120 µl HBS after 15 min.
> 2. PEI/DNA/INF7/flMAG-PEI: 240 µl PEI-DNA stock were added to 120 µl INF7 stock, incubated for 10 min, and added to 120 µl flMAG-PEI stock.
> 3. PEI/DNA/flMAG-PEI/INF7: 240 µl PEI-DNA stock were added to 120 µl flMAG-PEI stock, incubated for 10 min, and added to 120 µl INF7 stock.
> 4. pL/DNA/INF7: 240 µl pL-DNA stock were added to 120 µl INF7 stock, followed by addition of 120 µl HBS after 15 min.
> 5. pL/DNA/flMAG-PEI: 240 µl pL-DNA stock were added to 120 µl flMAG-PEI stock, followed by addition of 120 µl HBS after 15 min.
> 6. pL/DNA/flMAG-PEI/INF7: 240 µl pL-DNA stock were added to 120 µl flMAG-PEI stock, followed by addition to 120 µl INF7 stock after 15 min.
> 7. pL/DNA/INF7/flMAG-PEI: 240 µl pL-DNA stock were added to 120 µl INF7 stock, followed by addition to 120 µl flMAG-PEI stock after 15 min.

After 15 min of incubation, aliquots of 50 µl each were added to the cells in quadruples. The plates were incubated at 37°C for 10 min, one plate placed upon a 96-well magnetic plate (magnet type C), the other plate without magnet. Cells were washed once with 150 µl of fresh medium and then incubated over night in 150 µl medium. The luciferase assay was carried out 24 hrs after transfection. The results are shown in Figure 7.

**Reference Example 8: Magnetofection of NIH3T3 and HepG2 cells with negatively charged magnetic particles (flMAG-ARA; flMAG-pACRYL; flMAG-pACRYL-MAL; flMAG-pASP)**

Cells:

NIH3T3 cells (28.000 per well) and Hep G2 cells (45.000 per well) were seeded in 96-well plates the day prior transfection. Immediately before adding the transfection solutions (50 µl each), the medium was exchanged with 150 µl fresh medium.

PEI-DNA stock:

240 $\mu$g p55pCMV-IVS-luc+ DNA in 7200 $\mu$l water were added to 250.2 $\mu$g PEI in 7200 $\mu$l water and mixed by vortexing.

Dilution series of flMAGs:

Aliquots of 144 $\mu$l each of FIMAG stocks in water containing 38.4 $\mu$g flMAG each were added to columns 1 and 7 of a U-bottom 96-well plate in quadruples. All other wells contained 72 $\mu$l of water. Aliquots of 72 $\mu$l each were transferred from column 1 to column 2 with a multichannel pipettor, mixed by pipetting, 72 $\mu$l were transferred to column 3 and so on up to column 5. The surplus 72 $\mu$l from column 5 were discarded. The same procedure was carried out from column 7 to column 11. Columns 6 and 12 contained only water.

To the resulting dilutions, 144 $\mu$l each of PEI-DNA stock were added and mixed by pipetting. After 15 min incubation, 24 $\mu$l each of 50 % (w/w) glucose in water were added. Of the resulting samples, 50 $\mu$l each were added to the cells in quadruples. The plates were incubated at 37°C for 10 min, one plate placed upon a 96-well magnetic plate (magnet type B), the other plate without magnet. Cells were washed once with 150 $\mu$l of fresh medium and then incubated over night in 150 $\mu$l medium. The luciferase assay was carried out 24 hrs after transfection. The results are shown in Figure 8.

**Example 9: Transfection by calcium phosphate co-precipitation of flMAG-PEI and DNA in the presence and in the absence of protective copolymer P6YE5C**

Calcium phosphate precipitation of DNA has been used widely for the transfection of cell lines in culture. DNA is diluted in a calcium chloride solution and subsequently added dropwise to an equal volume of phosphate-containing buffer. Massive precipitates are formed, a dispersion of which is added to the cell culture. If the phosphate-containing buffer also contains the protective copolymer P6YE5C, nanoparticles are formed which are relatively stable. In order to show that flMAGs can be incorporated into calcium phosphate coprecipitates or nanoparticles, the following experiment was carried out.

Cells:

NIH3T3 cells (28.000 per well) and Hep G2 cells (45.000 per well) were seeded in 96-well plates the day prior transfection. Immediately before adding the transfection solutions (50 $\mu$l each), the medium was exchanged with 150 $\mu$l fresh medium.

DNA stock solution: 45.6 ng/$\mu$l p55pCMV-IVS-luc+ in water.

One hundred $\mu$l each of DNA stock solution were added to 100 $\mu$l each of water containing 0, 2.28, 4.56, 9.13, 18.25 and 36.5 $\mu$g flMAG-PEI and mixed by pipetting. Aliquots of 28.1 $\mu$l of 2.5 M calcium chloride each were added to these samples, resulting in a DNA concentration of 20 $\mu$g/ml in 308 mM calcium chloride. This procedure was carried out in duplicates. The samples were added dropwise while vortexing to equal volumes (228.1 $\mu$l) 2x HBS (50 mM HEPES pH 7.1, 280 mM sodium chloride, 1.5 mM di-sodium hydrogen phosphate) or 2x HBS containing 3 charge equivalents of protective copolymer P6YE5C. Charge equivalents means the ratio of negative charges from protective copolymer to negative charges of DNA (Finsinger et al. 2000). After 15-30 min incubation, aliquots of 50 $\mu$l each (corresponding to 0.5 $\mu$g DNA) were added to the cells. The plates were incubated at 37°C for 10 min, one plate placed upon a 96-well magnetic plate (magnet type B), the other plate without magnet Cells were washed once with 150 $\mu$l of fresh medium and then incubated over night in 150 $\mu$l medium. The luciferase assay was carried out 24 hrs after transfection. The results are shown in Figure 9.

**Example 10: Magnetofection with a recombinant adenovirus carrying the lacZ gene**

AdLacZ is an E1A-deleted human serotype 5 adenovirus which carries the gene for ß-galactosidase as a reporter gene.

NIH3T3 cells were, plated in 6-well plates at a density 500.000 cells per well the day before transfection. Immediately preceding transfection, fresh medium (1.5 ml) was added to the cells. Transfection (transduction) cocktails were added in a total volume of 500 $\mu$l per well. After incubation for 20 min in transduction medium, the cells were washed with 2 ml of fresh medium and then incubated over night. Twenty-four hours after transduction, X-gal staining was carried out. The cells were washed twice with PBS, fixed with 1 ml of fixative solution (0.2 % glutar aldehyde, 2 % para formaldehyde, 2 mM magnesium chloride in PBS) for 1 h, washed twice with HBS and stained for 1 h with 0.5 ml of staining solution (5 mM $K_4Fe(CN)_6$, 5 mM $K_3Fe(CN)_6$, 2 mM magnesium chloride, 1 mg/ml X-gal in PBS).

Controls: Five hundred $\mu$l HBS were added to the cells in one well. To a second well, 500 $\mu$l HBS buffer containing approx. $7.8 \times 10^9$ viral particles (AdLacZ) were added. Under both wells a magnet of type A was positioned during 20 min of incubation. AVET-type transfection: Virus stock (approx. $1.81 \times 10^{10}$ viral particles, AdLacZ, in 262.5 $\mu$l HBS, corresponding to $2.5 \times 10^8$ pfu) were added to flMAG-PEI stock (14.4 $\mu$g in 300 $\mu$l HBS), mixed by pipetting and incubated for 10 min. To this were added 14.4 $\mu$g plasmid DNA (coding for green fluorescent protein (GFP) under the control of the elongation factor alpha promoter) in 300 $\mu$l HBS, incubated for 10 min followed by addition to 14.4 $\mu$g PEI in 300 $\mu$l HBS. After 10 min of incubation, aliquots of 500 $\mu$l were added to the cells in two wells. A magnet of type A was positioned under one well. After 30 min, the cells were washed with 2 ml of fresh medium and then incubated over night. Twenty-four hours after transduction, X-gal staining was carried out (wells 2 and 2a in Figure 10).

The same procedure was carried out with chemically inactivated, biotinylated adenovirus (wells 3 and 3a in Figure 10).

The co-transfected GFP gene construct was expressed in each of the transfection reactions where it was used (wells 2, 2a, 3 and 3a; data not shown). In wells 2 and 3, GFP expression was restricted to the area, under which the magnet laid, like with β-galactosidase activity as for example in well 2. In wells 2a and 3a, GFP staining could also be observed, however, in contrast to the corresponding wells with magnetic field, in a weaker intensity and spread out over the complete well bottom area. Thus, the positive effect of enhancement and specific localization could also be demonstrated in this case, where a second reporter gene present in a plasmid was transfected together with a virus.

Titration of AdLacZ / flMAG-PEI ratio:

AdLacZ stock:

$1.09 \times 10^{11}$ AdLacZ particles in 1575 $\mu$l

Aliquots of AdLacZ stock (250 $\mu$l each) were added to 300 $\mu$l each of HBS containing 57.6, 28.8, 14.4, 7.2, 3.6, or 1.8 $\mu$g flMAG-PEI each, respectively. After 10 min of incubation, 250 $\mu$l each the resulting samples were added to the cells in duplicates, corresponding to $8.6 \times 10^9$ viral particles per well and 24, 12, 6, 3, 1.5 and 0.75 $\mu$g flMAG-PEI per well. Magnets of type A were positionend under one well of the duplicates each during 20 min of incubation. After that, cells were washed once with fresh medium and then incubated over night. X-gal staining was carried out as described above. The results are shown in Figure 10 (wells 4 to 9a).

**Example 11: DNA binding isotherms of ternary complexes of transMAG-PEI / DNA / PEI and transMAG-PEI / DNA / DOCHOL**

DNA stock solution: 128.4 $\mu$g plasmid DNA plus $1.56 \times 10^7$ CPM $^{32}$P-labeled plasmid DNA in 3120 $\mu$l of water.
PEI stock solution: 65.05 $\mu$g in 1560 $\mu$l of water.
DOCHOL liposome stock suspension: 189.1 $\mu$l 5 mM stock in 1560 $\mu$l water.

In two separate setups, 120 $\mu$l each of DNA stock solution (corresponding to ca. 600.000 CPM) were added to 120 $\mu$l each of suspensions containing the following amounts of transMAG-PEI, resulting in the following weight to weight ratios of transMAG-PEI to DNA. The complexes were prepared in Eppendorf tubes and mixed by pipetting.

| $\mu$g transMA G-PEI | 0 | 0.96 | 1.92 | 2.88 | 3.84 | 4.8 | 9.6 | 19.2 | 28.8 | 38.4 | 48 | 72 |
|---|---|---|---|---|---|---|---|---|---|---|---|---|
| Resulting w/w ratio to DNA | 0 | 0.2 | 0.4 | 0.6 | 0.8 | 1 | 2 | 4 | 6 | 8 | 10 | 15 |

After 15 min of incubation, the mixtures were added to either 120 $\mu$l each of PEI stock solutions or of DOCHOL liposome stock suspensions in Eppendorf tubes and mixed by pipetting. This results in PEI:DNA N/P ratios of 8 or DOTAP:DNA charge ratios of 5. After further 15 min incubation, the complexes were added to 120 $\mu$l each of 600 mM sodium chloride, initializing salt-induced aggregation. After 20 min of incubation, 120 $\mu$l each of the resulting complexes were transferred to the wells of a U-bottom 96-well plate in triplicates. The plate was positioned upon the 96-well format magnetic plate. After 30 min of magnetic sedimentation, 80 $\mu$l supernatants were removed and mixed with 125 $\mu$l each of Microscint 40 (Canberra Packard, Dreieich, Germany) in an opaque 96 well plate. In the same manner, 80 $\mu$l each of unsedimented samples were added to the plate as reference. The samples were counted using a Topcount instrument (Canberra Packard, count delay set to 10 min, count time in triplicates 5 min each).

DNA binding was calculated as

$$\%bound = 100 \times \frac{CPM_{sample}}{CPM_{reference}}$$

**Example 12: Salt-induced aggregation**

Ten $\mu$g of plasmid DNA in 333 $\mu$l water were mixed with 10 $\mu$g transMAG-PEI suspended in 333 $\mu$l of water. After 10 min of incubation, the resulting complex was added to and mixed with 10.42 $\mu$g of PEI dissolved in 333 $\mu$l of water. The size of the resulting particles was determined by dynamic light scattering using a Malvern 3000 HS zetasizer (Malvern, Herrenberg, Germany). The measured particle size averaged over 9 measurements was 217.0 $\pm$ 2.0 and was stable over time. Subsequently, 30.9 $\mu$l of 5 M sodium chloride were added to the cuvette and mixed with the vector suspension. Size measurements were accumulated with an acquisition time set to 90 seconds, giving a recorded result on the instrument on average every 98 seconds. Sixty measurements were carried out over a time span of 1 hr 48 min 22 sec with a delay time after the 30th measurement.

**Example 13: Binding isotherm of adenovirus and transMAG-PEI**

Iodination of adenovirus: Recombinant adenovirus (10 $\mu$l stock, corresponding to 7.2 x $10^{10}$ viral particles or 6.6 x $10^8$ PFU. PFU = plaque forming units) was diluted to 100 $\mu$l with HBSa buffer, mixed with 7.8 MBq $^{125}$I (2 $\mu$l; Amersham-Pharmacia, Freiburg, Germany) and incubated for 10 min at room temperature in a iodogen cap (Pierce). After addition of 200 $\mu$l HBSa buffer, the virus was separated from unbound label by gel filtration using a Pharmacia PD-10 column. The quality of separation as well as four virus-containing fractions were identified by radioactivity monitoring. Fraction 1, containing 2.61 x $10^{10}$ viral particles (determined by UV absorbance) and an activity of 485 kBq per ml was used for binding studies.

Thirtysix microliters of labeled adenovirus were mixed with 18 $\mu$l of transMAG-PEI suspensions in HBS containing the following amounts of transMAG and resulting in the following ratios of viral particles (VP) per pg of transMAG:

| ng transMAG-PEI | 0 | 108 | 216 | 324 | 432 | 540 | 648 | 864 | 1080 | 1296 | 1512 | 1728 |
|---|---|---|---|---|---|---|---|---|---|---|---|---|
| Resulting ratio VP per pg transMAG | | 8700 | 4350 | 2900 | 2175 | 1740 | 1450 | 1088 | 870 | 725 | 621 | 544 |

After 20 min incubation at room temperature, the samples were filled up to 432 $\mu$l with HBS. Aliquots of 120 $\mu$l each were transferred to a U-bottom 96-well plate in triplicates which subsequently was positioned on the 96-well format magnetic plate. After 1 hr magnetic sedimentation, 80 $\mu$l of the supernatants each and of unsedimented samples were transferred to individual scintillation tubes (Polyvials V, Zinsser Analytic GmbH, Frankfurt, Germany) and counted using a gamma counter (Wallac, Turku, Finland). The binding isotherm was calculated as above.

**Example 14: Biodistribution of $^{125}$I-labeled adenovirus in mice**

Eighty microliters of labeled virus (fraction 2, corresponding to 5,23 x $10^9$ VP and 97 kBq) were mixed with 2.4 $\mu$g of transMAG-PEI suspended in 20 $\mu$l of HBSa (corresponding to approx. 73 % virus binding according to the binding isotherm). After 20 min incubation, the mixture was filled up to 800 $\mu$l with HBSa. One hundred microliters each were injected into the animals via the tail vein. The animals were anesthesized with an i.p injection of 100 mg/kg body weight Ketamine / 8 mg/kg Xylazine. One animal received labeled virus alone which was not bound to transMAG-PEI. Six animals received magnetic particle-bound virus. Three animals (2 NMRI mice and one C57BL-6 mouse) had neodymium-iron-boron permanent magnet blocks (2 x 1 x 0.5 cm) attached to their right chests while three (2 NMRI mice and one C57BL-6 mouse) animals were injected without attached magnet. One hour after injection, the animals were opened. As much blood as possible was drawn from the right ventricle. Blood, heart, lung, liver, kidneys, spleen, the intestinal tract (stomach and guts), tail, head, thorax and abdominal tract were added to individual scintillation vials and counted using a gamma counter (Wallac, Turku, Finland).

**Example 15: Magnetofection in ear veins of pigs**

Vector preparation: Per treated animal, 1 mg DNA (pCMV-Luc) in 2.5 ml water was mixed with 1 mg transMAG-PEI in the same volume of water. After 15 min incubation, the mixture was added to 1.042 mg PEI diluted to 2.5 ml with water while vortexing. After further 15 min, the preparation wars mixed with 2.5 ml 600 mM sodium chloride. After 30 min incubation, 5 ml each of the vector preparation were injected via the vena auricularis magna at a rate of approx. 1 ml/min in both ears of anesthetized animals. A Nd-Fe-B permanent magnet block (3 x 1 x 0.5 cm, NeoDelta, IBS Magnet, Berlin Germany) was attached above the right veins proximal to the injection sites. Sections of the veins were isolated after 24 hours and assayed for luciferase expression as described in Example 17. No reporter gene expression (luciferase) was observed in the control blood vessels (left ears) and distal from the magnet positions (right ears), while reproducible, though variable (741,897 $\pm$ 693,298 RLU / g tissue; average $\pm$ standard deviation of 4 animals), luciferase expression was found in all vein samples which were under direct influence of the magnetic field. No luciferase signal (light emission) was found in samples of any other major organ. Premedication of animals: The animals were anesthetized with 2 mg/kg body weight Azaperon (Stresnil®, Janssen-Cliag, Neuss, Germany) / 15 mg/kg Ketamine (Narketan®, Chassot, Ravensburg, Germany) / 0.04 mg/kg Atropine (Eifelfango, Bad Neuenahr - Ahrweiler, Germany). Anesthesia was sustained with 1 % Propofol (Fresenius, Bad Homburg, Germany).

**Reference Example 16: Nonviral magnetofection in the ilea of rats**

**Vector preparation:** A stock solution of 800 $\mu$g transMAG-PEI 16/1 per ml 5 % glucose was added to a stock solution of 400 $\mu$g plasmid DNA (pCMV-ßgal) per ml in 5 % glucose while vortexing. Doses of 1 ml (corresponding to 200 $\mu$g DNA per animal) were injected 30 min after vector preparation.
**Anesthesia:** 75 $\mu$g/kg body weight Medetomidine / 1 mg/kg Midazolame / 2.5 $\mu$g/kg Fentanyl 25.
After laparatomy of anesthetized Wistar rats in the linea alba region, ileum and caecum were exposed and the guts were clamped off 8 cm in oral direction of the ileo-caecal junction. Ingested material was carefully rinsed towards the caecum by application of 1 ml of isotonic saline. Then, a second clamp was placed 3 cm aborally from the first clamp. The vector preparation was injected with a 20G needle adjacent to the first clamp. The injection site was closed with surgical suture while a sterile magnet block (20 x 10 x 5 mm; NeoDelta, IBS Magnet, Berlin, Germany) was placed under the clamped-off section for magnetofection, while in control animals, the treatment was performed without positioning of a magnet. Five min post injection, both clamps were removed. The magnet was left for a total of 20 min. Subsequently, the guts were returned carefully into the abdominal cavity which was closed with surgical suture. Anesthesia was antagonized with 375 $\mu$g/kg body weight Atipamezol /100 $\mu$g/kg Flumazenil / 60 $\mu$g/kg Naxolon. The animals were sacrificed after 48 hrs.
The treated section of the guts and adjacent areas were isolated, rinsed exhaustively with PBS and fixed for 30 min with 2 % formaldehyde and 0.2 % glutaraldehyde in PBS. The tissue was rinsed again with PBS followed by 4 hrs X-Gal staining at 37°C. Subsequently, the tissue was again rinsed exhaustively with PBS and stored over night at 4°C in 2 % formaldehyde/PBS followed by embedding for paraffin and cryosections. Sections were stained with eosin (see Figure 16).

**Reference Example 17: Magnetofection in the ear arteries of rabbits**

Vector preparation: A stock solution of 800 $\mu$g transMAG-PEI 16/1 per 750 $\mu$l 5 % glucose was added to a stock solution of 400 $\mu$g plasmid DNA (pCMV-Luc) per 750 $\mu$l in 5 % glucose while vortexing. Doses of 1.5 ml (corresponding to 400 $\mu$g DNA per animal) were injected 30 min after vector preparation.
The animals were anesthetized by an i.m. injection of 40 mg/kg body weight Ketamine / 20 mg/kg Xylazine. At one ear, a magnet block (NeoDelta, 2 x 1 x 0.5 cm, IBS magnet, Berlin, Germany) was placed distal of the injection site above-the ear artery using a custom-made device which prevented obstruction of the artery. The vector dose (1.5 ml each) was infused over 1.5 min in both ears. One hour after injection, the magnet was removed. The animals were sacrificed after 42 hours and segments of the ear arteries from upstream of the magnet position, the magnet position and downstream of the magnet position were isolated. From the control ears, the topologically analogous segments were isolated. The samples were rinsed with PBS buffer and homogenized 2 times for 30 seconds in 2 ml screw cap tubes (VWR scientific products, West Chester, USA) supplied with 500 $\mu$l lysis buffer (Promega, Mannheim, Germany; containing one Complete Protease Inhibitor Cocktail Tablet per 50 ml, Roche, Penzberg, Germany) and approx. 800-850 mg of zirconia beads (2,5 mm diameter, Biospec Products, Inc., Bartlesville, USA). Homogenization was carried out using a Mini Bead Beater (Biospec Products, Inc., Bartlesville, USA). After centrifugation at 20.000 x g at 4°C for 10 min, 50 $\mu$l aliquots were mixed with 100 $\mu$l of luciferase buffer (Promega Luciferase Assay System, Promega Corporation, Madison, USA) in a black Costar® 96-well-plate (opaque Plate-solid black 96 well, Corning Costar Corporation, Cambridge, USA) and counted for bioluminescence using a TopCount instrument (Canberra Packard, Dreieich, Germany).

Count time set to 12 sec, count delay 1 min). The results are shown in Figure 17B and C.

**Example 18: Magnetofection of porcine trachea ex vivo**

Vector preparation:

**(A)** Twelve microliters of chemically inactivated adenovirus (aprox. $3.4 \times 10^{10}$ viral particles) diluted to 200 $\mu$l in HBS buffer were mixed with 1200 ng streptavidin-polylysine (also dissolved in 200 $\mu$l of HBS). After 15 min, this was mixed with 24 $\mu$g DNA (pCMV-Luc) dissolved in 300 $\mu$l of HBS. After further 15 min, the resulting complex was mixed with 24 $\mu$g transferrin-polylysine dissolved in 300 $\mu$l HBS which had been mixed with 48 $\mu$g transMAG-PEI immediately before that. After 30 min, the DNA complex was applied to the trachea sample as described below.
**(B)** Twenty-four $\mu$g transMAG-PEI suspended in 250 $\mu$l of DMEM (without additives) were mixed with 12 $\mu$g of DNA in the same volume of DMEM followed by immediate addition to and mixing with 60 $\mu$l of GenePorter™ (Gene Therapy Systems, Inc., San Diego, CA, USA). After 30 min, the DNA complex was applied to the trachea sample as described below.

Trachea sections were isolated immediately after euthanization of animals. Subsequently the epithelial layer was dissected free of the majority of muscle and adventitia and the dissected tissues were placed in petri dishes containing DMEM supplemented with 10 % FCS and penicillin/streptomycin. The tissue was then dissected into pieces of approx. 2 x 1.5 cm. A Neodelta magnets (2 x 1 x 0.5 cm; IBS Magnet, Berlin, Germany) was placed in one well of a six well plate which then was filled with DMEM (containing 10 % FCS, penicillin and streptomycin) to the upper edge of the magnet. A trachea sample was spread directly on the magnet so that its basolateral face was in contact with medium while the airway epithelium was exposed at the air-liquid interface. The bottom of a second well was covered with medium and a trachea sample was placed in it. The DNA complex (250 $\mu$l each, corresponding to 6 $\mu$g of DNA) was trickled onto the trachea samples from above. After 30 min, the magnet was removed. The trachea sample was washed with fresh medium and cultivated over night. The luciferase assay was carried out after homogenization of the tissue as described in Example 17. Luciferase expression was 150,390 light units in 50 $\mu$l of the tissue homogenate from the sample under influence of the magnet and 3785 light units in the sample where no magnetic field was applied.

In a second setup, two trachea samples were placed in two wells of two separate 24 well plates with the basolateral side down. One plate was positioned upon a magnetic plate in 24 well plate format. The DNA complex (250 $\mu$l of DNA/transMAG-PEI/GenePorter in DMEM corresponding to 6 $\mu$g of DNA) was carefully distributed over the surface of the trachea samples. After 20 min, the magnetic plate was removed, the trachea samples were washed and cultivated over night with complete DMEM (containing 10 % FCS and penicillin/streptomycin). The luciferase assay was carried out as described in Example 17. Luciferase expression was zero light units without magnetic field and 5,870 light units with magnetic field.

**Example 19: Transfection of NIH3T3 cells with Superfect ± transMAG-PEI**

**Cells:** 18,000 cells seeded in 96-well plates 6 hrs prior transfection.
**Starting concentration:** 1 $\mu$g DNA / well
50 $\mu$l transfection cocktail per well added to cells in 150 $\mu$l complete DMEM.
Superfect:DNA N/P ratios = 2, 4 and 6.
transMAG-PEI:DNA ratio (w/w) = 1:1
**DNA stock:** 80 $\mu$g/ml pCMV-Luc in water.
**transMAG-PEI stock:** 80 $\mu$g/ml in water.
**Superfect stock solutions:** 40 / 80 / 120 $\mu$g as supplied from the manufacturer diluted to 250 $\mu$l each with water.
**DNA complex preparation:** For control experiments, 4.8 $\mu$g DNA in 80 $\mu$l water each were added to and mixed with 3.2, 6.4 and 9.6 $\mu$l of Superfect as supplied by the manufacturer diluted to 80 $\mu$l each with water. After 15 min, the resulting complexes were added to and mixed with 80 $\mu$l 450 mM sodium chloride each. Magnetofection: For each N/P ratio, 250 $\mu$l each of transMAG-PEI stock were added to and mixed with an equal volume of DNA stock. After 15 min incubation, the resulting suspension was added to and mixed with the respective Superfect stock solutions. After further 15 min incubation, the resulting complexes were added to and mixed with 250 $\mu$l each of 600 mM sodium chloride.
**Dilution series:** After 25 min incubation, four 240 $\mu$l aliquots of each N/P ratio were added to row A of a U-bottom 96-well plate. All other wells were filled with 120 $\mu$l 150 mM sodium chloride. Using a multichannel pipettor, 120 $\mu$l each were transferred from row A to row B, mixed by pipetting, then 120 $\mu$l each were transferred from row B to row C, etc.

**Transfection:** Cells were supplemented with fresh complete DMEM prior transfection. Fifty microliters each of DNA complexes were transferred per well to the cells in 2 separate plates. One plate was placed on the magnetic plate in 96-well format. After 15 min incubation, the plates were washed once with 150 $\mu$l fresh complete DMEM per well and then incubated in complete DMEM until luciferase assay after 24 hrs. The results are shown in Figure 18.

**Example 20: Transfection of NIH3T3 cells with Superfect $\pm$ transMAG-PEI to study the influence of a magnetic field apart from its role in particle concentration at the cell surface**

**Cells:** 18,000 cells seeded in 96-well plates 6 hrs prior transfection.
**Starting concentration:** 0.5 $\mu$g DNA / well.
50 $\mu$l transfection cocktail per well added to cells in 150 $\mu$l complete DMEM.
Superfect:DNA N/P ratio = 6.
transMAG-PEI:DNA ratio (w/w) = 1:1
**DNA stocks:** Six tubes containing 10 $\mu$g pCMV-Luc in 250 $\mu$l water each.
**transMAG-PEI stocks:** Six tubes containing 0, 2, 4, 6, 8, and 10 $\mu$g transMAG-PEI in 250 $\mu$l water each.
**Superfect stock solutions:** Six tubes containing 60 $\mu$g Superfect in 250 $\mu$l water each.
**DNA complex preparation:** transMAG-PEI stocks were added to and mixed with DNA stocks, incubated for 15 min followed by addition of the resulting complexes to and mixing with the Superfect stocks, incubation for 15 min, followed by addition of the resulting complexes to and mixing with 250 $\mu$l 600 mM sodium chloride each. **Dilution series:** After 25 min incubation, four 240 $\mu$l aliquots of each transMAG-DNA ratio were added to rows A and E of a U-bottom 96-well plate. All other wells were filled with 120 $\mu$l 150 mM sodium chloride. Using a multichannel pipettor, 120 $\mu$l each were transferred from row A to row B, mixed by pipetting, then 120 $\mu$l each were transferred from row B to row C, etc., and in analogous manner from row E to row F etc.
**Transfection:** Cells were supplemented with fresh complete DMEM prior transfection. Fifty microliters each of DNA complexes were transferred per well to the cells in 2 separate plates. After 20 min incubation, the cells were washed once with 150 $\mu$l fresh complete DMEM per well and then incubated in complete DMEM until luciferase assay after 24 hrs. One of the 2 plates was however positioned on the magnetic plate in 96-well format after the washing procedure for 40 min. The results are shown in Figure 19.

**Example 21: Transfection of CHO-K1 cells with PEI-DNA + transMAGs with various polycationic surface coatings**

**Cells:** 19,000 cells seeded in 96-well plates the day prior transfection.
**General settings:** 0.5 $\mu$g DNA / well.
50 $\mu$l transfection cocktail per well added to cells in 150 $\mu$l complete DMEM.
PEI:DNA N/P ratio = 8.
transMAG-PEI:DNA ratios (w/w) = 0.4, 0.8, 1, 2, 4, 8.
**DNA stock:** 40 $\mu$g/ml in water.
**PEI stock:** 41.7 $\mu$g/ml in water.
**transMAGs:** transMAG-DEAE, transMAG-DAEA, transMAG-STARCH-PEI, transMAG-PEI-ethoxylated, transMAG-PEI-epichlorhydrine, transMAG-PEI-SDS, transMAG-PEI-lowMW, transMAG-PEI-C1/1.
**Vector preparation:** 125 $\mu$l DNA stock each were added to the following amounts of transMAG suspensions, each in 125 $\mu$l of water, and mixed by pipetting:

| transMAG:DNA (w/w) | 0.4 | 0.8 | 1 | 2 | 4 | 8 |
|---|---|---|---|---|---|---|
| $\mu$g transMAG in 125 $\mu$l water | 2 | 4 | 5 | 10 | 20 | 30 |

After 15-30 min incubation, 125 $\mu$l each of PEI stock solution were added and mixed. After further 15-30 min, 125 $\mu$l each of 600 mM sodium chloride were added and mixed. After approx. 45 min of incubation, 50 $\mu$l of each DNA complex formulation were added to the cells in quadruples on two separate plates. Just prior to this, the cells had been

supplemented with 150 μl of fresh complete DMEM. During the following 15 min incubation, one plate was positioned upon the magnetic plate in 96-well format. After that, the cells were washed once with complete DMEM followed by incubation over night until luciferase assay. Figure 20 shows that, in principle, every kind of magnetic particles used for magnetofection in those experiments causes an increase of gene transfer in the presence of a magnetic field compared to its absence.

**Example 22: Transfection of NIH3T3 cells with DNA + transMAGs with various PEI surface coatings. Vector preparation in glucose and salt-containing solutions. Titration of optimal transMAG:DNA ratios**

Cells: 18,500 cells seeded in 96-well plates the day prior transfection.
General settings: 1 μg DNA / well.
50 μl transfection cocktail per well added to cells in 150 μl complete DMEM.
transMAG-PEI:DNA ratios (w/w) = 20, 13.33, 8.89, 5.93, 3.95, 2.63, 1.76, 1.17, 0.78, 0.52, 0.35, 0.
DNA stocks: 40 μg/ml in 5 % glucose and in 300 mM sodium chloride, respectively. transMAGs and dilution series: transMAG-1811, transMAG-19/1, transMAG-PEI-21/1, transMAG-23/1, transMAG-24/1, transMAG-25/1, trans-MAG-37, transMAG-38.
Two identical dilution series were carried out in 5 % glucose and in water. Ninety μl stock solutions in 5 % glucose and water, respectively, each containing 72 μg of transMAGs were added in triplicates to the first columns of U-bottom 96-well plates. All other wells were filled with 30 μl 5 % glucose and water, respectively. Using a multichannel pipettor, 60 μl each were transferred from column 1 to column 2, mixed by pipetting, 60 μl each were transferred from column 2 to column 3 and so on. Column 12 was left containing 30 μl of 5 % glucose and water, respectively. Vector preparation: Thirty μl each of DNA stock solution in 5 % glucose or 300 mM sodium chloride were added to and mixed with the dilution series in 5 % glucose and water, respectively, using a multichannel pipettor.
Transfection: After 30 min of incubation, 50 μl each of the DNA complex formulations were transferred to the cells in two separate plates. Just prior to this, the cells had been supplemented with 150 μl of fresh complete DMEM. During the following 20 min incubation, the plates were positioned upon magnetic plates in 96-well format. After that, the cells were washed once with complete DMEM followed by incubation over night until luciferase assay. The results of these experiments are shown in Figure 21.

**Example 23: Transfection of NIH3T3 and HepG2 cells with transMAG-pASP-DNA and various amounts of PEI**

Cells: 35.000 NIH3T3 cells and 45,000 HepG2 cells were seeded per well in 96-well plates 5 hrs prior transfection.
General settings: 0.5 μg DNA / well.
50 μl transfection cocktail per well added to cells in 150 μl complete DMEM.
PEI:DNA N/P ratios = 0, 1, 2, 3, 4, 5, 6, 7, 8, 9 10, 11.
transMAG-pASP:DNA ratio (w/w) = 1.
DNA stock: 40 μg/ml in 20 mM HEPES pH 7.4.
transMAG-pASP stock: 40 μg/ml in 20 mM HEPES pH 7.4.
PEI stocks:

| PEI:DNA (N/P ratio) | 0 | 1 | 2 | 3 | 4 | 5 |
|---|---|---|---|---|---|---|
| PEI (μg/ml in 20 mM HEPES) | 0 | 5.21 | 10.42 | 15.64 | 20.85 | 26.06 |
| PEI:DNA (N/P ratio) | 6 | 7 | 8 | 9 | 10 | 11 |
| PEI (μg/ml in 20 mM HEPES) | 31.27 | 36.49 | 41.70 | 46.91 | 52.12 | 57.33 |

Vector preparation: The DNA and transMAG-pASP stock solutions were mixed at a 1:1 vol/vol ratio. Of the resulting mixture, 430 μl each were added to 215 μl each of the PEI stocks and mixed. After 15 min incubation, the resulting complexes were mixed with 215 μl each of 600 mM sodium chloride in 20 mM HEPES pH 7.4.
Transfection: After approx. 30 min of incubation, 50 μl of each DNA complex formulation was added to the cells in quadruples on two separate plates of each cell line. Just prior to this, the cells had been supplemented with 150 μl fresh complete DMEM. During the following 10 min incubation, one plate per cell line was positioned upon a magnetic plate in 96-well format. After that, the medium was exchanged for fresh complete DMEM without washing. The cells were grown for 24 hrs until luciferase assay. The outcome of these assays is presented in Figure 22.

**Example 24: Transfection of CHO-K1 cells with GenePorter-DNA $\pm$ transMAG-PEI**

**Cells:** 19,500 cells per well seeded in 96-well plates the day prior transfection.
**Starting concentration:** 0.1 $\mu$g DNA / well.
100 $\mu$l transfection volume / well in serum-free DMEM.
5 $\mu$l GenePorter / 1 $\mu$g DNA.
**DNA stock:** 24 $\mu$g in 1.2 ml DMEM.
144 $\mu$l DNA stock each were added to the following amounts of transMAG-PEI suspensions in 144 $\mu$l of DMEM and mixed by pipetting:

| transMAG:DN A (w/w) | 0 | 0,5 | 1 | 2 | 4 | 6 | 8 | 10 |
|---|---|---|---|---|---|---|---|---|
| $\mu$g transMAG-PEI in 144 $\mu$l DMEM | 0 | 1.44 | 2.88 | 5.76 | 11.52 | 14.4 | 23.03 | 28.8 |

Incubation was not longer than the required handling time. The transMAG-DNA mixtures were immediately added and mixed to 8 tubes containing 14.4 $\mu$l of GenePorter diluted to 288 $\mu$l with DMEM. After 20 min incubation, the DNA complexes were filled up to 2880 $\mu$l with DMEM. Then, 3 x 230 $\mu$l of each composition (triplicates) were added consecutively to rows A and E, respectively, of 4 round bottom 96-well plates. All other rows were filled with 115 $\mu$l DMEM.
**Dilution series:** Using a multichannel pipettor, 115 $\mu$l each were transferred from row A and E, respectively, to rows B and F, respectively, mixed by pipetting, then 115 $\mu$l each were transferred from row B and F, respectively, to rows C and G, etc. Total handling time was about 20 min.
**Transfection:** Serum-containing medium was removed from 4 plates and replaced with 100 $\mu$l each of the DNA complex dilution series. Two plates were incubated for 10 min, followed by washing with complete DMEM (10 % FCS, Pen, Strep). Two plates were incubated for 4 hrs, followed by addition of 100 $\mu$l each of DMEM containing 20 % FCS (complexes were not removed from cells). For each incubation time, one plate was kept on the magnetic plate throughout the incubation time. Cells were harvested for luciferase assay 24 hrs after transfection. The results are shown in Figure 23.

**Example 25: Transfection of CHO-K1 cells with lipofectamine $\pm$ transMAG-PEI**

**Cells:** 19,500 cells seeded in 96-well plates the day prior transfection.
**Starting concentration:** 0.1 $\mu$g DNA / well.
100 $\mu$l transfection volume / well in serum-free DMEM.
4 $\mu$l Lipofectamine / 1 $\mu$g DNA.
**DNA stock:** 24 $\mu$g in 1.2 ml DMEM.
144 $\mu$l DNA stock each were added to the following amounts of transMAG-PEI suspensions in 144 $\mu$l of DMEM and mixed by pipetting:

| transMAG:DN A (w/w) | 0 | 0,5 | 1 | 2 | 4 | 6 | 8 | 10 |
|---|---|---|---|---|---|---|---|---|
| $\mu$g transMAG-PEI in 144 $\mu$l DMEM | 0 | 1.44 | 2.88 | 5.76 | 11.52 | 14.4 | 23.03 | 28.8 |

Incubation was not longer than the required handling time. The transMAG-DNA mixtures were immediately added and mixed to 8 tubes containing 11.52 $\mu$l of Lipofectamine diluted to 288 $\mu$l with DMEM. After 20 min incubation, the DNA complexes were filled up to 2880 $\mu$l with DMEM. Then, 3 x 230 $\mu$l of each composition (triplicates) were added consecutively to rows A and E, respectively, of 4 round bottom 96-well plates. All other rows were filled, with 115 $\mu$l DMEM.
**Dilution series:** Using a multichannel pipettor, 115 $\mu$l each were transferred from row A and E, respectively, to rows B and F, respectively, mixed by pipetting, then 115 $\mu$l each were transferred from row B and F, respectively, to rows C and G, etc. Total handling time was about 20 min.
**Transfection:** Serum-containing medium was removed from 4 plates and replaced with 100 $\mu$l each of the DNA complex dilution series. Two plates were incubated for 10 min, followed by washing with complete DMEM (10 % FCS, Pen, Strep). Two plates were incubated for 4 hrs, followed by addition of 100 $\mu$l each of DMEM containing 20 % FCS (complexes were not removed from cells). For each incubation time, one plate was kept on the magnetic plate throughout the incubation time. Cells were harvested for luciferase assay 24 hrs after transfection (see Figure 24).

**Example 26: Transfection of CHO-K1 cells with DOCHOL-DNA ± transMAG-PEI**

**Cells:** 19,500 cells seeded in 96-well plates the day prior transfection.
**Starting concentration:** 0.5 $\mu$g DNA / well.
50 $\mu$l transfection cocktail / well added to cells in 150 $\mu$l complete DMEM
DOTAP:DNA charge ratio = 5:1.
**DNA stock:** 92.16 $\mu$g in 2304 $\mu$l water
**DOCHOL stock solution:** 279.3 $\mu$l 5 mM DOTAP-Cholesterol liposomes diluted to 2304 $\mu$l with water.
250 $\mu$l DNA stock each were added to the following amounts of transMAG-PEI suspensions in 250 $\mu$l water and mixed by pipetting:

| transMAG:DN A (w/w) | 0 | 0,2 | 0.4 | 0.6 | 0.8 | 1 | 2 | 4 |
|---|---|---|---|---|---|---|---|---|
| $\mu$g transMAG-PEI in 250 $\mu$l water | 0 | 2 | 4 | 6 | 8 | 10 | 20 | 40 |

After 15 min incubation, the resulting mixtures were added to 250 $\mu$l each of DOCHOL stock solution and mixed. After further 15 min, the resulting DNA complexes were added to and mixed with 250 $\mu$l each of 600 mM sodium chloride, followed by 30 min incubation.
**Dilution series:** Four times 240 $\mu$l of each vector preparation were added per well in columns 1 and 7, respectively, of two U-bottom 96-well plates. All other wells were filled with 120 $\mu$l 150 mM sodium chloride. Using a multichannel pipettor, 120 $\mu$l each were transferred from column 1 and 7, respectively, to columns 2 and 8, respectively, mixed by pipetting, then 120 $\mu$l each were transferred from columns 2 and 8, respectively, to columns 3 and 9, etc.
**Transfection:** Cells were supplemented with fresh complete DMEM prior transfection. Fifty microliters each of DNA complexes were transferred per well to the cells in 2 times 2 separate plates. One plate each of the 2 times 2 plates having received identical transfection cocktails was placed on the magnetic plate in 96-well format. After 10 min incubation, all plates were washed once with 150 $\mu$l fresh complete DMEM per well and then incubated in complete DMEM until luciferase assay after 24 hrs (see Figure 25).

**Example 27: Kinetics of magnetofection with cationic lipids in NIH3T3 cells**

**Cells:** 22,000 NIH3T3 cells per well seeded in two separate 96-well plates the day prior transfection.
**DNA dose:** 0.1 $\mu$g pCMV-Luc per well.
**TransMAG-PEI:DNA ratio (w/w):** 2:1.
**DNA stock:** 20 $\mu$g/ml in DMEM.
**Vector preparation:** In two times two separate setups, 216 $\mu$l DNA stock (corresponding to 4.32 $\mu$g DNA) were mixed a) with an equal volume of DMEM, b) with an equal volume of DMEM containing 8.64 $\mu$g transMAG-PEI. The transMAG-PEI suspensions in DMEM were prepared freshly immediately preceeding this step. The resulting suspensions were mixed immediately with a) 17.28 $\mu$l Lipofectamine® diluted to 432 $\mu$l with DMEM, b) with 21.6 $\mu$l GenePorter™ diluted to 432 $\mu$l with DMEM. After 20 min of incubation, the resulting complexes were diluted to 4320 $\mu$l with DMEM. This resulted in the following four vector preparations: 1. Lipofectamine-DNA (4 $\mu$l Lipofectamine per $\mu$g of DNA); 2. Lipofectamine/transMAG-PEI/DNA (4 $\mu$l Lipofectamine and 2 $\mu$g transMAG-PEI per $\mu$g DNA); 3. GenePorter/DNA (5 $\mu$l GenePorter per $\mu$g of DNA); 4. GenePorterttransMAG-PEI/DNA (5 $\mu$l GenePorter and 2 $\mu$g transMAG-PEI per $\mu$g of DNA).
**Transfection:** The culture medium was removed from the cells in two separate 96-well plates. Eighteen wells each on each plate were filled with 100 $\mu$l each of the four different DNA complex preparations. One of the two plates was positioned on the magnetic plate in 96-well format. Five, 10, 20, 40 and 240 minutes after DNA complex addition, the transfection cocktails were removed from 3 wells each for each DNA complex preparation on both plates, the cells were washed once with fresh complete medium (DMEM containing 10 % FCS and penicillin/streptomycin) and then cultivated in complete medium until the luciferase assay was carried out approx. 20 hrs after transfection. The magnetic plate was removed after the last time point (240 minutes). The results of these experiments are presented in Figure 26.

**Example 28: Retroviral magnetofection**

NIH 3T3 cells (1 x $10^5$ cells plated on 35 mm dishes 24 h before infection) were incubated for 3 hrs with 1 ml aliquots of 24 hr supernatants from low titer MuLV producing ecotropic packaging cells (subclone A6.LT of GP86-NA. 6; ~1-5 x $10^3$ Xgal CFU/ml; Kruger et al. 1994). These supernatants were applied untreated or treated with transMAG$^{PEI}$ (3 μg/ml for 20 min) and/or polybrene (8 μg/ml immediately prior infection). Magnets were applied to specified groups for 1 h. After 48 h, the cells were stained with X-Gal, and blue nuclei were counted (Figure 27).

**Example 29: Retroviral magnetofection - vector accumulation at target cells by magnetic field compared to vector accumulation by centrifugal force**

NIH3T3 cells were seeded in 96-well plates at a density of 7,000 cells per well the day prior transduction. Three milliliter aliquots of 24 hr supernatants from low titer MuLV producing ecotropic packaging cells (subclone A6.LT of GP86-NA.6; ~1-5 x $10^3$ Xgal CFU/ml) were mixed and incubated with 9 μg, 27 μg and 45 μg of transMAG-PEI. Fifty μl aliquots of these preparations were added to the NIH3T3 cells from which the medium had just been removed. This 96-well plate was positioned upon the magnetic plate in 96-well format for one hour. Then, the wells were filled up with complete medium to 200 μl and incubated for 48 hrs.

For comparison, the retroviral supernatant was mixed with 8 μg/ml polybrene and cells were incubated with identical virus doses as during magnetofection for 48 hrs on two plates. One of the two plates was centrifuged at 1330 x g at 37°C for 90 minutes and then returned to the incubator. ß-galactosidase expression was determined after 48 hours using the CPRG stain of cell lysates (Plank et al.1999). Figure 28 shows the results of these experiments. The data confirms that retroviral magnetofection is superior to the standard polybrene-mediated transduction. Standard transduction assisted by centrifugation improves transduction efficiency by about 2-fold. However, highest transduction levels are achieved by magnetofection, dependent on the transMAG to virus ratio.

**REFERENCES**

1. Babincova, M., Altanerova, V., Lampert, M., Altaner, C., Machova, E., Sramka, M. and Babinec, P. (2000). "Site-specific in vivo targeting of magnetoliposomes using externally applied magnetic field." Z Naturforsch [C] 55(3-4): 278-81.

2. Bailey, A. L. and Sullivan, S. M. (2000). "Efficient encapsulation of DNA plasmids in small neutral liposomes induced by ethanol and calcium." Biochim Biophys Acta 1468(1-2): 239-52.

3. Bergemann, C., Muller-Schulte, D., Oster, J., à Brassard, L. and Lübbe, A. S. (1999). "Magnetic ion-exchange nano- and microparticles for medical, biochemical and molecular biological applications." J. Magnetism and Magnetic Materials 194: 45-52.

4. Bildirici, L., Smith, P., Tzavelas, C., Horefti, E. and Rickwood, D. (2000). "Transfection of cells by immunoporation." Nature 405: 298.

5. Bonadio, J., Goldstein, S. A. and Levy, R. J. (1998). "Gene therapy for tissue repair and regeneration." Advanced Drug Delivery Reviews 33(1-2): 53-69.

6. Boussif, O., Lezoualch, F., Zanta, M. A., Mergny, M. D., Scherman, D., Demeneix, B. and Behr, J. P. (1995). "A Versatile Vector For Gene And Oligonucleotide Transfer Into Cells In Culture And In Vivo - Polyethylenimine." Proceedings Of The National Academy Of Sciences Of The United States Of America 92(16): 7297-7301.

7. Carson, J. J., Prato, F. S., Drost, D. J., Diesbourg, L. D. and Dixon, S. J. (1990). "Time-varying magnetic fields increase cytosolic free Ca2+ in HL-60 cells." Am J Physiol 259(4 Pt 1): C687-92.

8. Chen, C. A. and Okayama, H. (1988). "Calcium phosphate-mediated gene transfer: a highly efficient transfection system for stably transforming cells with plasmid DNA." Biotechniques 6(7): 632-8.

9. Cohen, H., Levy, R. J., Gao, J., Fishbein, I., Kousaev, V., Sosnowski, S., Slomkowski, S. and Golomb, G. (2000). "Sustained delivery and expression of DNA encapsulated in polymeric nanoparticles." Gene Ther7(22): 1896-905.

10. Colestrauss, A., Yoon, K. G., Xiang, Y. F., Byrne, B. C., Rice, M. C., Gryn, J., Holloman, W. K. and Kmiec, E. B. (1996). "Correction of the Mutation Responsible For Sickle Cell Anemia By an Rna-Dna Oligonucleotide." Science 273(5280): 1386-1389.

11. Curiel, D. T. (1999). "Strategies to adapt adenoviral vectors for targeted delivery." Ann N Y Acad Sci 886: 158-71.

12. Curiel, D. T., Agarwal, S., Wagner, E. and Cotten, M. (1991). "Adenovirus Enhancement Of Transferrin Polylysine-Mediated Gene Delivery." Proceedings Of The National Academy Of Sciences Of The United States Of America 88(19): 8850-8854.

13. Fahlvik, A. K., Klaveness, J. and Stark, D. D. (1993). "Iron oxides as MR imaging contrast agents." J Magn Reson Imaging 3(1): 187-94.

14. Fasbender, A., Lee, J. H., Walters, R. W., Moninger, T. O., Zabner, J. and Welsh, M. J. (1998). "Incorporation of adenovirus in calcium phosphate precipitates enhances gene transfer to airway epithelia in vitro and in vivo." J Clin Invest 102(1): 184-93.

15. Fasbender, A., Zabner, J., Chillon, M., Moninger, T. O., Puga, A. P., Davidson, B. L. and Welsh, M. J. (1997). "Complexes of adenovirus with polycationic polymers and cationic lipids increase the efficiency of gene transfer in vitro and in vivo." J Biol Chem 272(10): 6479-89.

16. Felgner, P. L., Barenholz, Y., Behr, J. P., Cheng, S. H., Cullis, P., Huang, L., Jessee, J. A., Seymour, L., Szoka, F., Thierry, A. R., Wagner, E. and Wu, G. (1997). "Nomenclature for synthetic gene delivery systems." Hum Gene Ther 8(5): 511-2.

17. Felgner, P. L. and Ringold, G. M. (1989). "Cationic liposome-mediated transfection." Nature 337: 387-388.

18. Fender, P., Ruigrok, R. W., Gout, E., Buffet, S. and Chroboczek, J. (1997). "Adenovirus dodecahedron, a new vector for human gene transfer." Nat Biotechnol 15(1): 52-6.

19. Finsinger, D., Remy, J. S., Erbacher, P., Koch, C. and Plank, C. (2000). "Protective copolymers for nonviral gene vectors: synthesis, vector characterization and application in gene delivery." Gene Ther7(14): 1183-92.

20. Freund, O., Mahy, P., Amedee, J., Roux, D. and Laversanne, R. (2000). "Encapsulation of DNA in new multilamellar vesicles prepared by shearing a lyotropic lamellar phase." J Microencapsul 17(2): 157-68.

21. Gao, X. and Huang, L. (1996). "Potentiation Of Cationic Liposome-Mediated Gene Delivery By Polycations." Biochemistry 35(3): 1027-1036.

22. Gill, I. and Ballesteros, A. (2000). "Bioencapsulation within synthetic polymers (Part 1): sol-gel encapsulated biologicals." Trends Biotechnol 18(7): 282-96.

23. Gossfing, H. R., Bernstein, R. A. and Abbott, J. (1992). "Treatment of ununited tibial fractures: a comparison of surgery and pulsed electromagnetic fields (PEMF)." Orthopedics 15(6): 711-9.

24. GrillotCourvalin, C., Goussard, S., Huetz, F., Ojcius, D. M. and Courvalin, P. (1998). "Functional gene transfer from intracellular bacteria to mammalian cells." Nature Biotechnology 16(9): 862-866.

25. Haensler, J. and Szoka, F. C. (1993). "Polyamidoamine Cascade Polymers Mediate Efficient Transfection Of Cells In Culture." Bioconjugate Chemistry 4(5): 372-379.

26. Hiemenz, P. C. (1986). Principles of Colloid and Surface Chemistry, Marcel Dekker, Inc. New York.

27. Hirosue, S., Muller, B. G., Mulligan, R. C. and Langer, R. (2001). "Plasmid DNA encapsulation and release from solvent diffusion nanospheres." J Control Release 70(1-2): 231-42.

28. Isner, J. M., Walsh, K., Symes, J., Pieczek, A., Takeshita, S., Lowry, J., Rosenfield, K., Weir, L., Brogi, E. and Jurayj, D. (1996). "Arterial gene transfer for therapeutic angiogenesis in patients with peripheral artery disease." Hum. Gene Ther. 20(7): 959-988.

29. Kalyanasundaram, S., Feinstein, S., Nicholson, J. P., Leong, K. W. and Garver, R. I., Jr. (1999). "Coacervate microspheres as carriers of recombinant adenoviruses." Cancer Gene Ther 6(2): 107-12.

30. Kaneda, Y. (1998). "Fusigenic Sendai-virus liposomes: a novel hybrid type liposome for gene therapy." Biogenic Amines 14(5): 553-572.

31. Kasahara, N., Dozy, A. M. and Kan, Y. W. (1994). "Tissue-specific targeting of retroviral vectors through ligand-receptor interactions." Science 266(5189): 1373-6.

32. Kass-Eisler, A., Leinwand, L., Gall, J., Bloom, B. and Falck-Pedersen, E. (1996). "Circumventing the immune response to adenovirus-mediated gene delivery." Gene Therapy 3: 154-162.

33. Kruger, A., Schirrmacher, V. and von Hoegen, P. (1994). "Scattered micrometastases visualized at the single-cell level: detection and re-isolation of lacZ-labeled metastasized lymphoma cells." Int J Cancer 58(2): 275-84.

34. Lee, R. J. and Huang, L. (1997). "Lipidic vector systems for gene transfer." Crit. Rev. Ther. Drug Carrier Syst. 1997 14(2)(2): 173-206.

35. Leong, K. W., Mao, H. Q., Truong-Le, V. L., Roy, K., Walsh, S. M. and August, J. T. (1998). "DNA-polycation nanospheres as non-viral gene delivery vehicles." J Control Release 53(1-3): 183-93.

36. Li, S., Rizzo, M. A., Bhattacharya, S. and Huang, L. (1998). "Characterization of cationic lipid-protamine-DNA (LPD) complexes for intravenous gene delivery." Gene Therapy 5(7): 930-937.

37. Lübbe, A. S., Bergemann, C., Huhnt, W., Fricke, T., Riess, H., Brock, J. W. and Huhn, D. (1996). "Preclinical experiences with magnetic drug targeting: tolerance and efficacy." Cancer Res 56(20): 4694-701.

38. Lübbe, A. S., Bergemann, C., Riess, H., Schriever, F., Reichardt, P., Possinger, K., Matthias, M., Dorken, B., Herrmann, F., Gurtler, R., Hohenberger, P., Haas, N., Sohr, R., Sander, B., Lemke, A. J., Ohlendorf, D., Huhnt, W. and Huhn, D. (1996). "Clinical experiences with magnetic drug targeting: a phase I study with 4'-epidoxorubicin in 14 patients with advanced solid tumors." Cancer Res 56(20): 4686-93.

39. Luo, D. and Saltzman, W. M. (2000). "Enhancement of transfection by physical concentration of DNA at the cell surface." Nat Biotechnol 18(8): 893-5.

40. Meyer, K. B., Thompson, M. M., Levy, M. Y., Barron, L. G. and Szoka, F. C. (1995). "Intratracheal Gene Delivery To The Mouse Airway - Characterization Of Plasmid DNA Expression And Pharmacokinetics." Gene Therapy 2(7): 450-460.

41. Michael, S. I., Hong, J. S., Curiel, D. T. and Engler, J. A. (1995). "Addition of a short peptide ligand to the adenovirus fiber protein." Gene Ther 2(9): 660-8.

42. Nicolau, C. and Cudd, A. (1989). "Liposomes As Carriers Of DNA." Critical Reviews In Therapeutic Drug Carrier Systems 6(3): 239-271.

43. O'Riordan, C. R., Lachapelle, A., Delgado, C., Parkes, V., Wadsworth, S. C., Smith, A. E. and Francis, G. E. (1999). "PEGylation of adenovirus with retention of infectivity and protection from neutralizing antibody in vitro and in vivo." Hum Gene Ther 10(8): 1349-58.

44. Ogris, M., Brunner, S., Schuller, S., Kircheis, R. and Wagner, E. (1999). "PEGylated DNA/transferrin-PEI complexes: reduced interaction with blood components, extended circulation in blood and potential for systemic gene delivery." Gene Therapy 6(4): 595-605.

45. Padmanabhan, R., Howard, T., Gottesman, M. M. and Howard, B. H. (1993). "Magnetic affinity cell sorting to isolate transiently transfected cells, multidrug-resistant cells, somatic cell hybrids, and virally infected cells." Methods Enzymol 218: 637-51.

46. Petka, W. A., Harden, J. L., McGrath, K. P., Wirtz, D. and Tirrell, D. A. (1998). "Reversible hydrogels from self-assembling artificial proteins." Science 281(5375): 389-92.

47. Plank, C., Mechtler, K., Szoka, F. C. and Wagner, E. (1996). "Activation of the Complement System by Synthetic DNA Complexes: A Potential Barrier for Intravenous Gene Delivery." Hum. Gene Ther. 7(12): 1437-1446.

48. Plank, C., Oberhauser, B., Mechtler, K., Koch, C. and Wagner, E. (1994). "The Influence Of Endosome-Disruptive Peptides On Gene Transfer Using Synthetic Virus-Like Gene Transfer Systems." Journal Of Biological Chemistry 269(17): 12918-12924.

49. Plank, C., Tang, M., Wolfe, A. and Szoka, F. C. (1999). "Branched Cationic Peptides for Gene Delivery. Role of Type and Number of Cationic Residues in Formation and In Vitro Activity of DNA Polyplexes." Human Gene Therapy 10(2): 319-333.

50. Ponimaskin, E., Bareesel, K. K., Markgraf, K., Reszka, R., Lehmann, K., Gelderblom, H. R., Gawaz, M. and Schmidt, M. F. (2000). "Sendai virosomes revisited: reconstitution with exogenous lipids leads to potent vehicles for gene transfer." Virology 269(2): 391-403.

51. Poul, M. A. and Marks, J. D. (1999). "Targeted gene delivery to mammalian cells by filamentous bacteriophage." J Mol Biol 288(2): 203-11.

52. Quong, D. and Neufeld, R. J. (1999). "DNA encapsulation within co-guanidine membrane coated alginate beads and protection from extracapsular nuclease." J Microencapsul 16(5): 573-85.

53. Rajasubramanian, G., Meidell, R. S., Landau, C., Dollar, M. L., Holt, D. B., Willard, J. E., Prager, M. D. and Eberhart, R. C. (1994). "Fabrication of resorbable microporous intravascular stents for gene therapy applications." Asaio J 40(3): M584-9.

54. Reszka, R., Wegner, D., Richter, J., Pohl, B., Lehmann, C., Scheffel, A. and Gerbsch, N. (2000). Gene transfer using the cationic lipid-magnetosome-DNA complexes (CLMDC). Scientific and clinical applications of magnetic carriers, Rostock, May 3-6, Abstract book.

55. Romanczuk, H., Galer, C. E., Zabner, J., Barsomian, G., Wadsworth, S. C. and O'Riordan, C. R. (1999). "Modification of an adenoviral vector with biologically selected peptides: a novel strategy for gene delivery to cells of choice." Hum Gene Ther 10(16): 2615-26.

56. Rowley, J. A., Madlambayan, G. and Mooney, D. J. (1999). "Alginate hydrogels as synthetic extracellular matrix materials." Biomaterials 20(1): 45-53.

57. Roy, K., Mao, H. Q., Huang, S. K. and Leong, K. W. (1999). "Oral gene delivery with chitosan-DNA nanoparticles generates immunologic protection in a murine model of peanut allergy." Nat Med 5(4): 387-91.

58. Rubin, C. T., Donahue, H. J., Rubin, J. E. and McLeod, K. J. (1993). "Optimization of electric field parameters for the control of bone remodeling: exploitation of an indigenous mechanism for the prevention of osteopenia." J Bone Miner Res 8 Suppl 2: S573-81.

59. Shea, L. D., Smiley, E., Bonadio, J. and Mooney, D. J. (1999). "DNA delivery from polymer matrices for tissue engineering." Nat Biotechnol 17(6): 551-4.

60. Trock, D. H. (2000). "Electromagnetic fields and magnets. Investigational treatment for musculoskeletal disorders." Rheum Dis Clin North Am 26(1): 51-62, viii.

61. Truong-Le, V. L., Walsh, S. M., Schweibert, E., Mao, H. Q., Guggino, W. B., August, J. T. and Leong, K. W. (1999). "Gene transfer by DNA-gelatin nanospheres. "Arch Biochem Biophys 361(1): 47-56.

62. TruongLe, V. L., August, J. T. and Leong, K. W. (1998). "Controlled gene delivery by DNA-gelatin nanospheres." Human Gene Therapy 9(12): 1709-1717.

63. Wagner, E., Plank, C., Zatloukal, K., Cotten, M. and Birnstiel, M. L. (1992). "Influenza virus hemagglutinin HA-2 N-terminal fusogenic peptides augment gene transfer by transferrin-polylysine-DNA complexes: toward a synthetic virus-like gene-transfer vehicle." Proc Natl Acad Sci USA 89(17): 7934-8.

64. Weissleder, R., Stark, D. D., Engelstad, B. L., Bacon, B. R., Compton, C. C., White, D. L., Jacobs, P. and Lewis, J. (1989). "Superparamagnetic iron oxide: pharmacokinetics and toxicity." AJR Am J Roentgenol 152(1): 167-73.

65. Wotff, J. A., Malone, R. W., Williams, P., Chong, W., Acsadi, G., Jani, A. and Felgner, P. L. (1990). "Direct Gene Transfer Into Mouse Muscle Invivo." Science 247(4949): 1465-1468.

66. Wu, G. Y. and Wu, C. H. (1987). "Receptor-mediated in vitro gene transformation by a soluble DNA carrier system." Journal of Biological Chemistry 262: 4429-4432.

67. Zabner, J. (1997). "Cationic lipids used in gene transfer." Advanced Drug Delivery Reviews 27(1): 17-28.

68. Zborowski, M., Fuh, C. B., Green, R., Sun, L. and Chalmers, J. J. (1995). "Analytical magnetapheresis of ferritin-labeled lymphocytes." Anal Chem 67(20): 3702-12.

69. Zenke, M., Steinlein, P., Wagner, E., Cotten, M., Beug, H. and Birnstiel, M. L. (1990). "Receptor-Mediated Endocytosis Of Transferrin Polycation Conjugates - An Efficient Way To Introduce DNA Into Hematopoietic Cells." Proceedings Of The National Academy Of Sciences Of The United States Of America 87(10): 3655-3659.

70. Zhang, F., Andreassen, P., Fender, P., Geissler, E., Hernandez, J. F. and Chroboczek, J. (1999). "A transfecting peptide derived from adenovirus fiber protein." Gene Ther 6(2): 171-81.

Claims

1. An in vitro method for transfecting a cell comprising the step of bringing a complex comprising

   (i) one or more vectors comprising one or more nucleic acid molecules; and
   (ii) one or more magnetic particles which are coupled with one or more oligo-or polycations or oligo- or polyanions

   in contact with a cell by applying a suitable magnetic field, wherein said magnetic particle(s) and said vector(s) are assembled in the complex by salt-induced aggregation.

2. The method of claim 1, wherein said magnetic particles have a size of up to 2000 nm.

3. The method of claim 1 or 2, wherein said magnetic particles are coupled with a compound selected from the group consisting of poly(ethylene imine) (PEI), PEI-streptavidin, PEI-biotin, starch-phosphate, polyaspartic acid, polyacrylic acid, polyacrylic-co-maleic acid, arabinic acid, PEI-ethoxylated, PEI-epichlorhydrin and PEI-sodium dodecyl sulfate.

4. The method of any one of claims 1 to 3, wherein said magnetic field is a permanent field or an electromagnetic field.

5. The method of claim 4, wherein said electromagnetic field oscillates.

6. The method of any one of claims 1 to 5, wherein the cell is transfected in a high-throughput assay.

7. A method for the preparation of a complex useful for transfecting a cell, said method comprising the step of linking together one or more vectors comprising one or more nucleic acid molecules with one or more magnetic particles which are coupled with one or more oligo- or polycations or oligo- or polyanions, wherein the components of said complex are assembled by salt-induced aggregation.

8. A complex useful for transfecting a cell comprising

(i) one or more vectors comprising one or more nucleic acid molecules; and
(ii) one or more magnetic particles which are coupled with one or more oligo-or polycations or oligo- or polyanions,

wherein said magnetic particle(s) and said vector(s) are assembled in the complex by salt-induced aggregation.

9. The complex of claim 9, wherein said magnetic particles have a size of up to 2000 nm.

10. The complex of claim 8 or 9, wherein said magnetic particles are coupled with a compound selected from the group consisting of poly(ethylene imine) (PEI), PEI-streptavidin, PEI-biotin, starch-phosphate, polyaspartic acid, polyacrylic acid, polyacrylic-co-maleic acid, arabinic acid, PEI-ethoxylated, PEI-epichlorhydrin and PEI-sodium dodecyl sulfate.

11. A pharmaceutical composition comprising the complex of any one of claims 8 to 10 and optionally a pharmaceutically acceptable carrier and/or diluent.

12. Use of the complex of any one of claims claim 8 to 10 for the preparation of a pharmaceutical composition for preventing, treating or vaccinating against a disease by way of delivery of a therapeutically useful vector comprising one or more nucleic acid molecules into a subject, said preventing, treating or vaccinating involving the transfection of cells with the vector(s) present in said complex by way of applying a suitable magnetic field.

13. The complex of any one of claims 8 to 10 for use in a method of preventing, treating or vaccinating against a disease by way of delivery of a therapeutically useful vector comprising one or more nucleic acid molecules into a subject, said preventing, treating or vaccinating involving the transfection of cells with the vector(s) present in said complex by way of applying a suitable magnetic field.

14. Use of the complex of any one of claims 8 to 10 for transfecting a cell in vitro wherein the complex is brought in contact with a cell by applying a suitable magnetic field.

15. Use of the complex of any one of claims 8 to 10 for transfecting cells in vitro in a high-throughput assay wherein the complex is brought in contact with a cell by applying a suitable magnetic field.

16. A kit comprising

(i) the complex of any one of claims 8 to 10 and
(ii) optionally magnetic particles and/or vectors comprising one or more nucleic acid molecules and/or vector components suitable for taking up a nucleic acid molecule to be expressed and instructions for applying a method of any one of claims 1 to 7, and
(iii) optionally cells and/or gene therapeutical adjuvant(s) and/or one or more permanent magnets useful for applying the method of any one of claims 1 to 7.

**Patentansprüche**

1. In vitro-Verfahren zum Transfizieren einer Zelle, umfassend den Schritt des Inkontaktbringens eines Komplexes umfassend

   (i) einen oder mehrere Vektor(en), umfassend ein oder mehrere Nucleinsäuremolekül(e); und
   (ii) ein oder mehrere magnetische(s) Partikel, welche(s) an ein oder mehrere Oligo- oder Polykation(en) oder Oligo- oder Polyanion(en) gekoppelt ist/sind

   mit einer Zelle durch Anlegen eines geeigneten magnetischen Feldes, wobei die/das magnetische(n) Partikel und der/die Vektor(en) durch salzinduzierte Aggregation in dem Komplex zusammengefügt sind.

2. Verfahren nach Anspruch 1, wobei die magnetischen Partikel eine Größe von bis zu 2000nm haben.

3. Verfahren nach Anspruch 1 oder 2, wobei die magnetischen Partikel an eine Verbindung gekoppelt sind, die ausgewählt ist aus der Gruppe bestehend aus Poly(ethylenimin) (PEI), PEI-Streptavidin, PEI-Biotin, Stärke-Phosphat, Polyasparaginsäure, Polyacrylsäure, Polyacryl-co-maleinsäure, Arabinsäure, PEI-ethoxyliert, PEI-Epichlorhydrin und PEI-Natriumdodecylsulfat.

4. Verfahren nach einem der Ansprüche 1 bis 3, wobei das magnetische Feld ein permanentes Feld oder ein elektromagnetisches Feld ist.

5. Verfahren nach Anspruch 4, wobei das elektromagnetische Feld oszilliert.

6. Verfahren nach einem der Ansprüche 1 bis 5, wobei die Zelle in einem Hochdurchsatz-Assay transfiziert wird.

7. Verfahren zur Herstellung eines Komplexes, welcher verwendbar ist, um eine Zelle zu transfizieren, wobei das Verfahren den Schritt des Verknüpfens von einem oder mehreren Vektor(en), umfassend ein oder mehrere Nucleinsäueremolokül(e), mit einem oder mehreren magnetischen Partikel(n) umfasst, welche(s) an ein oder mehrere Oligo- oder Polykation(en) oder Oligo- oder Polyanion(en) gekoppelt ist/sind, wobei die Komponenten des Komplexes durch salzinduzierte Aggregation zusammengefügt werden.

8. Komplex, verwendbar zum Transfizieren einer Zelle, umfassend

   (i) einen oder mehrere Vektor(en), umfassend ein oder mehrere Nucleinsäuremolekül(e); und
   (ii) ein oder mehrere magnetische(s) Partikel, welche(s) an ein oder mehrere Oligo- oder Polykation(en) oder Oligo- oder Polyanion(en) gekoppelt ist/sind,

   wobei das/die magnetische(n) Partikel und der/die Vektor(en) durch salzinduzierte Aggregation zusammengefügt sind.

9. Komplex nach Anspruch 9, wobei die magnetischen Partikel eine Größe von bis zu 2000nm haben.

10. Komplex nach Anspruch 8 oder 9, wobei die magnetischen Partikel an eine Verbindung gekoppelt sind, die ausgewählt ist aus der Gruppe bestehend aus Poly(ethylenimin) (PEI), PEI-Streptavidin, PEI-Biotin, Stärke-Phosphat, Polyasparaginsäure, Polyacrylsäure, Polyacryl-co-maleinsäure, Arabinsäure, PEI-ethoxyliert, PEI-Epichlorhydrin und PEI-Natriumdodecylsulfat.

11. Arzneimittel, umfassend den Komplex nach einem der Ansprüche 8 bis 10 und gegebenenfalls ein(en) pharmazeutisch verträglichen/verträgliches Träger und/oder Verdünnungsmittel.

12. Verwendung des Komplexes nach einem der Ansprüche 8 bis 10 zur Herstellung eines Arzneimittels für die Prävention, Behandlung oder Impfung gegen eine Krankheit durch die Verabreichung eines therapeutisch verwendbaren Vektors umfassend ein oder mehrere Nucleinsäuremolekül(e) an einen Patienten, wobei die Prävention, Behandlung oder Impfung die Transfektion von Zellen mit dem/den Vektor(en), welcher/welche in dem Komplex vorhanden ist/sind, durch die Anlegung eines geeigneten magnetischen Feldes beinhaltet.

**13.** Komplex nach einem der Ansprüche 8 bis 10 zur Verwendung in einem Verfahren für die Prävention, Behandlung oder Impfung gegen eine Krankheit, durch die Verabreichung eines therapeutisch verwendbaren Vektors umfassend ein oder mehrere Nucleinsäuremolekül(e) an einen Patienten, wobei die Prävention, Behandlung oder Impfung die Transfektion von Zellen mit dem/den Vektor(en) in dem Komplex durch die Anlegung eines geeigneten magnetischen Feldes beinhaltet.

**14.** Verwendung des Komplexes nach einem der Ansprüche 8 bis 10 zum Transfizieren einer Zelle in vitro, wobei der Komplex durch Anlegen eines geeigneten magnetischen Feldes in Kontakt mit einer Zelle gebracht wird.

**15.** Verwendung eines Komplexes nach einem der Ansprüche 8 bis 10 zum Transfizieren von Zellen in vitro in einem Hochdurchsatz-Assay, wobei der Komplex durch Anlegen eines geeigneten magnetischen Feldes in Kontakt mit einer Zelle gebracht wird.

**16.** Kit umfassend

(i) den Komplex gemäß einem der Ansprüche 8 bis 10 und
(ii) gegebenenfalls magnetische Partikel und/oder Vektoren umfassend ein oder mehrere Nucleinsäuremolekül (e) und/oder Vektorkomponenten, die geeignet zur Aufnahme eines Nucleinsäuremoleküls sind, welches zu exprimieren ist, und Anweisungen zur Anwendung eines Verfahrens gemäß einem der Ansprüche 1 bis 7, und
(iii) gegebenenfalls Zellen und/oder (ein) gentherapeutische(s) Adjuvans/Adjuvanzien und/oder ein oder mehrere permanente(r) Magnet(en), die für die Anwendung des Verfahrens gemäß einem der Ansprüche 1 bis 7 verwendbar sind.

## Revendications

**1.** Procédé in vitro destiné à transfecter une cellule, comprenant l'étape qui consiste à mettre un complexe comprenant

(i) un ou plusieurs vecteurs contenant une ou plusieurs molécules d'acide nucléique, et
(ii) une ou plusieurs particules magnétiques couplées à un ou plusieurs oligo- ou polycations ou oligo- ou polyanions,

en contact avec une cellule en appliquant un champ magnétique approprié, où ladite/lesdites particule(s) magnétique (s) et ledit/lesdits vecteur(s) sont assemblés dans le complexe par une agrégation induite par un sel.

**2.** Procédé selon la revendication 1, dans lequel lesdites particules magnétiques ont une taille allant jusqu'à 2 000 nm.

**3.** Procédé selon la revendication 1 ou 2, dans lequel lesdites particules magnétiques sont couplées à un composé sélectionné dans le groupe constitué de :polyéthylèneimine (PEI), PEI-streptavidine, PEI-biotine, phosphate d'amidon, acide polyaspartique, acide polyacrylique, copolymère d'acide acrylique et d'acide maléique, acide arabinique, PEI éthoxylée, PEI-épichlorhydrine et PEI-dodécylsulfate de sodium.

**4.** Procédé selon l'une quelconque des revendications 1 à 3, dans lequel ledit champ magnétique est un champ permanent ou un champ électromagnétique.

**5.** Procédé selon la revendication 4, dans lequel ledit champ électromagnétique est oscillant.

**6.** Procédé selon l'une quelconque des revendications 1 à 5, dans lequel la cellule est transfectée dans un essai à haut débit.

**7.** Procédé de préparation d'un complexe utile pour transfecter une cellule, ledit procédé comprenant l'étape qui consiste à relier un ou plusieurs vecteurs contenant une ou plusieurs molécules d'acide nucléique à une ou plusieurs particules magnétiques qui sont couplées à un ou plusieurs oligo- ou polycations ou oligo- ou polyanions, dans lequel les composants dudit complexe sont assemblés par une agrégation induite par un sel.

**8.** Complexe utile pour transfecter une cellule, comprenant

(i) un ou plusieurs vecteurs contenant une ou plusieurs molécules d'acide nucléique ; et
(ii) une ou plusieurs particules magnétiques couplées à un ou plusieurs oligo- ou polycations ou oligo- ou polyanions,

dans lequel ladite/lesdites particule(s) magnétique(s) et ledit/lesdits vecteur(s) sont assemblés dans le complexe par une agrégation induite par un sel.

**9.** Complexe selon la revendication 9, dans lequel lesdites particules magnétiques ont une taille allant jusqu'à 2 000 nm.

**10.** Complexe selon la revendication 8 ou 9, dans lequel lesdites particules magnétiques sont couplées à un composé sélectionné dans le groupe constitué de :polyéthylèneimine (PEI), PEI-streptavidine, PEI-biotine, phosphate d'amidon, acide polyaspartique, acide polyacrylique, copolymère d'acide acrylique et d'acide maléique, acide arabinique, PEI éthoxylée, PEI-épichlorhydrine et PEI-dodécylsulfate de sodium.

**11.** Composition pharmaceutique comprenant le complexe selon l'une quelconque des revendications 8 à 10, et facultativement un véhicule et/ou un diluant pharmaceutiquement acceptable.

**12.** Utilisation du complexe selon l'une quelconque des revendications 8 à 10, pour la préparation d'une composition pharmaceutique destinée à la prévention, au traitement ou à la vaccination contre une maladie, au moyen de la délivrance d'un vecteur thérapeutiquement utile comprenant une ou plusieurs molécules d'acide nucléique chez un sujet, ledit/ladite prévention, traitement ou vaccination impliquant la transfection de cellules avec le(s) vecteur(s) présent(s) dans ledit complexe, au moyen de l'application d'un champ magnétique approprié.

**13.** Complexe selon l'une quelconque des revendications 8 à 10, destiné à être utilisé dans un procédé de prévention, de traitement ou de vaccination contre une maladie, au moyen de la délivrance d'un vecteur thérapeutiquement utile comprenant une ou plusieurs molécules d'acide nucléique chez un sujet, ledit/ladite prévention, traitement ou vaccination impliquant la transfection de cellules avec le(s) vecteur(s) présent(s) dans ledit complexe, au moyen de l'application d'un champ magnétique approprié.

**14.** Utilisation du complexe selon l'une quelconque des revendications 8 à 10, pour transfecter une cellule in vitro, où le complexe est mis en contact avec une cellule en appliquant un champ magnétique approprié.

**15.** Utilisation du complexe selon l'une quelconque des revendications 8 à 10, pour transfecter des cellules *in vitro* dans un essai à haut débit, où le complexe est mis en contact avec une cellule en appliquant un champ magnétique approprié.

**16.** Kit comprenant

(i) le complexe selon l'une quelconque des revendications 8 à 10, et
(ii) facultativement des particules magnétiques et/ou des vecteurs comprenant une ou plusieurs molécules d'acide nucléique et/ou des composants de vecteur appropriés pour incorporer une molécule d'acide nucléique devant être exprimée, et des instructions pour appliquer un procédé selon l'une quelconque des revendications 1 à 7, et
(iii) facultativement des cellules et/ou un/des adjuvant(s) thérapeutique(s) génétiques et/ou un ou plusieurs aimants permanents utiles pour appliquer le procédé selon l'une quelconque des revendications 1 à 7.

Fig. 1

Fig. 2

**Fig. 3**

Fig. 4

Fig. 5

Fig. 6

**Fig. 7**

**A**

pg luciferase / mg protein

µg fIMAG / µg DNA

□ fIMAG-ARA, 10 min with magnet

□ fIMAG-ARA, 10 min without magnet

**B**

pg luciferase / mg protein

µg fIMAG / µg DNA

▣ fIMAG-pACRYL, 10 min with magnet

□ fIMAG-pACRYL, 10 min without magnet

**C**

pg luciferase / mg protein

µg fIMAG / µg DNA

□ fIMAG-pACRYL-MAL, 10 min with magnet

□ fIMAG-pACRYL-MAL, 10 min without magnet

**D**

pg luciferase / mg protein

µg fIMAG / µg DNA

□ fIMAG-pASP, 10 min with magnet

□ fIMAG-pASP, 10 min without magnet

Fig. 8

E

pg luciferase / mg protein

□ flMAG-ARA, 10 min with magnet

□ flMAG-ARA, 10 min without magnet

F

pg luciferase / mg protein

□ flMAG-pACRYL, 10 min with magnet

□ flMAG-pACRYL, 10 min without magnet

G

pg luciferase / mg protein

□ flMAG-pACRYL-MAL, 10 min with magnet

□ flMAG-pACRYL-MAL, 10 min without magnet

H

pg luciferase / mg protein

□ flMAG-pASP, 10 min with magnet

□ flMAG-pASP, 10 min without magnet

Fig. 8 continued

EP 1 297 169 B1

Fig. 9

**A**

**B**

**C**

**Fig. 10**

**Fig. 11**

**Fig. 12**

**Fig. 13**

**Fig. 14**

Fig. 15

A

B

A-1

B-1

Fig. 16

**Fig. 17A**

**Fig. 17B**

| B | proximal of magnet | magnet position | distal of magnet | remote distal |
|---|---|---|---|---|
| ■ with magnet | 47,36 | 448,81 | 13,44 | |
| □ without magnet | 1,54 | 18,61 | 0,00 | 9,67 |

**Fig. 17C**

| C | proximal of magnet | magnet position | distal of magnet | remote distal |
|---|---|---|---|---|
| ■ with magnet | 8,38 | 72,99 | 60,59 | 111,47 |
| □ without magnet | 0,05 | 0,11 | 0,10 | 0,03 |

**Fig. 18**

EP 1 297 169 B1

Fig. 19

EP 1 297 169 B1

Fig. 20

Fig. 20 continued

Fig. 21A

Fig. 21B

**A**

NIH3T3

PEI (N/P)

**B**

HepG2

PEI (N/P)

**Fig. 22**

**Fig. 23-A1**          **GenePorter 10 min.**

| transMAG:DNA → | 0 | 0.5 | 1 | 2 | 4 | 6 | 8 | 10 |
|---|---|---|---|---|---|---|---|---|
| DNA dose (μg/well)↓ | | | | | | | | |
| 0.1 | 1 | 21 | 15 | 13 | 38 | 68 | 36 | 11 |
| 0.05 | 0 | 30 | 26 | 48 | 38 | 73 | 170 | 33 |
| 0.025 | 1 | 132 | 76 | 27 | 49 | 74 | 55 | 46 |
| 0.0125 | 0 | 123 | 40 | 99 | 73 | 50 | 48 | 29 |

**Fig. 23-A2**          **GenePorter 4 hrs.**

| transMAG:DNA → | 0 | 0.5 | 1 | 2 | 4 | 6 | 8 | 10 |
|---|---|---|---|---|---|---|---|---|
| DNA dose (μg/well)↓ | | | | | | | | |
| 0.1 | 1 | 1 | 1 | 0 | 1 | 1 | 1 | 1 |
| 0.05 | 1 | 1 | 1 | 0 | 1 | 1 | 1 | 1 |
| 0.025 | 1 | 2 | 2 | 1 | 2 | 1 | 1 | 1 |
| 0.0125 | 1 | 3 | 3 | 2 | 4 | 3 | 3 | 1 |

**Fig. 23A Table**

Fig. 23B

Fig. 23C

**24A-1:**          **Lipofectamine 10 min.**

| transMAG:DNA → <br> DNA dose (µg/well)↓ | 0 | 0.5 | 1 | 2 | 4 | 6 | 8 | 10 |
|---|---|---|---|---|---|---|---|---|
| 0.1 | 1 | 19 | 37 | 147 | 9 | 5 | 5 | 4 |
| 0.05 | 1 | 0 | 15 | 506 | 43 | 19 | 20 | 6 |
| 0.025 | 1 | 0 | 79 | 22 | 30 | 12 | 18 | 6 |
| 0.0125 | 1 | 0 | 23 | 42 | 9 | 5 | 17 | 6 |

**24A-2:**          **Lipofectamine 4 hrs.**

| transMAG:DNA → <br> DNA dose (µg/well)↓ | 0 | 0.5 | 1 | 2 | 4 | 6 | 8 | 10 |
|---|---|---|---|---|---|---|---|---|
| 0.1 | 0 | 8 | 2 | 2 | 0 | 0 | 0 | 0 |
| 0.05 | 0 | 2 | 1 | 2 | 1 | 0 | 0 | 0 |
| 0.025 | 1 | 3 | 1 | 3 | 2 | 1 | 1 | 0 |
| 0.0125 | 1 | 2 | 1 | 1 | 1 | 1 | 1 | 1 |

**Fig. 24A Table**

Fig. 24B

## 24C-1: 10 min, with magnet

## 24C-2: 10 min, without magnet

## 24C-3: 4 hr, with magnet

## 24C-4: 4 hr, without magnet

**DNA dose/well**

—□— 0.1 µg

—◇— 0.05 µg

—○— 0.025 µg

—△— 0.0125 µg

**Fig. 24C**

DOCHOL 10 min.

| transMAG:DNA → | 0 | 0.2 | 0.4 | 0.6 | 0.8 | 1 | 2 | 4 |
|---|---|---|---|---|---|---|---|---|
| DNA dose (µg/well) ↓ | | | | | | | | |
| 0.5 | 0 | 3 | 2 | 6 | 3 | 3 | 12 | 11 |
| 0.25 | 0 | 10 | 4 | 14 | 8 | 8 | 33 | 19 |
| 0.125 | 0 | 1 | 13 | 29 | 48 | 4 | 14 | 54 |
| 0.0625 | 1 | 5 | 5 | 18 | 4 | 8 | 16 | 100 |
| 0.03125 | 0 | 9 | 40 | 3 | 2 | 6 | 76 | 93 |
| 0.015625 | 1 | 4 | 56 | 414 | 10 | 2 | 112 | 4300 |

**Fig. 25A Table**

**25B-1:**
**with magnet**

**25B-2:**
**without magnet**

μg transMAG-PEI/
μg DNA

| | | | |
|---|---|---|---|
| □ | 0 | ⊞ | 0.8 |
| ◇ | 0.2 | ◆ | 1 |
| ○ | 0.4 | ⊕ | 2 |
| △ | 0.6 | ▽ | 4 |

y-axis: pg luciferase / mg protein

x-axis: μg DNA/well

Fig. 25B

EP 1 297 169 B1

Fig. 25C

**26A-2: GenePORTER**

**26A-1: LIPOFECTAMINE**

transfection time (min)

ng luciferase / mg protein

- ● transMAG complex with magnetic field
- ○ transMAG complex without magnetic field
- ■ standard complex with magnetic field
- □ standard complex without magnetic field

**Fig. 26**

| | | | | | | |
|---|---|---|---|---|---|---|
| Polybrene | + | - | + | - | + | - |
| transMAG-PEI | - | - | + | + | + | + |
| Magn. field | - | - | - | - | + | + |

Fig. 27

Fig. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 911207 A
- US 5525711 A
- US 4711955 A
- US 5792608 A
- EP 302175 A
- WO 9429469 A
- WO 9700957 A
- DE 19624426
- US 4554088 A
- US 4554089 A
- US 4208294 A
- US 4101435 A
- US 5981273 A

**Non-patent literature cited in the description**

- **Hughes C et al.** *Mol. Therap.,* 2001, vol. 3 (4), 623-630
- **Sambrook et al.** Molecular Cloning A Laboratory Manual. Cold Spring Harbor Laboratory, 1989
- **Ausubel et al.** Current Protocols in Molecular Biology. Green Publishing Associates and Wiley Interscience, 1989
- **Giordano.** *Nature Medicine,* 1996, vol. 2, 534-539
- **Schaper.** *Circ. Res.,* 1996, vol. 79, 911-919
- **Anderson.** *Science,* 1992, vol. 256, 808-813
- **Isner.** *Lancet,* 1996, vol. 348, 370-374
- **Muhlhauser.** *Circ. Res.,* 1995, vol. 77, 1077-1086
- **Wang.** *Nature Medicine,* 1996, vol. 2, 714-716
- **Schaper.** *Current Opinion in Biotechnology,* 1996, vol. 7, 635-640
- *5th New Jersey Symposium on Biomaterials Science,* 09 November 2000
- **U. Schwertmann ; R.M. Cornell.** Iron Oxides in the Laboratory. VCH, 1991
- **L.D. Landau et al.** The Field Theory. Nauka, 1973, vol. 2, 128-136
- **Mah et al.** *Mol. Therapy,* 2000, vol. 1 (5), 239
- **Schper.** *Circ. Res.,* 1996, vol. 79, 911-919
- **Scharper.** *Current Opinion in Biotechnology,* 1996, vol. 7, 635-640
- **Berks.** *TIBTECH,* 1994, vol. 12, 352-364
- **Babincova, M. ; Altanerova, V. ; Lampert, M. ; Altaner, C. ; Machova, E. ; Sramka, M. ; Babinec, P.** Site-specific in vivo targeting of magnetoliposomes using externally applied magnetic field. *Z Naturforsch [C],* 2000, vol. 55 (3-4), 278-81
- **Bailey, A. L. ; Sullivan, S. M.** Efficient encapsulation of DNA plasmids in small neutral liposomes induced by ethanol and calcium. *Biochim Biophys Acta,* 2000, vol. 1468 (1-2), 239-52
- **Bergemann, C. ; Muller-Schulte, D. ; Oster, J. ; à Brassard, L. ; Lübbe, A. S.** Magnetic ion-exchange nano- and microparticles for medical, biochemical and molecular biological applications. *J. Magnetism and Magnetic Materials,* 1999, vol. 194, 45-52
- **Bildirici, L. ; Smith, P. ; Tzavelas, C. ; Horefti, E. ; Rickwood, D.** Transfection of cells by immunoporation. *Nature,* 2000, vol. 405, 298
- **Bonadio, J. ; Goldstein, S. A. ; Levy, R. J.** Gene therapy for tissue repair and regeneration. *Advanced Drug Delivery Reviews,* 1998, vol. 33 (1-2), 53-69
- **Boussif, O. ; Lezoualch, F. ; Zanta, M. A. ; Mergny, M. D. ; Scherman, D. ; Demeneix, B. ; Behr, J. P.** A Versatile Vector For Gene And Oligonucleotide Transfer Into Cells In Culture And In Vivo - Polyethylenimine. *Proceedings Of The National Academy Of Sciences Of The United States Of America,* 1995, vol. 92 (16), 7297-7301
- **Carson, J. J. ; Prato, F. S. ; Drost, D. J. ; Diesbourg, L. D. ; Dixon, S. J.** Time-varying magnetic fields increase cytosolic free Ca2+ in HL-60 cells. *Am J Physiol,* 1990, vol. 259, C687-92
- **Chen, C. A. ; Okayama, H.** Calcium phosphate-mediated gene transfer: a highly efficient transfection system for stably transforming cells with plasmid DNA. *Biotechniques,* 1988, vol. 6 (7), 632-8
- **Cohen, H. ; Levy, R. J. ; Gao, J. ; Fishbein, I. ; Kousaev, V. ; Sosnowski, S. ; Slomkowski, S. ; Golomb, G.** Sustained delivery and expression of DNA encapsulated in polymeric nanoparticles. *Gene Ther,* 2000, vol. 7 (22), 1896-905

- **Colestrauss, A. ; Yoon, K. G. ; Xiang, Y. F. ; Byrne, B. C. ; Rice, M. C. ; Gryn, J. ; Holloman, W. K. ; Kmiec, E. B.** Correction of the Mutation Responsible For Sickle Cell Anemia By an Rna-Dna Oligonucleotide. *Science,* 1996, vol. 273 (5280), 1386-1389
- **Curiel, D. T.** Strategies to adapt adenoviral vectors for targeted delivery. *Ann N Y Acad Sci,* 1999, vol. 886, 158-71
- **Curiel, D. T. ; Agarwal, S. ; Wagner, E. ; Cotten, M.** Adenovirus Enhancement Of Transferrin Polylysine-Mediated Gene Delivery. *Proceedings Of The National Academy Of Sciences Of The United States Of America,* 1991, vol. 88 (19), 8850-8854
- **Fahlvik, A. K. ; Klaveness, J. ; Stark, D. D.** Iron oxides as MR imaging contrast agents. *J Magn Reson Imaging,* 1993, vol. 3 (1), 187-94
- **Fasbender, A. ; Lee, J. H. ; Walters, R. W. ; Moninger, T. O. ; Zabner, J. ; Welsh, M. J.** Incorporation of adenovirus in calcium phosphate precipitates enhances gene transfer to airway epithelia in vitro and in vivo. *J Clin Invest,* 1998, vol. 102 (1), 184-93
- **Fasbender, A. ; Zabner, J. ; Chillon, M. ; Moninger, T. O. ; Puga, A. P. ; Davidson, B. L. ; Welsh, M. J.** Complexes of adenovirus with polycationic polymers and cationic lipids increase the efficiency of gene transfer in vitro and in vivo. *J Biol Chem,* 1997, vol. 272 (10), 6479-89
- **Felgner, P. L. ; Barenholz, Y. ; Behr, J. P. ; Cheng, S. H. ; Cullis, P. ; Huang, L. ; Jessee, J. A. ; Seymour, L. ; Szoka, F. ; Thierry, A. R.** Nomenclature for synthetic gene delivery systems. *Hum Gene Ther,* 1997, vol. 8 (5), 511-2
- **Felgner, P. L. ; Ringold, G. M.** Cationic liposome-mediated transfection. *Nature,* 1989, vol. 337, 387-388
- **Fender, P. ; Ruigrok, R. W. ; Gout, E. ; Buffet, S. ; Chroboczek, J.** Adenovirus dodecahedron, a new vector for human gene transfer. *Nat Biotechnol,* 1997, vol. 15 (1), 52-6
- **Finsinger, D. ; Remy, J. S. ; Erbacher, P. ; Koch, C. ; Plank, C.** Protective copolymers for nonviral gene vectors: synthesis, vector characterization and application in gene delivery. *Gene Ther,* 2000, vol. 7 (14), 1183-92
- **Freund, O. ; Mahy, P. ; Amedee, J. ; Roux, D. ; Laversanne, R.** Encapsulation of DNA in new multilamellar vesicles prepared by shearing a lyotropic lamellar phase. *J Microencapsul,* 2000, vol. 17 (2), 157-68
- **Gao, X. ; Huang, L.** Potentiation Of Cationic Liposome-Mediated Gene Delivery By Polycations. *Biochemistry,* 1996, vol. 35 (3), 1027-1036
- **Gill, I. ; Ballesteros, A.** Bioencapsulation within synthetic polymers (Part 1): sol-gel encapsulated biologicals. *Trends Biotechnol,* 2000, vol. 18 (7), 282-96
- **Gossfing, H. R. ; Bernstein, R. A. ; Abbott, J.** Treatment of ununited tibial fractures: a comparison of surgery and pulsed electromagnetic fields (PEMF). *Orthopedics,* 1992, vol. 15 (6), 711-9
- **GrillotCourvalin, C. ; Goussard, S. ; Huetz, F. ; Ojcius, D. M. ; Courvalin, P.** Functional gene transfer from intracellular bacteria to mammalian cells. *Nature Biotechnology,* 1998, vol. 16 (9), 862-866
- **Haensler, J. ; Szoka, F. C.** Polyamidoamine Cascade Polymers Mediate Efficient Transfection Of Cells In Culture. *Bioconjugate Chemistry,* 1993, vol. 4 (5), 372-379
- **Hiemenz, P. C.** Principles of Colloid and Surface Chemistry. Marcel Dekker, Inc, 1986
- **Hirosue, S. ; Muller, B. G. ; Mulligan, R. C. ; Langer, R.** Plasmid DNA encapsulation and release from solvent diffusion nanospheres. *J Control Release,* 2001, vol. 70 (1-2), 231-42
- **Isner, J. M. ; Walsh, K. ; Symes, J. ; Pieczek, A. ; Takeshita, S. ; Lowry, J. ; Rosenfield, K. ; Weir, L. ; Brogi, E. ; Jurayj, D.** Arterial gene transfer for therapeutic angiogenesis in patients with peripheral artery disease. *Hum. Gene Ther.,* 1996, vol. 20 (7), 959-988
- **Kalyanasundaram, S. ; Feinstein, S. ; Nicholson, J. P. ; Leong, K. W. ; Garver, R. I., Jr.** Coacervate microspheres as carriers of recombinant adenoviruses. *Cancer Gene Ther,* 1999, vol. 6 (2), 107-12
- **Kaneda, Y.** Fusigenic Sendai-virus liposomes: a novel hybrid type liposome for gene therapy. *Biogenic Amines,* 1998, vol. 14 (5), 553-572
- **Kasahara, N. ; Dozy, A. M. ; Kan, Y. W.** Tissue-specific targeting of retroviral vectors through ligand-receptor interactions. *Science,* 1994, vol. 266 (5189), 1373-6
- **Kass-Eisler, A. ; Leinwand, L. ; Gall, J. ; Bloom, B. ; Falck-Pedersen, E.** Circumventing the immune response to adenovirus-mediated gene delivery. *Gene Therapy,* 1996, vol. 3, 154-162
- **Kruger, A. ; Schirrmacher, V. ; von Hoegen, P.** Scattered micrometastases visualized at the single-cell level: detection and re-isolation of lacZ-labeled metastasized lymphoma cells. *Int J Cancer,* 1994, vol. 58 (2), 275-84
- **Lee, R. J. ; Huang, L.** Lipidic vector systems for gene transfer. *Crit. Rev. Ther. Drug Carrier Syst.,* 1997, vol. 14 (2), 173-206
- **Leong, K. W. ; Mao, H. Q. ; Truong-Le, V. L. ; Roy, K. ; Walsh, S. M. ; August, J. T.** DNA-polycation nanospheres as non-viral gene delivery vehicles. *J Control Release,* 1998, vol. 53 (1-3), 183-93
- **Li, S. ; Rizzo, M. A. ; Bhattacharya, S. ; Huang, L.** Characterization of cationic lipid-protamine-DNA (LPD) complexes for intravenous gene delivery. *Gene Therapy,* 1998, vol. 5 (7), 930-937

- **Lübbe, A. S. ; Bergemann, C. ; Huhnt, W. ; Fricke, T. ; Riess, H. ; Brock, J. W. ; Huhn, D.** Preclinical experiences with magnetic drug targeting: tolerance and efficacy. *Cancer Res,* 1996, vol. 56 (20), 4694-701
- **Lübbe, A. S. ; Bergemann, C. ; Riess, H. ; Schriever, F. ; Reichardt, P. ; Possinger, K. ; Matthias, M. ; Dorken, B. ; Herrmann, F. ; Gurtler, R.** Clinical experiences with magnetic drug targeting: a phase I study with 4'-epidoxorubicin in 14 patients with advanced solid tumors. *Cancer Res,* 1996, vol. 56 (20), 4686-93
- **Luo, D. ; Saltzman, W. M.** Enhancement of transfection by physical concentration of DNA at the cell surface. *Nat Biotechnol,* 2000, vol. 18 (8), 893-5
- **Meyer, K. B. ; Thompson, M. M. ; Levy, M. Y. ; Barron, L. G. ; Szoka, F. C.** Intratracheal Gene Delivery To The Mouse Airway - Characterization Of Plasmid DNA Expression And Pharmacokinetics. *Gene Therapy,* 1995, vol. 2 (7), 450-460
- **Michael, S. I. ; Hong, J. S. ; Curiel, D. T. ; Engler, J. A.** Addition of a short peptide ligand to the adenovirus fiber protein. *Gene Ther,* 1995, vol. 2 (9), 660-8
- **Nicolau, C. ; Cudd, A.** Liposomes As Carriers Of DNA. *Critical Reviews In Therapeutic Drug Carrier Systems,* 1989, vol. 6 (3), 239-271
- **O'Riordan, C. R. ; Lachapelle, A. ; Delgado, C. ; Parkes, V. ; Wadsworth, S. C. ; Smith, A. E. ; Francis, G. E.** PEGylation of adenovirus with retention of infectivity and protection from neutralizing antibody in vitro and in vivo. *Hum Gene Ther,* 1999, vol. 10 (8), 1349-58
- **Ogris, M. ; Brunner, S. ; Schuller, S. ; Kircheis, R. ; Wagner, E.** PEGylated DNA/transferrin-PEI complexes: reduced interaction with blood components, extended circulation in blood and potential for systemic gene delivery. *Gene Therapy,* 1999, vol. 6 (4), 595-605
- **Padmanabhan, R. ; Howard, T. ; Gottesman, M. M. ; Howard, B. H.** Magnetic affinity cell sorting to isolate transiently transfected cells, multidrug-resistant cells, somatic cell hybrids, and virally infected cells. *Methods Enzymol,* 1993, vol. 218, 637-51
- **Petka, W. A. ; Harden, J. L. ; McGrath, K. P. ; Wirtz, D. ; Tirrell, D. A.** Reversible hydrogels from self-assembling artificial proteins. *Science,* 1998, vol. 281 (5375), 389-92
- **Plank, C. ; Mechtler, K. ; Szoka, F. C. ; Wagner, E.** Activation of the Complement System by Synthetic DNA Complexes: A Potential Barrier for Intravenous Gene Delivery. *Hum. Gene Ther.,* 1996, vol. 7 (12), 1437-1446
- **Plank, C. ; Oberhauser, B. ; Mechtler, K. ; Koch, C. ; Wagner, E.** The Influence Of Endosome-Disruptive Peptides On Gene Transfer Using Synthetic Virus-Like Gene Transfer Systems. *Journal Of Biological Chemistry,* 1994, vol. 269 (17), 12918-12924
- **Plank, C. ; Tang, M. ; Wolfe, A. ; Szoka, F. C.** Branched Cationic Peptides for Gene Delivery. Role of Type and Number of Cationic Residues in Formation and In Vitro Activity of DNA Polyplexes. *Human Gene Therapy,* 1999, vol. 10 (2), 319-333
- **Ponimaskin, E. ; Bareesel, K. K. ; Markgraf, K. ; Reszka, R. ; Lehmann, K. ; Gelderblom, H. R. ; Gawaz, M. ; Schmidt, M. F.** Sendai virosomes revisited: reconstitution with exogenous lipids leads to potent vehicles for gene transfer. *Virology,* 2000, vol. 269 (2), 391-403
- **Poul, M. A. ; Marks, J. D.** Targeted gene delivery to mammalian cells by filamentous bacteriophage. *J Mol Biol,* 1999, vol. 288 (2), 203-11
- **Quong, D. ; Neufeld, R. J.** DNA encapsulation within co-guanidine membrane coated alginate beads and protection from extracapsular nuclease. *J Microencapsul,* 1999, vol. 16 (5), 573-85
- **Rajasubramanian, G. ; Meidell, R. S. ; Landau, C. ; Dollar, M. L. ; Holt, D. B. ; Willard, J. E. ; Prager, M. D. ; Eberhart, R. C.** Fabrication of resorbable microporous intravascular stents for gene therapy applications. *Asaio J,* 1994, vol. 40 (3), M584-9
- Gene transfer-using the cationic lipid-magnetosome-DNA complexes (CLMDC). **Reszka, R. ; Wegner, D. ; Richter, J. ; Pohl, B. ; Lehmann, C. ; Scheffel, A. ; Gerbsch, N.** Scientific and clinical applications of magnetic carriers. 2000
- **Romanczuk, H. ; Galer, C. E. ; Zabner, J. ; Barsomian, G. ; Wadsworth, S. C. ; O'Riordan, C. R.** Modification of an adenoviral vector with biologically selected peptides: a novel strategy for gene delivery to cells of choice. *Hum Gene Ther,* 1999, vol. 10 (16), 2615-26
- **Rowley, J. A. ; Madlambayan, G. ; Mooney, D. J.** Alginate hydrogels as synthetic extracellular matrix materials. *Biomaterials,* 1999, vol. 20 (1), 45-53
- **Roy, K. ; Mao, H. Q. ; Huang, S. K. ; Leong, K. W.** Oral gene delivery with chitosan-DNA nanoparticles generates immunologic protection in a murine model of peanut allergy. *Nat Med,* 1999, vol. 5 (4), 387-91
- **Rubin, C. T. ; Donahue, H. J. ; Rubin, J. E. ; McLeod, K. J.** Optimization of electric field parameters for the control of bone remodeling: exploitation of an indigenous mechanism for the prevention of osteopenia. *J Bone Miner Res,* 1993, vol. 8 (2), 573-81

- **Shea, L. D. ; Smiley, E. ; Bonadio, J. ; Mooney, D. J.** DNA delivery from polymer matrices for tissue engineering. *Nat Biotechnol,* 1999, vol. 17 (6), 551-4
- **Trock, D. H.** Electromagnetic fields and magnets. Investigational treatment for musculoskeletal disorders. *Rheum Dis Clin North Am,* 2000, vol. 26 (1), 51-62
- **Truong-Le, V. L. ; Walsh, S. M. ; Schweibert, E. ; Mao, H. Q. ; Guggino, W. B. ; August, J. T. ; Leong, K. W.** Gene transfer by DNA-gelatin nanospheres. *Arch Biochem Biophys,* 1999, vol. 361 (1), 47-56
- **TruongLe, V. L. ; August, J. T. ; Leong, K. W.** Controlled gene delivery by DNA-gelatin nanospheres. *Human Gene Therapy,* 1998, vol. 9 (12), 1709-1717
- **Wagner, E. ; Plank, C. ; Zatloukal, K. ; Cotten, M. ; Birnstiel, M. L.** Influenza virus hemagglutinin HA-2 N-terminal fusogenic peptides augment gene transfer by transferrin-polylysine-DNA complexes: toward a synthetic virus-like gene-transfer vehicle. *Proc Natl Acad Sci USA,* 1992, vol. 89 (17), 7934-8
- **Weissleder, R. ; Stark, D. D. ; Engelstad, B. L. ; Bacon, B. R. ; Compton, C. C. ; White, D. L. ; Jacobs, P. ; Lewis, J.** Superparamagnetic iron oxide: pharmacokinetics and toxicity. *AJR Am J Roentgenol,* 1989, vol. 152 (1), 167-73
- **Wotff, J. A. ; Malone, R. W. ; Williams, P. ; Chong, W. ; Acsadi, G. ; Jani, A. ; Felgner, P. L.** Direct Gene Transfer Into Mouse Muscle Invivo. *Science,* 1990, vol. 247 (4949), 1465-1468
- **Wu, G. Y. ; Wu, C. H.** Receptor-mediated in vitro gene transformation by a soluble DNA carrier system. *Journal of Biological Chemistry,* 1987, vol. 262, 4429-4432
- **Zabner, J.** Cationic lipids used in gene transfer. *Advanced Drug Delivery Reviews,* 1997, vol. 27 (1), 17-28
- **Zborowski, M. ; Fuh, C. B. ; Green, R. ; Sun, L. ; Chalmers, J. J.** Analytical magnetapheresis of ferritin-labeled lymphocytes. *Anal Chem,* 1995, vol. 67 (20), 3702-12
- **Zenke, M. ; Steinlein, P. ; Wagner, E. ; Cotten, M. ; Beug, H. ; Birnstiel, M. L.** Receptor-Mediated Endocytosis Of Transferrin Polycation Conjugates - An Efficient Way To Introduce DNA Into Hematopoietic Cells. *Proceedings Of The National Academy Of Sciences Of The United States Of America,* 1990, vol. 87 (10), 3655-3659
- **Zhang, F. ; Andreassen, P. ; Fender, P. ; Geissler, E. ; Hernandez, J. F. ; Chroboczek, J.** A transfecting peptide derived from adenovirus fiber protein. *Gene Ther,* 1999, vol. 6 (2), 171-81